(19) Europäisches Patentamt
European Patent Office
Office européen des brevets

(11) EP 2 934 678 B1

(12) # EUROPEAN PATENT SPECIFICATION

(45) Date of publication and mention of the grant of the patent:
**21.06.2017 Bulletin 2017/25**

(21) Application number: **13814342.5**

(22) Date of filing: **11.12.2013**

(51) Int Cl.:
***A61Q 5/02*** (2006.01) ***A61Q 5/12*** (2006.01)
***A61K 8/81*** (2006.01) ***A61K 8/891*** (2006.01)

(86) International application number:
**PCT/US2013/074246**

(87) International publication number:
**WO 2014/099512 (26.06.2014 Gazette 2014/26)**

(54) **CARBOXYETHYL ACRYLATE CONTAINING COPOLYMER STABILIZER/THICKENERS AND METHODS TO MITIGATE THE LOSS OF SILICONE DEPOSITION ON KERATINOUS SUBSTRATES**

CARBOXYETHYLACRYLATHALTIGE COPOLYMERSTABILISATOREN/-VERDICKUNGSMITTEL UND VERFAHREN ZUR REDUZIERUNG DES VERLUSTES EINER SILIKONABSCHEIDUNG AUF KERATINISCHEN SUBSTRATEN

STABILISANT/ÉPAISSISSANTS DE COPOLYMÈRE CONTENANT DE L'ACRYLATE DE CARBOXYÉTHYLE ET PROCÉDÉS POUR ATTÉNUER LA PERTE DE DÉPÔT DE SILICONE SUR DES SUBSTRATS DE KÉRATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2012 US 201261739838 P**

(43) Date of publication of application:
**28.10.2015 Bulletin 2015/44**

(73) Proprietor: **Lubrizol Advanced Materials, Inc.**
**Cleveland, OH 44141-3247 (US)**

(72) Inventors:
- **TAMARESELVY, Krishnan**
  **Cleveland, Ohio 44141-3247 (US)**
- **FILLA, Deborah, S.**
  **Cleveland, Ohio 44141-3247 (US)**
- **RAFFERTY, Denise, W.**
  **Cleveland, Ohio 44141-3247 (US)**
- **LEPILLEUR, Carole, A.**
  **Cleveland, Ohio 44141-3247 (US)**
- **DASHIELL, David, L.**
  **Cleveland, Ohio 44141-3247 (US)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2012/053598**
**WO-A1-2013/148904**
**US-A1- 2008 311 066**
**US-A1- 2011 064 688**
**US-A9- 2004 001 796**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 934 678 B1

## Description

## FIELD OF THE INVENTION

The present invention relates to personal care compositions, such as detersive conditioning compositions for keratinous substrates. More specifically, the invention relates to detersive conditioning compositions suitable for use in personal care cleansing applications. The conditioning cleansing compositions comprise water, at least one detersive surfactant, a silicone, and a suspending agent that mitigates the loss of silicone(s) on keratinous substrates upon rinsing following application to the hair and skin.

## BACKGROUND OF THE INVENTION

Historically, it has been considered desirable to cleanse the hair and skin and then to condition them after cleansing. In the past it was necessary to perform these steps in two separate procedures. With the advent of two-in-one cleansers (e.g., conditioning shampoos), it became possible to condition and cleanse simultaneously. Detersive conditioning compositions such as the two-in-one conditioning shampoos are well-known in the art. Two-in-one conditioning shampoos are popular among consumers as a means of conveniently obtaining hair conditioning and cleansing performance from a single hair care product. One of the most important classes of conditioning agents utilized by cleansing product formulators is the silicones. Wide varieties of silicone conditioning agents are known. A general review of silicone conditioning agents is set forth in the Cosmetic Science and Technology Series; Vol. 2, entitled Conditioning Agents for Hair and Skin edited by Randy Schueller and Perry Romanowski, copyright 1999 by Marcel Dekker, Inc., New York, New York.

During formulation, silicones are suspended in shampoo compositions as dispersed oily hydrophobic emulsion droplets. The conditioning effect is achieved by the silicone material being deposited onto the hair during rinsing, resulting in the formation of a film. Silicone film deposition is known to give good sensory benefits (e.g., smoothness, soft feel), provide improved tangle resistance in wet and dry combing, and repair of damaged hair (e.g. split ends).

The basis for making an emulsion is to stabilize the insoluble silicone material in the aqueous phase. In the early two-in-one formulations, the silicone droplets (particles) were stabilized by adding surfactants to the formulation. The choice and amount of surfactant became critical in determining the type of emulsion formed, and its uniformity and stability. However, such compositions often had relatively low viscosities and were perceived by the consumer to be low in quality as a result. Moreover, obtaining good deposition of the silicone conditioning agent is further complicated by the detersive action of the surfactants in the shampoo. Detersive surfactants by their very nature carry away or remove oil, grease, dirt, and particulate matter from the treated substrate. In doing so, the detersive surfactants can also interfere with deposition of the silicone conditioning agent by carrying away both deposited and non-deposited silicone material during rinsing.

In conditioning applications, the particle size of the silicone was also determined to be important. The effectiveness of the emulsion to provide the conditioning effect depends upon the amount of silicone that is deposited onto the hair. As discussed in U.S. Pat. No. 5,302,658 the larger the particle size of the silicone material, the faster the destabilization or breaking of the emulsion during rinsing, thus increasing the deposition of the silicone onto the hair.

A problem encountered in the formulation of silicone containing cleansing compositions, such as the two-in-one shampoos, is keeping the dispersed water insoluble silicone oily materials suspended and the total product stable while still providing satisfactory rheology (e.g., thickening, yield value, shear thinning and shear flow) as well as detersive and conditioning performance. Poorly stabilized silicone containing shampoos are prone to phase separation leading to the formation of an unattractive silicone layer at the surface of the shampoo which is detrimental to the performance and the consumer appeal of the product. A variety of materials, including rheology modifiers, have been incorporated into silicone containing cleansing compositions for purposes of thickening and stabilization against phase separation.

Rheology modifiers have been used in shampoo products to increase viscosity at low shear rates and to maintain flow properties at higher shear rates. In addition, it has been discovered that certain rheology modifiers not only provide for a thickening effect, but also provide for effective storage stable suspensions of insoluble and particulate materials in aqueous surfactant systems. A select kind of copolymer thickener has been proposed for this purpose. For example, U.S. Pat. No. 6,635,702 discloses a crosslinked acrylic emulsion polymer for use in aqueous surfactant containing compositions to thicken and stabilize products containing insoluble and particulate materials including silicones. U.S. Pat. Appln. Pub. No. 2003/0108503 discloses a cosmetic composition containing a crosslinked acrylic copolymer prepared from methacrylic acid and a $C_1$-$C_4$ alkyl acrylate, at least one polymer chosen from cationic and amphoteric polymers and at least one particulate silicone. The compositions are said to be stable, have an attractive visual appearance and can provide good cosmetic properties to the hair, for example, lightness, softness, smooth feel, suppleness and manageability.

While crosslinked acrylic copolymers prepared from methacrylic acid and a $C_1$-$C_4$ alkyl acrylate are known to

provide storage stable suspensions of silicone particles in aqueous surfactant compositions, it has been found that only silicones of larger particle sizes (e.g., average particle size ≥5 μm) are able to efficiently deposit onto the hair from detersive compositions containing such copolymers. The ability of smaller particle size silicones (e.g., average particle size <5 μm) to efficiently deposit onto hair from such crosslinked acrylic copolymer containing compositions is severely reduced. Without wishing to be bound by theory it is surmised that crosslinked acrylic copolymers prepared from methacrylic acid and a $C_1$-$C_4$ alkyl acrylate are much more efficient in the stabilization of smaller particle sized silicone emulsions such that these emulsions are difficult to break during the shampooing process, resulting in much of the silicone being rinsed away. This deleterious effect becomes more pronounced as the amount of the copolymer in the formulation is increased. In other words, silicone deposition is inversely proportional to the amount of the acrylic stabilizer thickener present. Consequently, less than an optimal amount of silicone conditioning agent is released to the hair and the conditioning benefit is diminished.

U.S. Pat. No. 7,504,094 proposes to solve the problem of small particle size silicone deposition onto hair by providing a combination of a detersive surfactant, a thickener selected from a crosslinked acrylic copolymer prepared from methacrylic acid and a $C_1$-$C_6$ alkyl acrylate, and a microemulsion of a particular amino functionalized (aminosilicone) having a particle size ranging from 0.005 to 0.06 μm in a cosmetically acceptable medium. While small particle sized silicone microemulsions are disclosed, the resolution of the small particle size silicone deposition problem is restricted to the selection of a specific amino functionalized silicone.

US-A-2004/001796 discloses pesonal care compositions comprising a surfactant, a silicone conditioning agent, water and a cross-linked methacrylic acid/ethyl acrylate copolymer as a thickener.

US-A-2011/064688 discloses a personal care composition comprising an aqueous dispersion comprising silicone and an ethylene acrylic acid deposition polymer and at least one cosmetically acceptable surfactant.

US-A-2008/311066 discloses a personal care composition comprising a polymer, a surfactant and a silicone. The polymer can be derived from monomers such as carboxyethyl acrylate and ethyl acrylate.

While a variety of polymeric materials have been included in two-in-one shampoo compositions for purposes of thickening, pearlescence, and stabilization and deposition of silicone conditioning agents, there remains a need for a polymer thickener system that provides the desired rheological properties (e.g., thickening, shear thinning, flow and suspension of particulate materials), stabilization and deposition of silicone conditioning agents regardless of silicone type and particle size.

Surprisingly, it has been discovered that the polymeric thickeners/stabilizers prepared from carboxyethyl acrylate and/or oligomers thereof provide stable silicone containing cleansing compositions without interfering with the deposition of the silicone material onto keratinous substrates and other detersive functions.

In one aspect, embodiments of the present invention relate to stable cleansing compositions comprising a silicone conditioning agent.

In another aspect,embodiments of the invention relate to a stable cleansing composition comprising a silicone conditioning agent and a polymeric thickener which mitigates the loss of silicone deposition on keratinous substrates.

In still another aspect, embodiments of the invention relate to a thickened stable personal care cleansing composition which provides good conditioning properties to keratinous substrates.

In a further aspect, embodiments of the invention relate to a two-in-one shampoo composition comprising a silicone conditioning agent and a polymeric thickener which mitigates the loss of silicone deposition on the hair and scalp.

In a still further aspect, embodiments of the invention relate to a two-in-one shampoo composition comprising a silicone conditioning agent and a polymeric thickener which mitigates the loss of silicone deposition on the hair and scalp.

In an additional aspect, embodiments of the invention relate to a cleansing composition comprising a silicone conditioning agent and a polymeric thickener which mitigates the loss of silicone deposition on the skin, and provides stable suspensions of pearlescent and other insoluble materials to deliver an aesthetic appearance and good shelf appeal.

In a still additional aspect, embodiments of the invention relate to a method of mitigating the loss of silicone deposition on keratinous substrates by providing a stable personal care detersive composition comprising a silicone conditioning agent and a polymeric thickener which does not unduly interfere with the deposit of silicone materials.

## SUMMARY OF THE INVENTION

The present invention relates to the following embodiments:

1. A personal care composition comprising:

   A) at least one detersive surfactant selected from anionic, amphoteric, cationic, or nonionic surfactant;
   B) at least one silicone conditioning agent;
   C) water; and
   D) a stabilizer/thickener polymer comprising a crosslinked acrylic copolymer prepared from a monomer composition comprising:

a) from 10 to 35 wt. %, from 15 to 35 wt. %, from 20 to 35 wt. %, or from 25 to 30 wt. % of a carboxyethyl acrylate monomer represented by the formula:

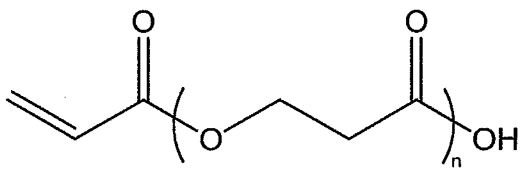

I

wherein n is an integer ranging from 1 to 10, from 2 to 8, or from 3 to 5;
b) from 30 to 70 wt.%, from 40 to 70 wt.%, from 50 to 70 wt.%, or from 55 to 65 wt. % of a monomer selected from ethyl acrylate, vinyl acetate, or mixtures thereof;
c) from 0 to 40 wt.%, from 5 to 40 wt.%, from 10 to 40 wt.%, from 15 to 35 wt. %, or from 20 or 25 to 30 wt. % of (meth)acrylic acid; and
d) from 0.001 to 5 wt.%, from 0.05 to 3.5 wt.%, from 0.5 to 3 wt.%, or from 1 to 2.5 wt. % of a polyunsaturated crosslinking monomer containing at least two polymerizable ethylenically unsaturated moieties.

2. A personal care composition of embodiment 1, wherein said monomer composition further comprises from 0.01 to 15 wt.% of at least one copolymerizable monomer selected from:

e) at least one $C_1$-$C_{22}$ alkyl (meth)acrylate other than ethyl acrylate;
f) at least one $C_1$-$C_8$ hydroxyalkyl (meth)acrylate;
g) at least one (meth)acrylamide selected from (meth)acrylamide, N-($C_1$-$C_{10}$)alkyl (meth)acrylamide, N,N-di($C_1$-$C_{10}$)alkyl (meth)acrylamide, N-($C_1$-$C_{10}$)alkylamino($C_1$-$C_{10}$)alkyl(meth)acrylamide or N,N-di($C_1$-$C_{10}$)alkylamino($C_1$-$C_{10}$)alkyl(meth)acrylamide;
h) at least one N-vinyl amide and/or N-vinyl lactam;
i) at least one an associative monomer comprising (i) a polymerizable ethylenically unsaturated end group portion, (ii) a polyoxyalkylene mid-section portion, and (iii) a hydrophobic end group portion containing 7 to 30 carbon atoms;
j) at least one semi-hydrophobic monomer selected from at least one monomer represented by formulas VI and VII:

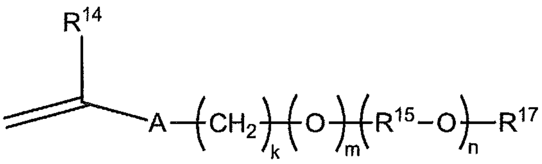

VI

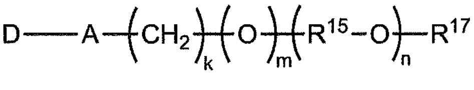

VII

wherein $R^{14}$ is hydrogen or methyl; A is $-CH_2C(O)O-$, $-C(O)O-$, $-O-$, $-CH_2O-$ ,$-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; $(R^{15}-O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150, from 5 to 120, or from 10 to 60; $R^{17}$ is selected from hydrogen and a linear or branched $C_1$-$C_4$ alkyl group; and D represents a vinyl or an allyl moiety;
k) at least one silicone macromer;
l) at least one vinyl ester of an aliphatic carboxylic acid containing an acyl moiety having 3 to 21 carbon atoms;
m) at least one alpha-olefinic monomer;
n) at least one monomer represented by the formula:

$CH_2=C(R)C(O)OAOR^2$

wherein A is a divalent radical selected from $-CH_2CH(OH)CH_2-$ and $-CH_2CH(CH_2OH)-$, R is selected from hydrogen or methyl, and $R^2$ is an acyl residue of a linear or branched, saturated or unsaturated $C_{10}$ to $C_{22}$ fatty acid;

o) at least one copolymerizable sulfonic acid containing monomer selected from styrenesulfonic acid, 2-(meth)acrylamido-2-methylpropane sulfonic acid, vinylsulfonic acid, allylsulfonic acid, methallylsulfonic acid; and salts thereof; and

combinations of monomers e) through o).

3. A personal care composition of any of the preceding embodiments, wherein said monomer composition further comprises from 0.01 to 5 wt.% of a copolymerizable sulfonic acid group containing monomer.

4. A personal care composition of any of the preceding embodiments, wherein said $C_1$-$C_{22}$ alkyl (meth)acrylate monomer other than ethyl acrylate is represented by the formula:

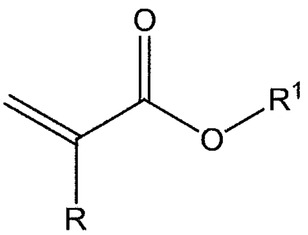

II

wherein R is hydrogen or methyl, and $R^1$ is methyl or a linear or branched $C_3$-$C_{22}$ alkyl group.

5. A personal care composition of any of the preceding embodiments, wherein said $C_1$-$C_8$ hydroxyalkyl (meth)acrylate monomer is represented by the formula:

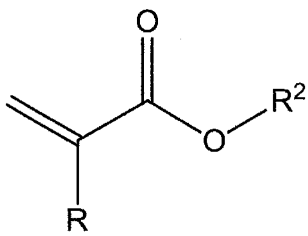

III

wherein R is hydrogen or methyl; and $R^2$ is a linear or branched $C_1$-$C_8$ hydroxyalkyl group.

6. A personal care composition of any of the preceding embodiments, wherein said (meth)acrylamide monomer is represented by the formulas:

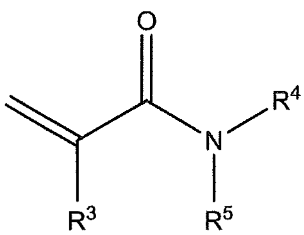

IV

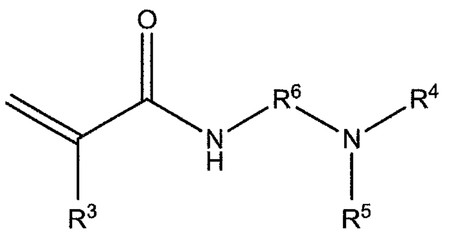

IVA

wherein $R^3$ represents hydrogen or methyl, $R^4$ and $R^5$ independently represent hydrogen, $C_1$ to $C_{10}$ alkyl and $C_1$ to $C_{10}$ hydroxyalkyl, and $R^6$ represents $C_1$ to $C_5$ alkylene.

7. A personal care composition of any of the preceding embodiments, wherein said N-vinyl amide is selected from N-vinylformamide, N-methyl-N-vinylformamide, N-(hydroxymethyl)-N-vinylformamide, N-vinylacetamide, N-vinylmethylacetamide, N-(hydroxymethyl)-N-vinylacetamide, and mixtures thereof; and said N-vinyl lactam is selected from N-vinyl-2-pyrrolidinone, N-(1-methyl vinyl) pyrrolidinone, N-vinyl-2-piperidone, N-vinyl-2-caprolactam, N-vinyl-5-methyl pyrrolidinone, N-vinyl-3,3-dimethyl pyrrolidinone, N-vinyl-5-ethyl pyrrolidinone and N-vinyl-6-methyl piperidone, and mixtures thereof.

8. A personal care composition of any of the preceding embodiments, wherein said associative monomer is represented by formulas V and/or VA:

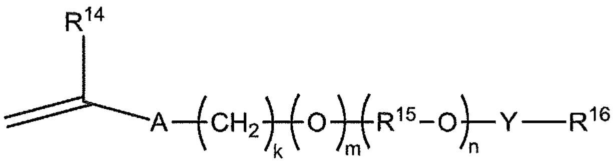

V

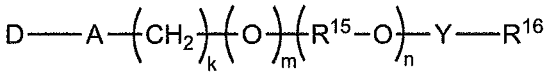

VA

wherein $R^{14}$ is hydrogen or methyl; A is $-CH_2C(O)O-$, $-C(O)O-$, $-O-$, $-CH_2O-$ ,$-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; a divalent alkylene radical containing 1 to 5 carbon atoms; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; $(R^{15}-O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150, from 10 to 120, or from 15 to 60; Y is $-R^{15}O-$, $-R^{15}NH-$, $-C(O)-$, $-C(O)NH-$, $-R^{15}NHC(O)NH-$, or $-C(O)NHC(O)-$; $R^{16}$ is a substituted or unsubstituted alkyl selected from a $C_8$-$C_{30}$ linear alkyl, a $C_8$-$C_{30}$ branched alkyl, a $C_7$-$C_{30}$ carbocyclic alkyl, a $C_2$-$C_{30}$ alkyl-substituted phenyl, an araalkyl substituted phenyl, and an aryl-substituted $C_2$-$C_{30}$ alkyl; wherein the $R^{16}$ alkyl group, carbocyclic alkyl group, aryl group, phenyl group optionally comprises one or more substituents selected from the group selected from a methyl group, hydroxyl group, an alkoxyl group, benzyl group styryl group, and a halogen group.

9. A personal care composition of any of the preceding embodiments, wherein said associative monomer is represented by formula VB:

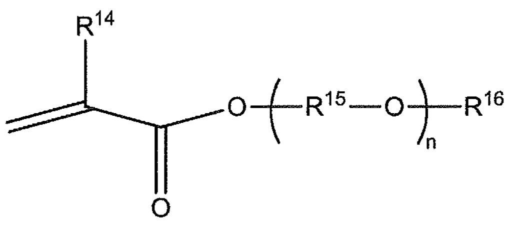

VB

wherein $R^{14}$ is hydrogen or methyl; $R^{15}$ is a divalent alkylene moiety independently selected from $C_2H_4$, $C_3H_6$, and $C_4H_8$, and n represents an integer ranging from 10 to 60, ($R^{15}$-O) can be arranged in a random or a block configuration; $R^{16}$ is a substituted or unsubstituted alkyl selected from a $C_8$-$C_{30}$ linear alkyl, a $C_8$-$C_{30}$ branched alkyl, a $C_7$-$C_{30}$ carbocyclic alkyl, a $C_2$-$C_{30}$ alkyl-substituted phenyl, an araalkyl substituted phenyl, and an aryl-substituted $C_2$-$C_{30}$ alkyl, wherein the $R^{16}$ alkyl group, aryl group, phenyl group optionally comprises one or more substituents selected from the group consisting of a hydroxyl group, an alkoxyl group, benzyl group styryl group, and a halogen group.

10. A personal care composition of any of the preceding embodiments, wherein said semi-hydrophobic monomer comprises (i) a polymerizable ethylenically unsaturated end group portion, (ii) a polyoxyalkylene mid-section portion, and (iii) an end group portion selected from hydrogen or an alkyl group containing 1 to 4 carbon atoms.

11. A personal care composition of any of the preceding embodiments, wherein said semi-hydrophobic monomer is selected from at least one monomer represented by formulas VIA and VIB:

$$CH_2{=}C(R^{14})C(O)O\text{-}(C_2H_{40})_a(C_3H_6O)_b\text{-}H \quad \text{VIA}$$

$$CH_2{=}C(R^{14})C(O)O\text{-}(C_2H_4O)_a(C_3H_6O)_b\text{-}CH_3 \quad \text{VIB}$$

wherein $R^{14}$ is hydrogen or methyl, and "a" is an integer ranging from 0 or 2 to 120, from 5 to 45, or from 10 to 25, and "b" is an integer ranging from 0 or 2 to 120, from 5 to 45, or from 10 to 25, subject to the proviso that "a" and "b" cannot be 0 at the same time.

12. A personal care composition of any of the preceding embodiments, wherein said silicon macromer comprises (i) a polymerizable citraconate end group portion, (ii) a polyoxyalkylene mid-section portion, and (iii) a dimethicone end group portion.

13. A personal care composition of any of the preceding embodiments, wherein said silicone macromer is selected from at least one macromer represented by the formula:

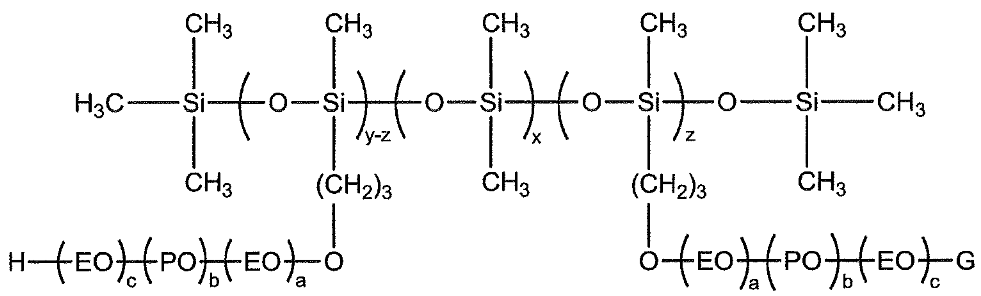

VIII

wherein EO represents a divalent ethylene oxide residue, PO independently represents a divalent propylene oxide residue; a, b, and c are independently from 0 to 100, from 1 to 50, or from 5 to 25, subject to the proviso that a, b, and c cannot all be 0 at the same time; wherein the EO and PO residues are arranged in random or blocky sequences; x is 0 to 200; y is 1 to 200; z ≤ y; and G represents a polymerizable reactive moiety represented as follows:

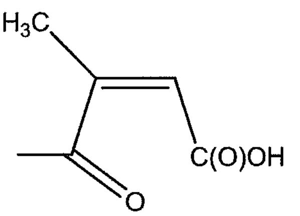

,

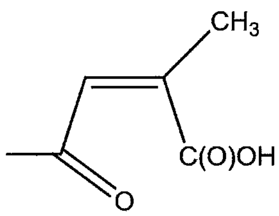

,

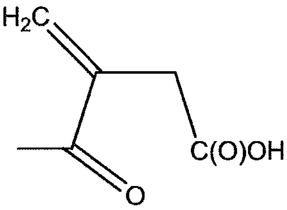
,
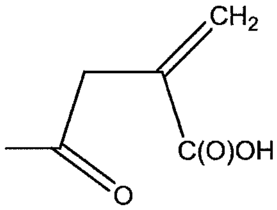

14. A personal care composition of any of the preceding embodiments, wherein said vinyl ester of a carboxylic acid is represented by the formula:

$$CH_2{=}CHOC(O)R^{18} \qquad IX$$

wherein $R^{18}$ is selected from a linear or branched $C_2$-$C_{21}$ alkyl group.

15. A personal care composition of any of the preceding embodiments, wherein said alpha-olefinic monomer is represented by the formula:

$$CH_2{=}CH\text{-}R^{19} \qquad X$$

wherein $R^{19}$ represents a moiety selected from hydrogen, a linear or branched $C_1$-$C_8$ alkyl, or aryl.

16. A personal care composition of any of the preceding embodiments, wherein said polyunsaturated crosslinking monomer is selected from the esterification product of (meth)acrylic acid and a linear or branched polyol having 2 to 12 carbon atoms; the etherification product of allyl alcohol and a linear or branched polyol having 2 to 12 carbon atoms; divinyl glycol, divinyl benzene; and methylenebisacrylamide.

17. A personal care composition of any of the preceding embodiments, wherein said copolymerizable sulfonic acid containing monomer is selected from styrenesulfonic acid, acrylamidomethylpropanesulfonic acid, vinylsulfonic acid, vinylphosphonic acid, allylsulfonic acid, methallylsulfonic acid; and salts thereof.

18. A personal care composition of any of the preceding embodiments, wherein said at least one $C_1$-$C_{22}$ alkyl (meth)acrylate other than ethyl acrylate is selected from methyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, behenyl (meth)acrylate, or mixtures thereof.

19. A personal care composition of any of the preceding embodiments, wherein said at least one $C_1$-$C_8$ hydroxyalkyl (meth)acrylate monomer is selected from 2-hydroxyethyl(meth)acrylate, 3-hydroxypropyl(meth)acrylate, 4-hydroxybutyl(meth)acrylate, or mixtures thereof.

20. A personal care composition of any of the preceding embodiments, wherein said at least one (meth)acrylamide is selected from N-methyl(meth)acrylamide, N-ethyl(meth)acrylamide, N-propyl(meth)acrylamide, N-isopropyl(meth)acrylamide, N-tert-butyl(meth)acrylamide, N-tert-octyl(meth)acrylamide, N-(2-hydroxyethyl)(meth)acrylamide, N-(3-hydroxypropyl)(meth)acrylamide; N,N-dimethyl(meth)acrylamide, N,N-diethyl(meth)acrylamide, N,N-(di-2-hydroxyethyl)(meth)acrylamide, N,N-(di-3-hydroxypropyl)(meth)acrylamide, N-methyl,N-ethyl(meth)acrylamide; N,N-dimethylaminoethyl(meth)acrylamide, N,N-diethylaminoethyl(meth)acrylamide, N,N-dimethylaminopropyl(meth)acrylamide; and mixtures thereof.

21. A personal care composition of any of the preceding embodiments, wherein said at least one N-vinyl amide and/or N-vinyl lactam is selected from N-vinylformamide, N-methyl-N-vinylformamide, N-(hydroxymethyl)-N-vinylformamide, N-vinylacetamide, N-vinylmethylacetamide, N-(hydroxymethyl)-N-vinylacetamide; N-vinyl-2-pyrrolidinone, N-(1-methyl vinyl) pyrrolidinone, N-vinyl-2-piperidone, N-vinyl-2-caprolactam, N-vinyl-5-methyl pyrrolidinone, N-vinyl-3,3-dimethyl pyrrolidinone, N-vinyl-5-ethyl pyrrolidinone and N-vinyl-6-methyl piperidone; and mixtures thereof.

22. A personal care composition of any of the preceding embodiments, wherein said at least one associative monomer

is selected from lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene methacrylate, where the polyethoxylated portion of the monomer contains from 2 to 150 ethylene oxide units, from 5 to 120, or from 10 to 60; octyloxy polyethyleneglycol polypropyleneglycol (meth)acrylate, phenoxy polyethylene glycol polypropylene glycol (meth)acrylate, and nonylphenoxy polyethylene glycol polypropylene glycol (meth)acrylate, where the polyethoxylated and/or the polypropoxylated portion of the monomer independently contain 0 or 2 to 120, from 5 to 45, or from 10 to 25; and mixtures thereof.

23. A personal care composition of any of the preceding embodiments, wherein said at least one semi-hydrophobic monomer is selected from polyethyleneglycol (meth)acrylate, polypropyleneglycol (meth)acrylate, polyethyleneglycol polypropylene glycol methacrylate or methoxypolyethyleneglycol (meth)acrylate, where the polyethoxylated and/or the polypropoxylated portion of the monomer independently contain 0 or 2 to 120, from 5 to 45, or from 10 to 25; and mixtures thereof.

24. A personal care composition of any of the preceding embodiments, wherein said at least one semi-hydrophobic monomer is selected from a compound having the formula: $CH_2=CH-O(CH_2)_4O(C_3H_6O)_4(C_2H_4O)_{10}H$; $CH_2=CH-O(CH_2)_4O(C_3H_6O)_4(C_2H_4O)_{20}H$; $CH_2=CH-O(CH_2)_4O(C_3H_6O)_4(C_2H_4O)_{30}H$; $CH_2=CHCH_2O(C_3H_6O)_4(C_2H_4O)_{10}H$; $CH_2=CHCH_2O(C_3H_6O)_4(C_2H_4O)_{20}H$; $CH_2=CHCH_2O(C_3H_6O)_4(C_2H_4O)_{30}H$; and $CH_2=CHCH_2O(C_3H_6O)_5(C_2H_4O)_5H$.

25. A personal care composition of any of the preceding embodiments, wherein said at least one silicone macromer is selected from an alkoxylated dimethicone citraconate.

26. A personal care composition of any of the preceding embodiments, wherein said at least one vinyl ester of an aliphatic carboxylic acid is selected vinyl acetate, vinyl propionate, vinyl butyrate, vinyl isobutyrate, vinyl valerate, vinyl hexanoate, vinyl 2-methylhexanoate, vinyl 2-ethylhexanoate, vinyl iso-octanoate, vinyl nonanoate, vinyl neodecanoate, vinyl decanoate, vinyl laurate, vinyl palmitate, vinyl stearate, and mixtures thereof.

27. A personal care composition of any of the preceding embodiments, wherein said at least one alpha-olefinic monomer is selected from ethylene, propylene, 1-butene, iso-butylene, 1-hexene, 1-heptene, 4-methyl-1-pentene, styrene, alpha-methyl styrene, and mixtures thereof.

28. A personal care composition of any of the preceding embodiments, wherein said at least one monomer (n) is an acrylate ester of the glycidyl ester of neodecanoic acid.

29. A personal care composition of any of the preceding embodiments, wherein said crosslinked acrylic copolymer comprises repeating units polymerized from:

a) from 10 to 35 wt.% of carboxyethyl acrylate;
b) from 40 to 70 wt.% of a monomer selected from ethyl acrylate, vinyl acetate, or mixtures thereof;
c) 0 to 40 wt.% of a methacrylic acid; and
d) 0.1 to 1 wt.% of a polyunsaturated crosslinking monomer containing at least two polymerizable ethylenically unsaturated moieties.

30. A personal care composition of any of the preceding embodiments, wherein said crosslinked acrylic copolymer comprises repeating units polymerized from 10 to 40 wt.% methacrylic acid.

31. A personal care composition of any of the preceding embodiments, wherein said crosslinked acrylic copolymer further comprises repeating units polymerized from 1 to 15 wt.% of hydroxypropyl methacrylate, hydroxyethyl methacrylate, and mixtures thereof.

32. A personal care composition of any of the preceding embodiments, wherein said crosslinked acrylic copolymer comprises repeating units polymerized from carboxyethyl acrylate and/or oligomers thereof, ethyl acrylate, and methacrylic acid.

33. A personal care composition of any of the preceding embodiments, wherein said crosslinked acrylic copolymer comprises repeating units polymerized from carboxyethyl acrylate and/or oligomers thereof, vinyl acetate, and methacrylic acid.

34. A personal care composition of any of the preceding embodiments, wherein said crosslinked acrylic copolymer further comprises repeating units polymerized from 0.5 to 10 wt.% of N-vinyl pyrrolidone, styrene, N-tert-butylacrylamide, N-tert-octylacrylamide, 2-ethylhexyl acrylate, stearyl methacrylate, acrylate ester of the glycidyl ester of neodecanoic acid, an alkoxylated dimethicone citraconate, cetearyl polyethoxylated methacrylate, behenyl polyethoxylated methacrylate, methoxy polyethoxylated methacrylate (MPEG), acrylamidomethylpropanesulfonic acid, and mixtures thereof.

35. A personal care composition of any of the preceding embodiments, wherein said polyunsaturated crosslinking monomer is selected from trimethylolpropane triacrylate, trimethylolpropane diallyl ether, triethyleneglycol dimethacrylate, allyl pentaerythritol, allyl sucrose, and mixtures thereof.

36. A personal care composition of any of the preceding embodiments, wherein said crosslinked acrylic copolymer is prepared in a batch, semi-batch or a staged polymerization process.

37. A personal care composition of any of the preceding embodiments, further comprising a pearlizing agent.

38. A personal care composition of embodiment 37, wherein said pearlizing agent is selected from mica, metal oxide coated mica, silica coated mica, bismuth oxychloride coated mica, bismuth oxychloride, myristyl myristate, glass, metal oxide coated glass, various aluminum and magnesium salts, guanine, fish scales, glitter (polyester or metallic), and mixtures thereof.

39. A personal care composition of embodiment 37, wherein said pearlizing agent is selected from monoesters and/or diesters of ethylene glycol, propylene glycol, diethylene glycol, dipropylene glycol, triethylene glycol and tetraethylene glycol with fatty acids containing from 6 to 22 carbon atoms.

40. A personal care composition of embodiment 39, wherein said pearlizing agent is selected from ethylene glycol monostearate (EGMS), ethylene glycol distearate (EGDS), polyethylene glycol monostearate (PGMS), polyethyleneglycol distearate (PGDS), and mixtures thereof.

41. A personal care composition of any of the preceding embodiments, further comprising a particulate material selected from pigments, exfoliating agents, anti-dandruff agents, clay, swellable clay, laponite, gas bubbles, liposomes, UV absorbers, antibacterial compositions, hair fixative, anti-wrinkling and anti-aging compositions, microsponges, cosmetic beads and flakes

42. A personal care composition of any of the preceding embodiments, wherein said silicone conditioning agent is non-volatile silicone selected from a silicone oil, silicone gum, silicone resin and mixtures thereof.

43. A personal care composition of embodiment 42 further comprising a volatile silicone.

44. A personal care composition of any of the preceding embodiments, wherein said silicone oil is selected from a compound represented by the formula:

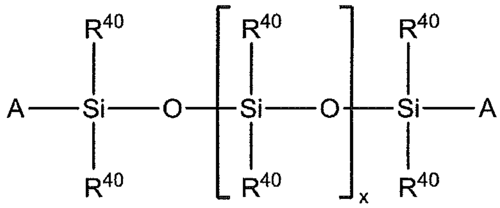

XII

wherein A independently represents hydroxy, methyl, methoxy, ethoxy, propoxy, and phenoxy;$R^{40}$ independently represents methyl, ethyl, propyl, phenyl, methylphenyl, phenylmethyl; and x is an integer ranging from 7 to 8000, from 50 to 5000, from 100 to 3000, or from 200 to 1000.

45. A personal care composition of any of the preceding embodiments, wherein said silicone conditioning agent is selected from polydimethylsiloxanes (dimethicones), polydiethylsiloxanes, polydimethyl siloxanes having terminal hydroxyl groups (dimethiconols), polymethylphenylsiloxanes, phenylmethylsiloxanes, and mixtures thereof.

46. A personal care composition of any of embodiments 1 to 43, wherein said silicone conditioning agent is selected from an amino functional polydimethylsiloxane (amodimethicone).

47. A personal care composition of embodiment 46, wherein said amino functional silicone is selected from a compound represented by the formula:

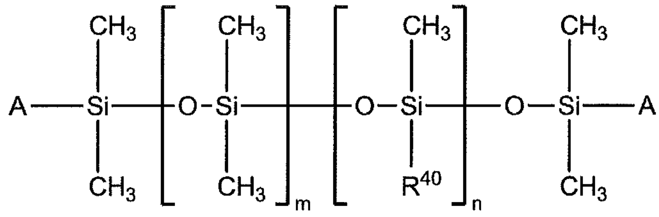

XIIA

wherein A independently represents hydroxy, methyl, methoxy, ethoxy, propoxy, and phenoxy; and $R^{40}$ is selected from:

$-R^{41}-N(R^{42})CH_2CH_2N(R^{42})_2$;

$-R^{41}-N(R^{42})_2$;

$-R^{41}-N^+(R^{42})_3CA^-$;

and

$-R^{41}-N(R^{42})CH_2CH_2N(R^{42})H_2CA^-$

wherein $R^{41}$ is a linear or branched, hydroxyl substituted or unsubstituted alkylene or alkylene ether moiety containing 2 to 10 carbon atoms; $R^{42}$ is hydrogen, $C_1$-$C_{20}$ alkyl (e.g, methyl), phenyl or benzyl; $CA^-$ is a halide ion selected from chlorine, bromine, iodine and fluorine; and the sum of m+n ranges from 7 to 1000, from 50 to 250, or from 100 to 200, subject to the proviso that m or n is not 0.

48. A personal care composition of any of the preceding embodiments, wherein said silicone conditioning agent is present in an amount ranging from 0.01 to 20 wt.%, from 0.05 to 15 wt. %, from 0.1% to 10 wt.%, or from 1 to 5 wt.%, based on the weight of the total composition.

49. A personal care composition of any of the preceding embodiments, wherein said silicone conditioning agent has a particle size ranging from 0.003 to 500 μm, from 0.05 to 200 μm, from 0.25 to 200 μm, from 0.5 to 150 μm, from 1 to 100 μm, from 5 to 80 μm, from 10 to 60 μm, or from 20 to 50 μm.

50. A personal care composition of any of the preceding embodiments, wherein said silicone conditioning agent is in the form of emulsion droplets.

51. A personal care composition of any of the preceding embodiments, wherein said at least one detersive surfactant is present in an amount ranging from 2 to 50 wt.%, from 6 to 40 wt.%, from 8 to 25 wt.%, from 10 to 20 wt.%, or from 12 to 16 wt.%, based on total weight of the composition.

52. A personal care composition of embodiment 51, wherein said at least one detersive surfactant is selected from an anionic surfactant and an amphoteric surfactant.

53. A personal care composition of embodiment 52, wherein the weight ratio of active anionic surfactant to active amphoteric surfactant ranges from 1:1 to 10:1, from 2.25:1 to 9:1, or from 4.5:1 to 7:1.

54. A personal care composition of any of the previous embodiments, wherein said crosslinked acrylic copolymer

is present in an amount ranging from 0.01 to 25 wt.%, from 0.1 to 15 wt.%, from 0.5 to 10 wt.%, or from 1 to 5 wt.%, based on the weight of the total composition (all polymer weights based on 100 % active polymer solids).

55. A personal care composition of any of the previous embodiments, further comprising an auxiliary conditioning agent selected from a hydrocarbon oil, an ester oil, and combinations thereof.

56. A personal care composition of any of the previous embodiments, further comprising a cationic polymer.

57. A personal care composition of any of the previous embodiments, wherein said composition is neutralized with a basic neutralizing agent to obtain a pH ranging from 5 to 12.

58. A personal care composition of embodiment 57, wherein the composition is back-acid treated with an acidic neutralizing agent to reduce the pH by at least 0.5, 1, 2, 3, 4, or 5 pH units.

59. A method of reducing the loss of silicone deposition from a detersive conditioning composition for keratinous substrates, said composition comprised of from 3 to 40 wt.% of an anionic and amphoteric surfactant combination and from 0.01 to 20 wt.% of a silicone conditioning agent, wherein the weight ratio of active anionic surfactant to active amphoteric surfactant ranges from 1:1 to 10:1, said method comprised of adding thereto from 0.01 to 25 wt.% of a crosslinked acrylic copolymer prepared from a monomer composition comprising:

a) from 10 to 35 wt.% of carboxyethyl acrylate;
b) from 30 to 70 wt.% of a monomer selected from ethyl acrylate, vinyl acetate, or mixtures thereof;
c) from 0 to 40 wt.% of (meth)acrylic acid; and
d) from 0.001 to 5 wt.% of a polyunsaturated crosslinking monomer containing at least two polymerizable ethylenically unsaturated moieties.

60. A method of embodiment 59, wherein said crosslinked acrylic copolymer is prepared from a monomer composition further comprising from 0.001 to 5 wt.% of at least one $C_1$-$C_8$ hydroxyalkyl (meth)acrylate.

61. A method of embodiment 60, wherein said at least one $C_1$-$C_5$ hydroxyalkyl (meth)acrylate is selected from 2-hydroxypropyl methacrylate, 2-hydroxyethyl methacrylate, and mixtures thereof.

62. Use of the personal care composition as defined in any of embodiments 1 to 58 as a detersive composition for keratinous substrates.

The silicone containing cleansing compositions comprising the polymeric thickener/stabilizers of the invention provide stable formulations without interfering with the deposition of silicone material onto the hair and/or the skin regardless of silicone type and silicone particle size.

The methods, polymers and compositions of the present invention may suitably comprise, consist of, or consist essentially of the components, elements, steps, and process delineations described herein. The invention illustratively disclosed herein suitably may be practiced in the absence of any element which is not specifically disclosed herein.

Unless otherwise stated, all percentages, parts, and ratios expressed herein are based upon weight of the total compositions of the present invention.

When referring to a specified monomer(s) that is incorporated into a polymer of the invention, it will be recognized that the monomer(s) will be incorporated into the polymer as a unit(s) derived from the specified monomer(s) (e.g., repeating unit).

For the purpose of the specification, the prefix "(meth)acryl" includes "acryl" as well as "methacryl". For example, the term (meth)acrylic includes both acrylic and methacrylic, and the term (meth)acrylate includes acrylate as well as methacrylate. By way of further example, the term "(meth)acrylamide" includes both acrylamide and methacrylamide.

The term "keratinous" material as used herein includes, without limitation, hair, nails and skin.

The term "personal care products" as used herein includes, without being limited thereto, cosmetics, toiletries, cosmeceuticals, beauty aids, insect repellents, personal hygiene and cleansing products applied to the body, including the skin, hair, scalp, and nails of humans and animals.

As used herein, the term "rheological properties" and grammatical variations thereof, includes, without limitation, such properties as Brookfield viscosity, increase or decrease in viscosity in response to shear stress, flow characteristics, gel properties such as stiffness, resilience, flowability, texture, and the like, foam properties such as foam stability, foam density, ability to hold a peak, and the like, suspension properties such as yield value, and aerosol properties such as ability to form aerosol droplets when dispensed from propellant based or mechanical pump type aerosol dispensers.

## DESCRIPTION OF EXEMPLARY EMBODIMENTS OF THE INVENTION

Exemplary embodiments in accordance with the present invention will be described.

According to the invention, the crosslinked acrylic stabilizing and thickening polymer is prepared by the free radical emulsion polymerization of a monomer composition comprising one or more ethylenically unsaturated monomers represented by Formula I:

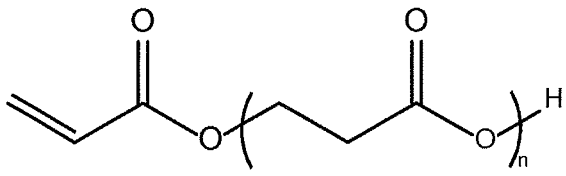 I

wherein n has an average value of from 1 to 10 in one aspect, from 2 to 8 in another aspect, and from 3 to 5 in a further aspect.

When n is 1, Formula 1 represents carboxyethyl acrylate (the dimer of acrylic acid), and when n is more than 1 Formula I represents an oligomer of acrylic acid (herein referred to as the oligomer of carboxyethyl acrylate). For brevity, compositions represented by Formula I are categorized herein as monomers. Carboxyethyl acrylate, oligomers of carboxyethyl acrylate and polymers prepared from these monomers are described in European Pat. Appln. Pub. No. 0 036 294.

In one aspect of the invention, the crosslinked acrylic stabilizing/thickening polymers are prepared by polymerizing a monomer composition comprising monomer (a) selected from at least one monomer represented by Formula I ; and monomer (b) selected from ethyl acrylate, vinyl acetate, and mixtures thereof.

In another aspect of the invention, the crosslinked acrylic stabilizing/thickening polymers are prepared by polymerizing a monomer composition comprising monomer (a) selected from at least one monomer represented by Formula I; monomer (b) selected from ethyl acrylate, vinyl acetate, and mixtures thereof; and monomer (c) selected from acrylic acid, methacrylic acid, mixtures thereof; and a crosslinking monomer (d).

In still another aspect of the invention, the crosslinked acrylic stabilizing/thickening polymers are prepared by polymerizing a monomer composition comprising monomer (a) selected from at least one monomer represented by Formula I; monomer (b) selected from ethyl acrylate, vinyl acetate, and mixtures thereof; a crosslinking monomer (d); and at least one monomer selected from the following:

(e) at least one $C_1$-$C_{22}$ alkyl (meth)acrylate monomer other than ethyl acrylate;
(f) at least one $C_1$-$C_8$ hydroxyalkyl (meth)acrylate monomer;
(g) at least one (meth)acrylamide monomer;
(h) at least one N-vinyl amide and/or N-vinyl lactam;
(i) at least one an associative monomer;
(j) at least one semi-hydrophobic monomer;
(k) at least one silicone macromer monomer;
(l) at least one vinyl ester of a carboxylic acid containing an acyl moiety having 3 to 21 carbon atoms;
(m) at least one alpha-olefinic monomer; and
(n) at least one acrylate ester of the glycidyl ester of a linear or branched, saturated or unsaturated $C_{10}$ to $C_{22}$ fatty acid.
(o) at least one copolymerizable sulfonic acid or salt thereof containing monomer; and combinations of monomers (e) through (o).

In a further aspect of the invention, the crosslinked acrylic stabilizing/thickening polymers are prepared by polymerizing a monomer composition comprising monomer (a) selected from at least one monomer represented by Formula I; monomer (b) selected from ethyl acrylate, vinyl acetate, and mixtures thereof; monomer (c) selected from acrylic acid, methacrylic acid, mixtures thereof; a crosslinking monomer (d); and at least one monomer selected from monomers (e) to (o) described herein.

Monomer (d)

In all of the aspects of the invention disclosed immediately above, the crosslinking monomer (d) utilized in the preparation of the crosslinked acrylic stabilizing/thickening polymers are ethylenically polyunsaturated compounds containing at least two polymerizable ethylenically unsaturated moieties. Exemplary polyunsaturated crosslinking monomer components include di(meth)acrylate compounds such as ethylene glycol di(meth)acrylate, polyethylene glycol

di(meth)acrylate, triethylene glycol di(meth)acrylate, 1,3-butylene glycol di(meth)acrylate, 1,6-butylene glycol di(meth)acrylate, 1,6-hexanediol di(meth)acrylate, neopentyl glycol di(meth)acrylate, 1,9-nonanediol di(meth)acrylate, 2,2'-bis(4-(acryloxy-propyloxyphenyl)propane, and 2,2'-bis(4-(acryloxydiethoxy-phenyl)propane; tri(meth)acrylate compounds such as, trimethylolpropane tri(meth)acrylate, trimethylolethane tri(meth)acrylate, and tetramethylolmethane tri(meth)acrylate; tetra(meth)acrylate compounds such as ditrimethylolpropane tetra(meth)acrylate, tetramethylolmethane tetra(meth)acrylate, and pentaerythritol tetra(meth)acrylate; hexa(meth)acrylate compounds such as dipentaerythritol hexa(meth)acrylate; allyl compounds such as allyl (meth)acrylate, diallylphthalate, diallyl itaconate, diallyl fumarate, and diallyl maleate; polyallyl ethers of sucrose having from 2 to 8 allyl groups per molecule, polyallyl ethers of pentaerythritol such as pentaerythritol diallyl ether, pentaerythritol triallyl ether, and pentaerythritol tetraallyl ether, and combinations thereof; polyallyl ethers of trimethylolpropane such as trimethylolpropane diallyl ether, trimethylolpropane triallyl ether, and combinations thereof. Other suitable polyunsaturated compounds include divinyl glycol, divinyl benzene, and methylenebisacrylamide.

Particularly useful are polyunsaturated crosslinkers derived from ethoxylated polyols, such as diols, triols and bis-phenols, ethoxylated with about 2 to about 100 moles of ethylene oxide per mole of hydroxyl functional group and end-capped with a polymerizable unsaturated group such as a vinyl ether, allyl ether, acrylate ester, methacrylate ester, and the like. Examples of such crosslinkers include bisphenol A ethoxylated di(meth)acrylate; bisphenol F ethoxylated di(meth)acrylate, ethoxylated trimethylol propane tri(meth)acrylate, and the like. Other ethoxylated crosslinkers useful in the polymers of the present invention include ethoxylated polyol-derived crosslinkers disclosed in U.S. Patent No. 6,140,435 to Zanotti-Russo.

Non limiting examples of crosslinking monomers derived from ethoxylated polyols are acrylate and methacrylate esters of polyols having at least two acrylate or methacrylate ester groups, such as trimethylolpropane ethoxylated (15) triacrylate (TMPEO15TA) and ethoxylated (30) bisphenol A dimethacrylate (EOBDMA), and the like.

Monomer (e)

The $C_1$-$C_{22}$ alkyl (meth)acrylate monomer other than ethyl acrylate is represented by the formula:

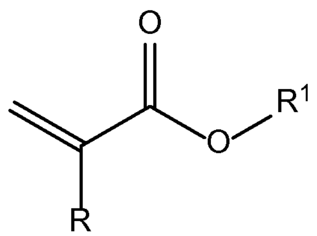

II

wherein R is hydrogen or methyl, and $R^1$ is methyl or a linear or branched $C_3$-$C_{22}$ alkyl group.

Representative monomers under Formula II include, but are not limited to, methyl (meth)acrylate, ethyl (meth)acrylate, propyl (meth)acrylate, isopropyl (meth)acrylate, butyl (meth)acrylate, isobutyl (meth)acrylate, hexyl (meth)acrylate, heptyl (meth)acrylate, octyl (meth)acrylate, 2-ethylhexyl (meth)acrylate, nonyl (meth)acrylate, decyl (meth)acrylate, isodecyl (meth)acrylate, lauryl (meth)acrylate, cetyl (meth)acrylate, stearyl (meth)acrylate, and behenyl (meth)acrylate, and mixtures thereof.

Monomer (f)

The $C_1$-$C_5$ hydroxyalkyl (meth)acrylate monomer is represented by Formula III below:

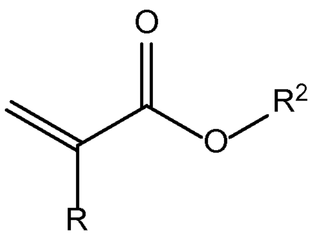

III

wherein R is hydrogen or methyl; and $R^2$ is a linear or branched $C_1$-$C_5$ hydroxyalkyl group.

Representative monomers under Formula III include, but are not limited to, 2-hydroxyethyl(meth)acrylate, 3-hydroxypropyl(meth)acrylate, 4-hydroxybutyl(meth)acrylate, and mixtures thereof.

Monomer (g)

The (meth)acrylamide monomer is represented by Formulas IV and IVA as follows:

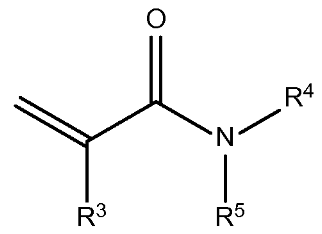

IV

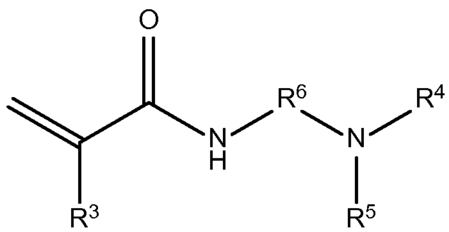

IVA

wherein $R^3$ represents hydrogen or methyl, $R^4$ and $R^5$ independently represent hydrogen, $C_1$ to $C_{10}$ alkyl and $C_1$ to $C_{10}$ hydroxyalkyl, and $R^6$ represents $C_1$ to $C_5$ alkylene.

Exemplary (meth)acrylamides under Formulas IV and IVA include N-($C_1$-$C_{10}$)alkyl (meth)acrylamides, N,N-di($C_1$-$C_{10}$)alkyl (meth)acrylamides, N-($C_1$-$C_{10}$)alkylamino($C_1$-$C_{10}$)alkyl(meth)acrylamides, and N,N-di($C_1$-$C_5$)alkylamino($C_1$-$C_{10}$)alkyl(meth)acrylamides.

Representative N-($C_1$-$C_{10}$)alkyl (meth)acrylamides include, for example, N-methyl(meth)acrylamide, N-ethyl(meth)acrylamide, N-propyl(meth)acrylamide, N-(butyl)(meth)acrylamide, N-tert-butyl(meth)acrylamide, n-pentyl(meth)acrylamide, and n-octyl(meth)acrylamide.

Representative N,N-di($C_1$-$C_{10}$)alkyl (meth)acrylamides include, for example, N,N-dimethyl(meth)acrylamide, N,N-diethyl(meth)acrylamide, N-methyl,N-ethyl(meth)acrylamide, N,N-(di-2-hydroxyethyl)(meth)acrylamide, N,N-(di-3-hydroxypropyl)(meth)acrylamide, and mixtures thereof.

Representative N-($C_1$-$C_{10}$)alkylamino($C_1$-$C_{10}$)alkyl(meth)acrylamides include, but are not limited to, N-methylamino methyl (meth)acrylamide, N-methylamino ethyl (meth)acrylamide, N-ethylamino methyl (meth)acrylamide, N-ethylamino ethyl (meth)acrylamide, N-propylamino methyl (meth)acrylamide, N-propylamino ethyl (meth)acrylamide, N-butylamino methyl (meth)acrylamide, N-butylamino ethyl (meth)acrylamide, N-pentylamino methyl (meth)acrylamide, N-pentylamino ethyl (meth)acrylamide, N-methylamino ethyl (meth)acrylamide, N-methylamino propyl (meth)acrylamide, N-ethylamino ethyl (meth)acrylamide, N-ethylamino propyl (meth)acrylamide, N-propylamino ethyl (meth)acrylamide, N-propylamino propyl (meth)acrylamide, N-butylamino ethyl (meth)acrylamide, N-butylamino propyl (meth)acrylamide, N-pentylamino ethyl (meth)acrylamide, N-pentylamino propyl (meth)acrylamide, N-methylamino ethyl (meth)acrylamide, N-methylamino butyl (meth)acrylamide, N-ethylamino ethyl (meth)acrylamide, N-ethylamino butyl (meth)acrylamide, N-propylamino ethyl (meth)acrylamide, N-propylamino butyl (meth)acrylamide, N-butylamino ethyl (meth)acrylamide, N-butylamino butyl acrylamide, N-pentylamino ethyl (meth)acrylamide, and N-pentylamino butyl (meth)acrylamide, and mixtures thereof.

Representative N,N-di($C_1$-$C_{10}$)alkylamino($C_1$-$C_{10}$)alkyl(meth)acrylamides include, but are not limited to, N,N-dimethylaminoethyl(meth)acrylamide, N,N-diethylaminoethyl(meth)acrylamide, N,N-dimethylaminopropyl(meth)acrylamide, and mixtures thereof.

Monomer (h)

Representative N-vinyl amides include, for example, N-vinylformamide, N-methyl-N-vinylformamide, N-(hydroxymethyl)-N-vinylformamide, N-vinylacetamide, N-vinylmethylacetamide, N-(hydroxymethyl)-N-vinylacetamide, and mixtures thereof.

Representative N-vinyl lactams include N-vinyl-2-pyrrolidinone (N-vinyl pyrrolidone), N-(1-methyl vinyl) pyrrolidinone, N-vinyl-2-piperidone, N-vinyl-2-caprolactam, N-vinyl-5-methyl pyrrolidinone, N-vinyl-3,3-dimethyl pyrrolidinone,

N-vinyl-5-ethyl pyrrolidinone, N-vinyl-6-methyl piperidone, N-vinyl-2-caprolactam, N-vinyl-3-methyl-2-pyrrolidone, N-vinyl-3-methyl-2-piperidone, N-vinyl-3-methyl-2-caprolactam, N-vinyl-4-methyl-2-pyrrolidone, N-vinyl-4-methyl-2-caprolactam, N-vinyl-5-methyl-2-pyrrolidone, N-vinyl-5-methyl-2-piperidone, N-vinyl-5,5-dimethyl-2-pyrrolidone, N-vinyl-3,3,5-trimethyl-2-pyrrolidone, N-vinyl-5-methyl-5-ethyl-2-pyrrolidone, N-vinyl-3,4,5-trimethyl-3-ethyl-2-pyrrolidone, N-vinyl-6-methyl-2-piperidone, N-vinyl-6-ethyl-2-piperidone, N-vinyl-3,5-dimethyl-2-piperidone, N-vinyl-4,4-dimethyl-2-piperidone, N-vinyl-7-methyl-2-caprolactam, N-vinyl-7-ethyl-2-caprolactam, N-vinyl-3,5-dimethyl-2-caprolactam, N-vinyl-4,6-dimethyl-2-caprolactam, N-vinyl-3, 5,7-trimethyl-2-caprolactam, and mixtures thereof.

Monomer (i)

The associative monomer of the invention has an ethylenically unsaturated end group portion (i) for addition polymerization with the other monomers of the invention; a polyoxyalkylene mid-section portion (ii) for imparting selective hydrophilic and/or hydrophobic properties to the product polymer, and a hydrophobic end group portion (iii) for providing selective associative hydrophobic properties to the polymer.

The portion (i) supplying the ethylenically unsaturated end group can be a residue derived from an $\alpha,\beta$-ethylenically unsaturated monocarboxylic acid. Alternatively, portion (i) of the associative monomer can be a residue derived from an allyl ether or vinyl ether; a nonionic vinyl-substituted urethane monomer, such as disclosed in U.S. Reissue Patent No. 33,156 or U.S. Patent No. 5,294,692; or a vinyl-substituted urea reaction product, such as disclosed in U.S. Patent No. 5,011,978.

The mid-section portion (ii) is a polyoxyalkylene segment of 2 to 150 in one aspect, from 10 to 120 in another aspect, and from 15 to 60 in a further aspect of repeating $C_2$-$C_4$ alkylene oxide units. The mid-section portion (ii) includes polyoxyethylene, polyoxypropylene, and polyoxybutylene segments, and combinations thereof comprising from 2 to 150 in one aspect, from 5 to 120 in another aspect, and from 10 to 60 in a further aspect of ethylene, propylene and/or butylene oxide units, arranged in random or block sequences of ethylene oxide, propylene oxide and/or butylene oxide units.

The hydrophobic end group portion (iii) of the associative monomer is a hydrocarbon moiety belonging to one of the following hydrocarbon classes: a $C_8$-$C_{30}$ linear alkyl, a $C_8$-$C_{30}$ branched alkyl, a $C_2$-$C_{30}$ alkyl-substituted phenyl, aryl-substituted $C_2$-$C_{30}$ alkyl groups, a $C_7$-$C_{30}$ saturated or unsaturated carbocyclic alkyl group. The saturated or unsaturated carbocyclic moiety can be a $C_1$-$C_5$ alkyl substituted or unsubstituted monocyclic or bicyclic moiety. In one aspect the bicyclic moiety is selected from bicycloheptyl or bicycloheptenyl. In another aspect the bicycloheptenyl moiety is disubstituted with the alkyl substituent(s). In a further aspect the bicycloheptenyl moiety is disubstituted with methyl on the same carbon atom.

Non-limiting examples of suitable hydrophobic end group portions (iii) of the associative monomers are linear or branched alkyl groups having 8 to 30 carbon atoms, such as capryl ($C_8$), iso-octyl (branched $C_8$), decyl ($C_{10}$), lauryl ($C_{12}$), myristyl ($C_{14}$), cetyl ($C_{16}$), cetearyl ($C_{16}$-$C_{18}$), stearyl ($C_{18}$), isostearyl (branched $C_{18}$), arachidyl ($C_{20}$), behenyl ($C_{22}$), lignoceryl ($C_{24}$), cerotyl ($C_{26}$), montanyl ($C_{28}$), melissyl ($C_{30}$), and the like.

Examples of linear and branched alkyl groups having about 8 to about 30 carbon atoms that are derived from a natural source include, without being limited thereto, alkyl groups derived from hydrogenated peanut oil, soybean oil and canola oil (all predominately $C_{18}$), hydrogenated tallow oil ($C_{16}$-$C_{18}$), and the like; and hydrogenated $C_{10}$-$C_{30}$ terpenols, such as hydrogenated geraniol (branched $C_{10}$), hydrogenated farnesol (branched $C_{15}$), hydrogenated phytol (branched $C_{20}$), and the like.

Non-limiting examples of suitable $C_2$-$C_{30}$ alkyl-substituted phenyl groups include octylphenyl, nonylphenyl, decylphenyl, dodecylphenyl, hexadecylphenyl, octadecylphenyl, isooctylphenyl, sec-butylphenyl, and the like.

Exemplary aryl-substituted $C_2$-$C_{40}$ alkyl groups include, without limitation thereto, styryl (e.g., 2-phenylethyl), distyryl (e.g., 2,4-diphenylbutyl), tristyryl (e.g., 2,4,6-triphenylhexyl), 4-phenylbutyl, 2-methyl-2-phenylethyl, tristyrylphenolyl, and the like.

Suitable $C_7$-$C_{30}$ carbocyclic groups include, without limitation, groups derived from sterols from animal sources, such as cholesterol, lanosterol, 7-dehydrocholesterol, and the like; from vegetable sources, such as phytosterol, stigmasterol, campesterol, and the like; and from yeast sources, such as ergosterol, mycosterol, and the like. Other carbocyclic alkyl hydrophobic end groups useful in the present invention include, without limitation, cyclooctyl, cyclododecyl, adamantyl, decahydronaphthyl, and groups derived from natural carbocyclic materials, such as pinene, hydrogenated retinol, camphor, isobornyl alcohol, norbornyl alcohol, nopol and the like.

Useful associative monomers can be prepared by any method known in the art. See, for example, U.S. Patents No. 4,421,902 to Chang et al.; No. 4,384,096 to Sonnabend; No. 4,514,552 to Shay et al.; No. 4,600,761 to Ruffner et al.; No. 4,616,074 to Ruffner; No. 5,294,692 to Barron et al.; No. 5,292,843 to Jenkins et al.; No. 5,770,760 to Robinson; No. 5,412,142 to Wilkerson, III et al.; and No. 7,772,421, to Yang et al..

In one aspect, exemplary associative monomers include those represented by Formulas (V) and (VA) as follows:

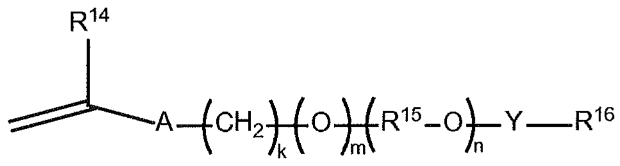

V

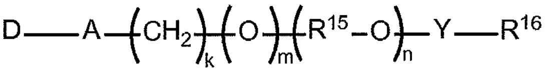

VA

wherein $R^{14}$ is hydrogen or methyl; A is $-CH_2C(O)O-$, $-C(O)O-$, $-O-$, $-CH_2O-$ ,$-NHC(O)NH-$, $-C(O)NH-$, $-Ar-(CE_2)_z-NHC(O)O-$, $-Ar-(CE_2)_z-NHC(O)NH-$ or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; $(R^{15}-O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150 in one aspect, from 10 to 120 in another aspect, and from 15 to 60 in a further aspect; Y is $-R^{15}O-$, $-R^{15}NH-$, $-C(O)-$, $-C(O)NH-$, $-R^{15}NHC(O)NH-$, $-C(O)NHC(O)-$, or a divalent alkylene radical containing 1 to 5 carbon atoms, e.g., methylene, ethylene, propylene, butylene, pentylene; $R^{16}$ is a substituted or unsubstituted alkyl selected from a $C_8$-$C_{30}$ linear alkyl, a $C_8$-$C_{30}$ branched alkyl, a $C_7$-$C_{30}$ carbocyclic, a $C_2$-$C_{30}$ alkyl-substituted phenyl, an araalkyl substituted phenyl, and an aryl-substituted $C_2$-$C_{30}$ alkyl; wherein the $R^{16}$ alkyl group, aryl group, phenyl group, or carbocyclic group optionally comprises one or more substituents selected from the group consisting of a methyl group, a hydroxyl group, an alkoxyl group, benzyl group phenylethyl group, and a halogen group. In one aspect, Y is ethylene and $R^{16}$ is

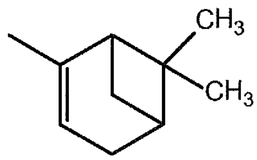

In one aspect, the hydrophobically modified associative monomer is an alkoxylated (meth)acrylate having a hydrophobic group containing 8 to 30 carbon atoms represented by the following Formula VB as follows:

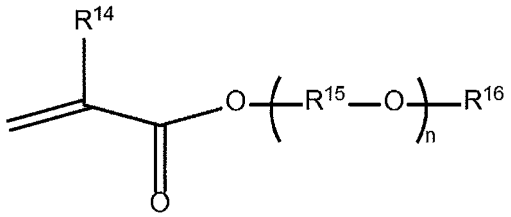

VB

wherein $R^{14}$ is hydrogen or methyl; $R^{15}$ is a divalent alkylene moiety independently selected from $C_2H_4$, $C_3H_6$, and $C_4H_8$, and n represents an integer ranging from 2 to 150 in one aspect, from 5 to 120 in another aspect, and from 10 to 60 in a further aspect, $(R^{15}-O)$ can be arranged in a random or a block configuration; $R^{16}$ is a substituted or unsubstituted alkyl selected from a $C_8$-$C_{30}$ linear alkyl, a $C_8$-$C_{30}$ branched alkyl, an alkyl substituted and unsubstituted $C_7$-$C_{30}$ carbocyclic alkyl, a $C_2$-$C_{30}$ alkyl-substituted phenyl, and an aryl-substituted $C_2$-$C_{30}$ alkyl.

Representative monomers under Formula V include lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene methacrylate, where the polyethoxylated portion of the monomer contains 2 to 150 ethylene oxide units in one aspect, from 5 to 120 in another aspect, and from 10 to 60 in a further aspect; octyloxy polyethyleneglycol (8) polypropyleneglycol (6) (meth)acrylate, phenoxy polyethylene glycol (6) polypropylene glycol (6) (meth)acrylate, and nonylphenoxy polyethylene glycol polypropylene glycol (meth)acrylate.

Monomer (j)

The semi-hydrophobic monomers of the invention are structurally similar to the associative monomer described above, but have a substantially non-hydrophobic end group portion. The semi-hydrophobic monomer has an ethylenically unsaturated end group portion (i) for addition polymerization with the other monomers of the invention; a polyoxyalkylene mid-section portion (ii) for imparting selective hydrophilic and/or hydrophobic properties to the product polymer and a semi- hydrophobic end group portion (iii). The unsaturated end group portion (i) supplying the vinyl or other ethylenically unsaturated end group for addition polymerization is preferably derived from an $\alpha,\beta$-ethylenically unsaturated mono carboxylic acid. Alternatively, the end group portion (i) can be derived from an allyl ether residue, a vinyl ether residue or a residue of a nonionic urethane monomer.

The polyoxyalkylene mid-section (ii) specifically comprises a polyoxyalkylene segment, which is substantially similar to the polyoxyalkylene portion of the associative monomers described above. In one aspect, the polyoxyalkylene portions (ii) include polyoxyethylene, polyoxypropylene, and/or polyoxybutylene units comprising from 2 to 150 in one aspect, from 5 to 120 in another aspect, and from 10 to 60 in a further aspect of ethylene oxide, propylene oxide, and/or butylene oxide units, arranged in random or blocky sequences.

The semi-hydrophobic end group portion (iii) is substantially non-hydrophobic and is selected from hydroxyl and a linear or branched $C_1$ to $C_4$ alkyl.

In one aspect, the semi-hydrophobic monomer can be represented by Formulas VI and VII as follows:

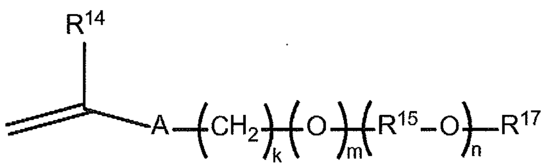

VI

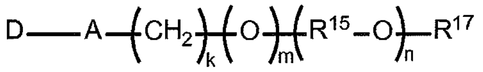

VII

wherein $R^{14}$ is hydrogen or methyl; A is $-CH_2C(O)O-$, $-C(O)O-$, $-O-$, $-CH_2O-$ ,$-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; $(R^{15}-O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150 in one aspect, from 5 to 120 in another aspect, and from 10 to 60 in a further aspect; $R^{17}$ is selected from hydrogen and a linear or branched $C_1$-$C_4$ alkyl group (e.g., methyl, ethyl, propyl, iso-propyl, butyl, iso-butyl, and tert-butyl); and D represents a vinyl or an allyl moiety.

In one aspect, the semi-hydrophobic monomer under formula VIII can be represented by Formulas VIA and VIB below:

$$CH_2=C(R^{14})C(O)O-(C_2H_4O)_a(C_3H_6O)_b-H \qquad VIA$$

$$CH_2=C(R^{14})C(O)O-(C_2H_4O)_a(C_3H_6O)_b-CH_3 \qquad VIB$$

wherein $R^{14}$ is hydrogen or methyl, and "a" is an integer ranging from 0 or 2 to 120 in one aspect, from 5 to 45 in another aspect, and from 10 to 25 in a further aspect, and "b" is an integer ranging from 0 or 2 to 120 in one aspect, from 5 to 45 in another aspect, and from 10 to 25 in a further aspect, subject to the proviso that "a" and "b" cannot be 0 at the same time.

Examples of semi-hydrophobic monomers under formula VIA include polyethyleneglycol methacrylate available under the product names Blemmer® PE-90 ($R^{14}$ = methyl, a = 2, b = 0), PE-200 ($R^{14}$ = methyl, a = 4.5, b = 0), and PE-350 ($R^{14}$ = methyl a = 8, b = 0,); polypropylene glycol methacrylate available under the product names Blemmer® PP-1000 ($R^{14}$ = methyl, b = 4-6, a = 0), PP-500 ($R^{14}$ = methyl, a = 0, b = 9), PP-800 ($R^{14}$ = methyl, a = 0, b = 13);

polyethyleneglycol polypropylene glycol methacrylate available under the product names Blemmer® 50PEP-300 ($R^{14}$ = methyl, a = 3.5, b = 2.5), 70PEP-350B ($R^{14}$ = methyl, a = 5, b = 2); polyethyleneglycol acrylate available under the product names Blemmer® AE-90 ($R^{14}$ = hydrogen, a = 2, b = 0), AE-200 ($R^{14}$ = hydrogen, a = 2, b = 4.5), AE-400 ($R^{14}$ = hydrogen, a = 10, b = 0); polypropyleneglycol acrylate available under the product names Blemmer® AP-150 ($R^{14}$ = hydrogen, a = 0, b = 3), AP-400($R^{14}$ = hydrogen, a = 0, b = 6), AP-550 ($R^{14}$ = hydrogen, a = 0, b = 9). Blemmer® is a trademark of NOF Corporation, Tokyo, Japan.

Examples of semi-hydrophobic monomers under formula VIB include methoxypolyethyleneglycol methacrylate available under the product names Visiomer® MPEG 750 MA W ($R^{14}$ = methyl, a = 17, b = 0), MPEG 1005 MA W ($R^{14}$ = methyl, a = 22, b = 0), MPEG 2005 MA W ($R^{14}$ = methyl, a = 45, b = 0), and MPEG 5005 MA W ($R^{14}$ = methyl, a = 113, b = 0) from Evonik Röhm GmbH, Darmstadt, Germany); Bisomer® MPEG 350 MA ($R^{14}$ = methyl, a = 8, b = 0), and MPEG 550 MA ($R^{14}$ = methyl, a = 12, b = 0) from GEO Specialty Chemicals, Ambler PA; Blemmer® PME-100 ($R^{14}$ = methyl, a = 2, b = 0), PME-200 ($R^{14}$ = methyl, a = 4, b = 0), PME-400 ($R^{14}$ = methyl, a = 9, b = 0), PME-1000 ($R^{14}$ = methyl, a = 23, b = 0), PME-4000 ($R^{14}$ = methyl, a = 90, b = 0).

In one aspect, the semi-hydrophobic monomer set forth in Formula VII can be represented Formulas VIA and VIB as follows:

$$CH_2{=}CH\text{-}O\text{-}(CH_2)_d\text{-}O\text{-}(C_3H_6O)_e\text{-}(C_2H_4O)_f\text{-}H \qquad \text{VIIA}$$

$$CH_2{=}CH\text{-}CH_2\text{-}O\text{-}(C_3H_6O)_g\text{-}(C_2H_4O)_h\text{-}H \qquad \text{VIIB}$$

wherein d is an integer of 2, 3, or 4; e is an integer in the range of from 1 to 10 in one aspect, from 2 to 8 in another aspect, and from 3 to 7 in a further aspect; f is an integer in the range of from 5 to 50 in one aspect, from 8 to 40 in another aspect, and from 10 to 30 in a further aspect; g is an integer in the range of from 1 to 10 in one aspect, from 2 to 8 in another aspect, and from 3 to 7 in a further aspect; and h is an integer in the range of from 5 to 50 in one aspect, and from 8 to 40 in another aspect; e, f, g, and h can be 0 subject to the proviso that e and f cannot be 0 at the same time, and g and h cannot be 0 at the same time.

Monomers under formulas VIIA and VIIB are commercially available under the trade names Emulsogen® R109, R208, R307, RAL109, RAL208, and RAL307 sold by Clariant Corporation; BX-AA-E5P5 sold by Bimax, Inc.; and combinations thereof. EMULSOGEN$_7$ R109 is a randomly ethoxylated/propoxylated 1,4-butanediol vinyl ether having the empirical formula $CH_2{=}CH\text{-}O(CH_2)_4O(C_3H_6O)_4(C_2H_4O)_{10}H$; Emulsogen® R208 is a randomly ethoxylated/propoxylated 1,4-butanediol vinyl ether having the empirical formula $CH_2{=}CH\text{-}O(CH_2)_4O(C_3H_6O)_4(C_2H_4O)_{20}H$; Emulsogen® R307 is a randomly ethoxylated/propoxylated 1,4-butanediol vinyl ether having the empirical formula $CH_2{=}CH\text{-}O(CH_2)_4O(C_3H_6O)_4(C_2H_4O)_{30}H$; Emulsogen® RAL109 is a randomly ethoxylated/propoxylated allyl ether having the empirical formula $CH_2{=}CHCH_2O(C_3H_6O)_4(C_2H_4O)_{10}H$; Emulsogen® RAL208 is a randomly ethoxylated/propoxylated allyl ether having the empirical formula $CH_2{=}CHCH_2O(C_3H_6O)_4(C_2H_4O)_{20}H$; Emulsogen® RAL307 is a randomly ethoxylated/propoxylated allyl ether having the empirical formula $CH_2{=}CHCH_2O(C_3H_6O)_4(C_2H_4O)_{30}H$; and BX-AA-E5P5 is a randomly ethoxylated/propoxylated allyl ether having the empirical formula $CH_2{=}CHCH_2O(C_3H_6O)_5(C_2H_4O)_5H$.

Monomer (k)

The silicon macromer monomer comprises (i) a polymerizable citraconate end group portion, (ii) a polyoxyalkylene mid-section portion, and (iii) a dimethicone end group portion. In one aspect, the silicone macromer is selected from at least one macromer monomer represented by Formula VIII as follows:

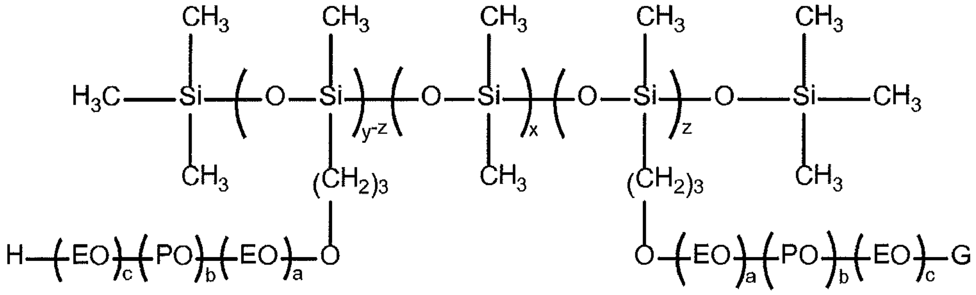

VIII

wherein EO represents a divalent ethylene oxide residue, PO independently represents a divalent propylene oxide residue; a, b, and c are independently from 0 to 100 in one aspect, from 1 to 50 in another aspect, and from 5 to 25 in a further aspect, subject to the proviso that a, b, and c cannot all be 0 at the same time; wherein the EO and PO residues

are arranged in random or blocky sequences; x is 0 to 200; y is 1 to 200; z ≤ y; and G represents a polymerizable reactive moiety represented as follows:

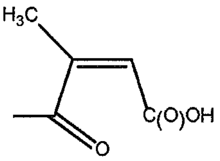
,
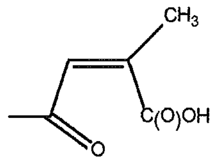
,
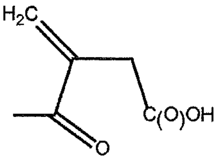
,
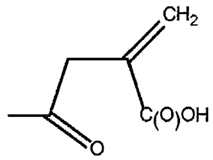

Monomer (l)

The vinyl ester of a carboxylic acid containing a acyl moiety having 3 to 22 carbon atoms is represented Formula IX as follows:

$CH_2$=CHOC(O)$R^{18}$ IX

wherein $R^{18}$ is selected from a linear or branched $C_2$-$C_{21}$ alkyl group.

Monomer (m)

The alpha-olefinic monomer is selected is represented by Formula X below:

$CH_2$=CH-$R^{19}$ X

wherein $R^{19}$ represents a moiety selected from hydrogen, a linear or branched $C_1$-$C_8$ alkyl and aryl such as phenyl, benzyl and the like.

Representative alpha-olefin monomers include, for example, ethylene, propylene, 1-butene, iso-butylene, 1-hexene, 1-heptene, 4-methyl-1-pentene, styrene, alpha-methyl styrene, and mixtures thereof.

Monomer (n)

The acrylate ester of the glycidyl ester of a linear or branched, saturated or unsaturated $C_{10}$ to $C_{22}$ fatty acid is represented by Formula XI below:

$CH_2$=C(R)C(O)OAO$R^{20}$ XI

wherein A is a divalent radical selected from -$CH_2$CH(OH)$CH_2$- and -$CH_2$CH($CH_2$OH)-, R is selected from hydrogen or methyl, and $R^{20}$ is an acyl residue of a linear or branched, saturated or unsaturated $C_{10}$ to $C_{22}$ fatty acid.

Monomer (o)

The copolymerizable sulfonic acid containing monomer is selected from styrenesulfonic acid, 2-(meth)acrylamido-2-methylpropane sulfonic acid e.g., 2-acrylamido-2-methylpropane sulfonic acid, vinylsulfonic acid, allylsulfonic acid, methallylsulfonic acid; and salts thereof.

In one embodiment, the crosslinked acrylic stabilizing/thickening polymers of the invention are prepared by polymerizing a monomer mixture comprising (a) from 10 to 35 wt. % in one aspect, from 10 to 35 wt. % in another aspect, from 15 to 35 wt. % in still another aspect, and from 20 to 35 wt. % in a further aspect, and from 25 to about 30 wt. % in a still further aspect of a monomer selected from the carboxyethyl and mixtures thereof; (b) from 30 to 70 wt. %. in one aspect, from 40 to 70 wt.% in another aspect, from 50 to 70 wt.% in still another aspect, and from 55 to 65 wt. % in a further aspect of a monomer selected from ethyl acrylate, vinyl acetate, and mixtures thereof; (c) from 0 to 40 wt.% in

one aspect, from 5 to 40 wt.% in another aspect, from 10 to 40 wt.% in still another aspect, from 15 to 35 wt. % in a further aspect, and from 20 or 25 to 30 wt. % in a still further aspect of a monomer selected form acrylic acid, methacrylic acid, and mixtures thereof; (d) from 0.001 to 5 wt.% in one aspect, from 0.05 to 3.5 wt.% in another aspect, from 0.5 to 3 wt.% in still another aspect, and from 1 to 2.5 wt. % in a further aspect of a polyunsaturated crosslinking monomer containing at least two polymerizable ethylenically unsaturated moieties; and from 0.01 to 15 wt.% in one aspect, from 0.5 to 10 wt.% in another aspect, and from 1 to 5 wt.% of at least one monomer selected from monomer(s) (e) to (o) described above, wherein the weight percent of each monomer is based on the total weight of monomers in the monomer mixture.

As one of ordinary skill in the art will readily recognize, the amounts of monomers (a) through (o) set forth herein are selected from the disclosed ranges such that the amount of each of the monomer components in the polymerizable monomer composition sums up to 100 wt.% of the polymerizable monomer mixture.

In one aspect of the invention, the crosslinked acrylic stabilizing/thickener polymers of the invention can be synthesized by free radical polymerization of the monomer mixture described above. The polymers can be prepared via solution, dispersion, mass or emulsion polymerization techniques that are well-known in the polymer art. In one aspect the present polymers are prepared by emulsion polymerization.

The emulsion polymerization can be carried out in a batch process, in a metered sequential monomer addition process (staged process), or the polymerization can be initiated as a batch process and then the bulk of the monomers can be continuously metered into the reactor (seed process). Typically, the polymerization process is carried out at a reaction temperature in the range of 20 to 99°C; however, higher or lower temperatures can be used. To facilitate emulsification of the monomer mixture, the emulsion polymerization is carried out in the presence of at least one surfactant. In one embodiment, the emulsion polymerization is carried out in the presence of surfactant ranging in the amount of 1 % to 10% by weight in one aspect, from about 3% to about 8% in another aspect, and from 3.5% to 7% by weight in a further aspect, based on a total emulsion weight basis. The emulsion polymerization reaction mixture also includes one or more free radical initiators which are present in an amount in the ranging from about 0.01% to about 3% by weight based on total monomer weight. The polymerization can be performed in an aqueous or aqueous alcohol medium.

Exemplary free radical initiators include, but are not limited to, water-soluble inorganic persulfate compounds, such as ammonium persulfate, potassium persulfate, and sodium persulfate; peroxides such as hydrogen peroxide, benzoyl peroxide, acetyl peroxide, and lauryl peroxide, cumene hydroperoxide and t-butyl hydroperoxide; organic peracids, such as peracetic acid; and oil and water soluble free radical producing agents, such as 2,2'-azobisisobutyronitrile, and the like, and mixtures thereof. Peroxides and peracids can optionally be activated with reducing agents, such as sodium bisulfite, sodium formaldehyde, or ascorbic acid, transition metals, hydrazine, and the like.

Optionally, the use of known redox initiator systems as polymerization initiators can be employed. Such redox initiator systems include an oxidant (intiator) and a reductant. Suitable oxidants include, for example, hydrogen peroxide, sodium peroxide, potassium peroxide, t-butyl hydroperoxide, t-amyl hydroperoxide, cumene hydroperoxide, sodium perborate, perphosphoric acid and salts thereof, potassium permanganate, and ammonium or alkali metal salts of peroxydisulfuric acid, typically at a level of 0.01 % to 3.0% by weight, based on dry polymer weight, are used. Suitable reductants include, for example, alkali metal and ammonium salts of sulfur-containing acids, such as sodium sulfite, bisulfite, thiosulfate, hydrosulfite, sulfide, hydrosulfide or dithionite, formadinesulfinic acid, hydroxymethanesulfonic acid, acetone bisulfite, amines such as ethanolamine, glycolic acid, glyoxylic acid hydrate, ascorbic acid, isoascorbic acid, lactic acid, glyceric acid, malic acid, 2-hydroxy-2-sulfinatoacetic acid, tartaric acid and salts of the preceding acids typically at a level of 0.01 % to 3.0% by weight, based on dry polymer weight, is used. In one aspect, combinations of peroxodisulfates with alkali metal or ammonium bisulfites can be used, for example, ammonium peroxodisulfate and ammonium bisulfite. In another aspect, combinations of hydrogen peroxide containing compounds (t-butyl hydroperoxide) as the oxidant with ascorbic or erythorbic acid as the reductant can be utilized. The ratio of peroxide-containing compound to reductant is within the range from 30:1 to 0.05:1.

If desired, the crosslinked acrylic stabilizer/thickener polymers useful in cleansing compositions of the invention can be prepared from a monomer mixture comprising one or more chain transfer agents. The chain transfer agent can be any chain transfer agent which reduces the molecular weight of the polymers of the invention. Suitable chain transfer agents include, but are not limited to, thio and disulfide containing compounds, such as $C_1$-$C_{18}$ alkyl mercaptans, mercaptocarboxylic acids, mercaptocarboxylic esters, thioesters, $C_1$-$C_{18}$ alkyl disulfides, aryldisulfides, polyfunctional thiols such as trimethylolpropane-tris-(3-mercaptopropionate), pentaerythritol-tetra-(3-mercaptopropionate), pentaerythritol-tetra-(thioglycolate), and pentaerythritoltetra-(thiolactate), dipentaerythritol-hexa-(thioglycolate), and the like; phosphites and hypophosphites; haloalkyl compounds, such as carbon tetrachloride, bromotrichloromethane, and the like; and catalytic chain transfer agents such as, for example, cobalt complexes (e.g., cobalt (II) chelates).

In one aspect of the invention, the chain transfer agent is selected from octyl mercaptan, n-dodecyl mercaptan, t-dodecyl mercaptan, hexadecyl mercaptan, octadecyl mercaptan (ODM), isooctyl 3-mercaptopropionate (IMP), butyl 3-mercaptopropionate, 3-mercaptopropionic acid, butyl thioglycolate, isooctyl thioglycolate, and dodecyl thioglycolate.

When utilized, the chain transfer agent can be present in an amount ranging from 0.1% to 10% by weight,

based on the total monomer mixture weight.

Surfactants for facilitating the emulsion polymerization include anionic, nonionic, amphoteric, and cationic surfactants, as well as mixtures thereof. Most commonly, anionic and nonionic surfactants can be utilized as emulsifying surfactants as well as mixtures thereof.

Suitable anionic surfactants for facilitating emulsion polymerizations are well known in the art and include, but are not limited to, sodium lauryl sulfate, sodium dodecyl benzene sulfonate, sodium ($C_6$-$C_{16}$) alkyl phenoxy benzene sulfonate, disodium ($C_6$-$C_{16}$) alkyl phenoxy benzene sulfonate, disodium ($C_6$-$C_{16}$) di-alkyl phenoxy benzene sulfonate, disodium laureth-3 sulfosuccinate, sodium dioctyl sulfosuccinate, sodium di-sec-butyl naphthalene sulfonate, disodium dodecyl diphenyl ether sulfonate, disodium n-octadecyl sulfosuccinate, phosphate esters of branched alcohol ethoxylates, and the like.

Nonionic surfactants suitable for facilitating emulsion polymerizations are well known in the polymer art, and include, without limitation, linear or branched $C_8$-$C_{30}$ fatty alcohol ethoxylates, such as capryl alcohol ethoxylate, lauryl alcohol ethoxylate, myristyl alcohol ethoxylate, cetyl alcohol ethoxylate, stearyl alcohol ethoxylate, cetearyl alcohol ethoxylate, sterol ethoxylate, oleyl alcohol ethoxylate, and, behenyl alcohol ethoxylate; alkylphenol alkoxylates, such as octylphenol ethoxylates; and polyoxyethylene polyoxypropylene block copolymers, and the like. Additional fatty alcohol ethoxylates suitable as non-ionic surfactants are described below. Other useful nonionic surfactants include $C_8$-$C_{22}$ fatty acid esters of polyoxyethylene glycol, ethoxylated mono- and diglycerides, sorbitan esters and ethoxylated sorbitan esters, $C_8$-$C_{22}$ fatty acid glycol esters, block copolymers of ethylene oxide and propylene oxide, and combinations thereof. The number of ethylene oxide units in each of the foregoing ethoxylates can range from 2 and above in one aspect, and from 2 to 150 in another aspect.

In emulsion polymerization processes, it can be advantageous to stabilize the monomer/polymer droplets or particles by means of surface active auxiliaries. Typically, these are auxiliary emulsifiers or protective colloids. The auxiliary emulsifiers used can be anionic, nonionic, cationic or amphoteric. Examples of anionic emulsifiers are alkylbenzenesulfonic acids, sulfonated fatty acids, sulfosuccinates, fatty alcohol sulfates, alkylphenol sulfates and fatty alcohol ether sulfates. Examples of usable nonionic emulsifiers are alkylphenol ethoxylates, primary alcohol ethoxylates, fatty acid ethoxylates, alkanolamide ethoxylates, fatty amine ethoxylates, EO/PO block copolymers and alkylpolyglucosides. Examples of cationic and amphoteric emulsifiers used are quaternized amine alkoxylates, alkylbetaines, alkylamidobetaines and sulfobetaines.

Examples of protective colloids are cellulose derivatives, polyethylene glycol, polypropylene glycol, copolymers of ethylene glycol and propylene glycol, polyvinyl acetate, partially hydrolyzed poly(vinyl acetate), poly(vinyl alcohol), polyvinyl ether, starch and starch derivatives, dextran, polyvinylpyrrolidone, polyvinylpyridine, polyethyleneimine, polyvinyl imidazole, polyvinylsuccinimide, polyvinyl-2-methylsuccinimide, polyvinyl-1,3-oxazolid-2-one, polyvinyl-2-methylimidazoline and maleic acid or anhydride copolymers. The emulsifiers or protective colloids are customarily used in concentrations from 0.05 to 20 wt.% in one aspect, from 0.2 to 10 wt.% in another aspect, and from 1 to 5 wt. % in a further aspect, based on the weight of the total monomers.

Optionally, other emulsion polymerization additives and processing aids which are well known in the emulsion polymerization art, such as, solvents, buffering agents, chelating agents, inorganic electrolytes, polymeric stabilizers, biocides, and pH adjusting agents can be included in the polymerization system.

In a typical emulsion polymerization, a mixture of the monomers is added to a first reactor under inert atmosphere to a solution of emulsifying surfactant (e.g., anionic surfactant) in water. Optional processing aids can be added as desired (e.g., auxiliary emulsifier(s)). The contents of the reactor are agitated to prepare a monomer emulsion. To a second reactor equipped with an agitator, an inert gas inlet, and feed pumps are added under inert atmosphere a desired amount of water and additional anionic surfactant and optional processing aids. The contents of the second reactor are heated with mixing agitation. After the contents of the second reactor reaches a temperature in the range of 55 to 98°C, a free radical initiator is injected into the so formed aqueous surfactant solution in the second reactor, and the monomer emulsion from the first reactor is gradually metered into the second reactor over a period typically ranging from about one half to about four hours. The reaction temperature is controlled in the range of 45 to 95°C. After completion of the monomer addition, an additional quantity of free radical initiator can optionally be added to the second reactor, and the resulting reaction mixture is typically held at a temperature of 45 to 95°C for a time period sufficient to complete the polymerization reaction to obtain an emulsion of the acrylic polymer particles.

Alternatively, successive free radical emulsion polymerization stages can be run to obtain multi-stage polymer morphologies such that successive polymer stages differ by polymer type (e.g., linear or crosslinked and/or monomer repeating unit content). In a stage where it is desired to have a linear polymer segment, the emulsion polymerizable monomer mixture will be devoid of crosslinking monomer, and in a stage where it is desired to have a crosslinked polymer segment the emulsion polymerizable monomer mixture will comprise a crosslinking monomer. In the multi-stage polymers of the invention at least one of the staged polymer segments must be crosslinked.

In one aspect, an embodiment of the present invention relates to stable, aqueous cleansing compositions comprising the crosslinked acrylic based stabilizer/thickener polymer described previously, at least one detersive surfactant and a silicone conditioning agent. The cleansing compositions comprising the crosslinked acrylic based stabilizer/thickener polymer of the invention have a desired pH range of 4 to 12 in one aspect, from 6 to 7.5 in another aspect, and from 6.5 to 7 in a further aspect. The crosslinked acrylic polymer/surfactant compositions of the invention form clear formulations while maintaining desirable stabilization and rheological properties (e.g., viscosity and yield values) with the ability to mitigate the loss of silicone deposition on keratinous substrates.

An alkaline material (neutralizing agent or pH adjusting agent) is incorporated to neutralize the polymer. Many types of alkaline neutralizing agents can be used in the present invention, including inorganic and organic bases, and combinations thereof. Examples of inorganic bases include but are not limited to the alkali metal hydroxides (especially sodium, potassium, and ammonium), and alkali metal salts of inorganic acids, such as sodium borate (borax), sodium phosphate, sodium pyrophosphate, and the like; and mixtures thereof. Examples of organic bases include, but are not limited to, triethanolamine (TEA), diisopropanolamine, triisopropanolamine, aminomethyl propanol, dodecylamine, cocamine, oleamine, morpholine, triamylamine, triethylamine, tetrakis(hydroxypropyl)ethylenediamine, L-arginine, aminomethyl propanol, tromethamine (2-amino 2-hydroxymethyl-1,3-propanediol), and PEG-15 cocamine. Alternatively, other alkaline materials can be used alone or in combination with the above-mentioned inorganic and organic bases. Such materials include surfactants, surfactant mixtures, pre-neutralized surfactants or materials that when combined in a composition containing a polymer of the invention is capable of neutralizing or partially neutralizing the carboxyl groups on the polymer backbone. Any material capable of increasing the pH of the composition is suitable.

Back Acid Formulation

The polymeric rheology modifiers of the present invention do not start to build substantial viscosity until a pH of 5 or 6 is achieved. There are some cleansing compositions, however, that require a pH of less than 6 for optimal and desired performance. This has limited the use of many rheology modifier polymers in such compositions. Additionally, it is difficult to formulate stable applications in this lower pH range.

It has been found that if these compositions are raised to a near neutral or even alkaline pH and then subsequently reduced in pH, the viscosity and yield value generally remain unchanged or often actually increase. This formulating technique is referred to as "Back Acid" thickening or "Back Acid Addition". This formulating technique broadens the scope of application of the present polymers and allows for formulation in the acidic pH regime. Additionally, the process of "Back Acid" thickening can also be used to further increase the viscosity and stability of compositions formulated in the alkaline pH regime.

The polymers of the invention can be formulated into a desired composition in any order during the formulation procedure. An alkaline material is added and mixed to increase the pH of the composition to at least 5 in one aspect, to at least 6 in another aspect, at least 6.5 in a further aspect, at least 7 in a still further aspect, and at least 8 in an additional aspect. The alkaline material can be any compound that can neutralize the polymer to the desired pH. In one aspect, the alkaline material is selected from any of the alkaline pH adjusting agents described above, such as, for example, sodium hydroxide, potassium hydroxide, triethanolamine, or a fatty acid amine neutralizing agent commonly used in personal care applications. Alternatively, other alkaline materials can be used, such as surfactants. In one aspect, the pH can be adjusted to at least 0.5, 1, 1.5, or 2 pH units above the final target pH of the composition. In another aspect, the pH can be adjusted to at least 3, 4, or even 5 pH units above the final target pH of the composition. Subsequent to the pH adjustment with the alkaline material, an acidic material is added to reduce the pH of the composition by 0.5, 1, 2, 3, 4, 5 or 6 pH units depending on the desired target pH for the composition. In one aspect of the invention, the target pH ranges from 3.5 to about 6, from 4 to 5.5 in another aspect, and from 4.5 to 5 in a further aspect.

The material used to decrease the pH of the composition can be any acidic material. In one aspect, the acidic material is selected from an organic acid, such as citric acid, acetic acid, alpha-hydroxy acid, beta-hydroxy acid, salicylic acid, lactic acid, glycolic acid, natural fruit acids, or combinations thereof. In addition, inorganic acids, for example, hydrochloric acid, nitric acid, sulfuric acid, sulfamic acid, phosphoric acid, and combinations thereof can be utilized. Mixtures of organic acids and inorganic acids are also contemplated.

The amount of crosslinked acrylic based stabilizer/thickener polymer that can be employed in the cleansing compositions can be determined by person skilled in the formulation art. Thus, as long as the physicochemical and functional properties of a desired product are achieved, a useful amount of polymer on a total composition weight basis, typically can vary in the range of from 0.01 to 25 wt.% in one aspect, from 0.1 to 15 wt.% in another aspect, from 0.5 to 10 wt.% in a further aspect, and from 1 to 5 wt.%t in a still further aspect (all polymer weights based on 100 % active polymer solids).

Detersive Surfactants

The cleansing composition of the present invention includes a detersive surfactant component. The detersive surfactant component is included to provide cleansing performance to the composition. The detersive surfactant component in turn includes anionic detersive surfactant, zwitterionic or amphoteric detersive surfactant, or a combination thereof. Such surfactants should be physically and chemically compatible with the other components contained in the cleansing composition, or should not otherwise unduly impair product stability, aesthetics or performance. The concentration of the active detersive surfactant component in the composition should be sufficient to provide the desired cleansing and lather performance, and generally ranges from 5 to 50 wt.% in one aspect, from 6 to 40 wt.% in another aspect, from 8 to 25 wt.% in still another aspect, from 10 to 20 wt.% in a further aspect, and from 12 to 16, based on total composition weight.

Suitable anionic detersive surfactant components for use in the hair conditioning shampoo composition include those which are known for use in hair care or other personal care cleansing compositions.

Exemplary anionic surfactants suitable for use in the compositions are the alkyl and alkyl ether sulfates. These materials have the respective formula $R^{25}OSO_3M$ and $R^{25}O(C_2H_4O)_x\ SO_3M$, wherein $R^{25}$ is alkyl or alkenyl of from about 8 to about 18 carbon atoms, x is an integer having a value of from 1 to 10, and M is a cation such as ammonium, alkanolamines, such as triethanolamine, monovalent metals, such as sodium and potassium, and polyvalent metal cations, such as magnesium, and calcium.

In one aspect, $R^{25}$ contains from 8 to 18 carbon atoms, in another aspect from 10 to 16 carbon atoms, and in a further aspect from 12 to 14 carbon atoms, in both the alkyl and alkyl ether sulfates set forth in the formulas above. The alkyl ether sulfates are typically made as condensation products of ethylene oxide and monohydric alcohols having from 8 to 24 carbon atoms. The alcohols can be synthetic or they can be derived from fats, e.g., coconut oil, palm kernel oil, and tallow. In one aspect the straight chain alcohols are derived from lauryl alcohol, coconut oil or palm kernel oil. Such alcohols are reacted with between 0 and 10 in one aspect, from 1 to 5 in another aspect, and 3 in a further aspect, molar proportions of ethylene oxide, and the resulting mixture of molecular species having, for example, an average of 3 moles of ethylene oxide per mole of alcohol, is sulfated and neutralized.

Other suitable anionic detersive surfactants are the water-soluble salts of organic, sulfuric acid reaction products conforming to the formula $R^{26}$-$SO_3$-M where $R^{26}$ is a straight or branched chain, saturated, aliphatic hydrocarbon radical having from 8 to 24 carbon atoms in one aspect, and from 10 to 18 carbon atoms in another aspect; and M is a cation described above.

Other suitable anionic detersive surfactants include olefin sulfonates containing from 10 to 24 carbon atoms. In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process. A non-limiting example of such an alpha-olefin sulfonate mixture is described in U.S. Pat. No. 3,332,880.

Another class of anionic detersive surfactants suitable for use in the compositions are the beta-alkyloxy alkane sulfonates. These surfactants conform to the formula $R^{27}CH(OR^{28})CH_2SO_3M$ where $R^{27}$ is a linear chain alkyl group containing from 6 to 20 carbon atoms, $R^{28}$ is a lower alkyl group containing from 1 to 3 carbon atoms, and M is as described above.

Other classes of anionic detersive surfactants include alkylamide sulphonates, alkarylpolyether sulphates, alkylamidoether sulphates, alkyl monoglyceryl ether sulfates, alkyl monoglyceride sulfates, alkyl monoglyceride sulfonates, alkyl succinates, alkyl sulfosuccinates, alkyl sulfosuccinamates, alkyl ether sulphosuccinates, alkyl amidosulfosuccinates; alkyl sulphoacetates, alkyl phosphates, alkyl ether phosphates, alkyl ether carboxylates, alkyl amidoethercarboxylates, N-alkylamino acids, N-acyl amino acids, alkyl peptides, N-acyl taurates, alkyl isethionates, carboxylate salts wherein the acyl group is derived from fatty acids; and the alkali metal, alkaline earth metal, ammonium, amine, and triethanolamine salts thereof. In one aspect, the cation moiety of the forgoing salts can be selected from sodium, potassium, magnesium, ammonium, mono-, di- and triethanolamine salts, and mono-, di-, and tri-isopropylamine salts. The alkyl and acyl groups of the foregoing surfactants contain from 6 to 24 carbon atoms in one aspect, from 8 to 22 carbon atoms in another aspect and from 12 to 18 carbon atoms in a further aspect and may be unsaturated. The aryl groups in the surfactants can be selected from phenyl or benzyl.

Examples of suitable anionic surfactants include sodium, potassium, lithium, magnesium, and ammonium salts of laureth sulfate, trideceth sulfate, myreth sulfate, $C_{12}$-$C_{13}$ pareth sulfate, $C_{12}$-$C_{14}$ pareth sulfate, and $C_{12}$-$C_{15}$ pareth sulfate, ethoxylated with 1, 2, and 3 moles of ethylene oxide; sodium, potassium, lithium, magnesium, ammonium, and triethanolamine lauryl sulfate, coco sulfate, tridecyl sulfate, myrstyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium $C_{12}$-$C_{14}$ olefin sulfonate, sodium laureth-6 carboxylate, sodium methyl cocoyl taurate, sodium cocoyl glycinate, sodium myristyl sarcocinate, sodium dodecylbenzene sulfonate, sodium cocoyl sarcosinate, sodium cocoyl glutamate, potassium myristoyl glutamate, triethanolamine monolauryl phosphate, and fatty acid soaps, including the sodium, potassium, ammonium, and triethanolamine salts of a saturated and unsaturated fatty acids containing from 8 to 22 carbon atoms; and combinations thereof.

When utilizing fatty acid soaps as the anionic surfactant the pH of the composition should be formulated at a pH above 7.8 in one aspect, above 8.0 in another aspect, above 8.5, 9, 9.5, 10 or 10.5 in a further aspect. If desired,

the composition can be back-acid treated with an acidic pH adjusting agent so long as the final pH of the composition does not go below 7.8.

Suitable amphoteric or zwitterionic detersive surfactants for use in the composition include those which are known for use in hair care or other personal care cleansing applications. The concentration of such amphoteric or zwitterionic detersive surfactants ranges from 0.5 to 20 wt.% in one aspect, and from 1 to 10 wt.% in another aspect. Non-limiting examples of suitable zwitterionic or amphoteric surfactants are described in U.S. Pat. No. 5,104,646 and U.S. Pat. No. 5,106,609.

Zwitterionic detersive surfactants suitable for use in the compositions are well known in the art, and include those surfactants that contain a cationic moiety and anionic moiety such as surfactants derived from amino acids (e.g., N-alkyl amino acids and N-acyl amino acids). Another class of zwitterionic detersive surfactants are derivatives of aliphatic quaternary ammonium, phosphonium, and sulfonium compounds, in which the aliphatic radicals can be straight or branched chain, and wherein one of the aliphatic substituents contains from 8 to 18 carbon atoms and one contains an anionic group such as carboxy, sulfonate, sulfate, phosphate or phosphonate. Exemplary zwitterionic surfactants in this class include betaine and sultaine surfactants

Amphoteric detersive surfactants suitable for use in the composition are well known in the art, and include those surfactants broadly described as derivatives of aliphatic secondary and tertiary amines in which the aliphatic radical can be straight or branched chain and wherein one of the aliphatic substituents contains from 8 to 18 carbon atoms and one contains an anionic group such as carboxy, sulfonate, sulfate, phosphate, or phosphonate. Exemplary amphoteric detersive surfactants for use in the present invention include cocoamphoacetate, cocoamphodiacetate, lauroamphoacetate, lauroamphodiacetate, and mixtures thereof.

Amino acid based surfactants suitable in the practice of the present invention include surfactants represented by the formula:

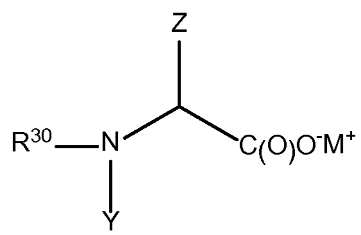

wherein $R^{30}$ represents a saturated or unsaturated hydrocarbon group having 10 to 22 carbon atoms or an acyl group containing a saturated or unsaturated hydrocarbon group having 9 to 22 carbon atoms, Y is hydrogen or methyl, Z is selected from hydrogen, $-CH_3$, $-CH(CH_3)_2$, $-CH_2CH(CH_3)_2$, $-CH(CH_3)CH_2CH_3$, $-CH_2C_6H_5$, $-CH_2C_6H_40H$, $-CH_2OH$, $-CH(OH)CH_3$, $-(CH_2)_4NH_2$, $-(CH_2)_3NHC(NH)NH_2$, $-CH_2C(O)O^-M^+$, $-(CH_2)_2C(O)O^-M^+$. M is a salt forming cation. In one aspect, $R^{30}$ represents a radical selected from a linear or branched $C_{10}$ to $C_{22}$ alkyl group, a linear or branched $C_{10}$ to $C_{22}$ alkenyl group, an acyl group represented by $R^{31}C(O)$-, wherein $R^{31}$ is selected from a linear or branched $C_9$ to $C_{22}$ alkyl group, a linear or branched $C_9$ to $C_{22}$ alkenyl group. In one aspect, $M^+$ is selected from sodium, potassium, ammonium, and triethanolamine (TEA).

The amino acid surfactants can be derived from the alkylation and acylation of $\alpha$-amino acids such as, for example, alanine, arginine, aspartic acid, glutamic acid, glycine, isoleucine, leucine, lysine, phenylalanine, serine, tyrosine, and valine. Representative N-acyl amino acid surfactants are, but not limited to the mono- and di- carboxylate salts (e.g., sodium, potassium, ammonium and TEA) of N-acylated glutamic acid, for example, sodium cocoyl glutamate, sodium lauroyl glutamate, sodium myristoyl glutamate, sodium palmitoyl glutamate, sodium stearoyl glutamate, disodium cocoyl glutamate, disodium stearoyl glutamate, potassium cocoyl glutamate, potassium lauroyl glutamate, and potassium myristoyl glutamate; the carboxylate salts (e.g., sodium, potassium, ammonium and TEA) of N-acylated alanine, for example, sodium cocoyl alaninate, and TEA lauroyl alaninate; the carboxylate salts (e.g., sodium, potassium, ammonium and TEA) of N-acylated glycine, for example, sodium cocoyl glycinate, and potassium cocoyl glycinate; the carboxylate salts (e.g., sodium, potassium, ammonium and TEA) of N-acylated sarcosine, for example, sodium lauroyl sarcosinate, sodium cocoyl sarcosinate, sodium myristoyl sarcosinate, sodium oleoyl sarcosinate, and ammonium lauroyl sarcosinate; and mixtures of the foregoing surfactants.

The betaines and sultaines useful in the present invention are selected from alkyl betaines, alkylamino betaines, and alkylamido betaines, as well as the corresponding sulfobetaines (sultaines) represented by the formulas:

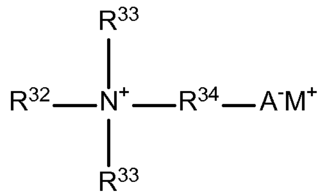

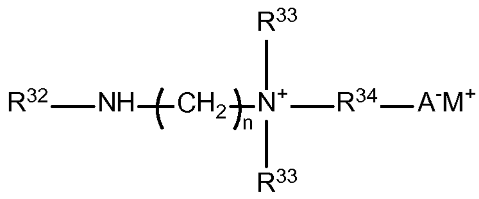

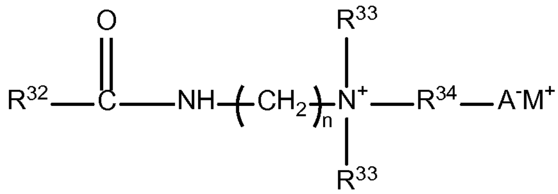

wherein $R^{32}$ is a $C_7$-$C_{22}$ alkyl or alkenyl group, each $R^{33}$ independently is a $C_1$-$C_4$ alkyl group, $R^{34}$ is a $C_1$-$C_5$ alkylene group or a hydroxy substituted $C_1$-$C_5$ alkylene group, n is an integer from 2 to 6, A is a carboxylate or sulfonate group, and M is a salt forming cation. In one aspect, $R^{32}$ is a $C_{11}$-$C_{18}$ alkyl group or a $C_{11}$-$C_{18}$ alkenyl group. In one aspect, $R^{33}$ is methyl. In one aspect, $R^{34}$ is methylene, ethylene or a hydroxyl substituted propylene moiety. In one aspect, n is 3. In a further aspect, M is selected from sodium, potassium, magnesium, ammonium, and mono-, di- and triethanolamine cations.

Examples of suitable betaines include, but are not limited to, lauryl betaine, coco betaine, oleyl betaine, cocohexadecyl dimethylbetaine, lauryl amidopropyl betaine, cocoamidopropyl betaine, and cocamidopropyl hydroxysultaine.

The alkylamphocarboxylates such as the alkylamphoacetates and alkylamphopropionates (mono- and disubstituted carboxylates) can be represented by the formula:

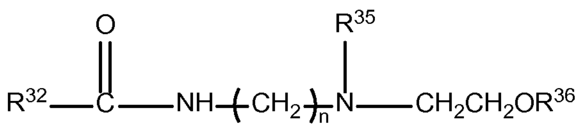

wherein $R^{32}$ is a $C_7$-$C_{22}$ alkyl or alkenyl group, $R^{35}$ is $-CH_2C(O)O^-M^+$, $-CH_2CH_2C(O)O^-M^+$, or $-CH_2CH(OH)CH_2SO_3^- M^+$, $R^{36}$ is a hydrogen or $-CH_2C(O)O^-M^+$, and M is a cation selected from sodium, potassium, magnesium, ammonium, and mono-, di- and triethanolamine, and n is an integer from 2 to 6.

The compositions of the present invention can further comprise additional surfactants for use in combination with the anionic detersive surfactant component described hereinbefore. Suitable optional surfactants include nonionic and cationic surfactants. Any such surfactant known in the art for use in hair or personal care products may be used, provided that the optional additional surfactant is also chemically and physically compatible with the essential components of the composition, or does not otherwise unduly impair product performance, aesthetics or stability. The concentration of the optional additional surfactants in the composition may vary with the cleansing or lather performance desired, the optional surfactant selected, the desired product concentration, the presence of other components in the composition, and other factors well known in the art.

Non-limiting examples of other anionic, zwitterionic, amphoteric or optional additional surfactants suitable for use in the compositions are described in McCutcheon's, Emulsifiers and Detergents, 2003 Annual, published by M. C. Publishing Co., and U.S. Pat. Nos. 3,929, 678, 2,658,072; 2,438,091; 2,528,378.

When mixtures of anionic and amphoteric surfactants are used, the weight ratio of active anionic surfactant to active amphoteric surfactant can range from 1:1 to 10:1 in one aspect, from 2.25:1 to 9:1 in another aspect, and from 4.5:1 to 7:1 in a further aspect.

Auxiliary Surfactant

Optionally, a non-ionic surfactant may be utilized in combination with the anionic or the anionic and amphoteric detersive surfactant package of the invention. The nonionic surfactant can be any of the nonionic surfactants known or previously used in the art of aqueous surfactant compositions. Suitable nonionic surfactants include, but are not limited to, aliphatic ($C_6$-$C_{18}$) primary or secondary linear or branched chain acids, alcohols or phenols; alkyl ethoxylates; alkyl phenol alkoxylates (especially ethoxylates and mixed ethoxy/propoxy moieties); block alkylene oxide condensates of alkyl phenols; alkylene oxide condensates of alkanols; and ethylene oxide/propylene oxide block copolymers. Other suitable nonionic surfactants include mono- or dialkyl alkanolamides; alkyl polyglucosides (APGs); sorbitan fatty acid esters; polyoxyethylene sorbitan fatty acid esters; polyoxyethylene sorbitol esters; polyoxyethylene acids, and polyoxyethylene alcohols. Other examples of suitable nonionic surfactants include coco mono- or diethanolamide, coco glucoside, decyl diglucoside, lauryl diglucoside, coco diglucoside, polysorbate 20, 40, 60, and 80, ethoxylated linear alcohols, cetearyl alcohol, lanolin alcohol, stearic acid, glyceryl stearate, PEG-100 stearate, laureth 7, and oleth 20.

In another aspect, non-ionic surfactants include, but are not limited to, alkoxylated methyl glucosides such as, for example, methyl gluceth-10, methyl gluceth-20, PPG-10 methyl glucose ether, and PPG-20 methyl glucose ether, available from Lubrizol Advanced Materials, Inc., under the trade names, Glucam® E10, Glucam® E20, Glucam® P10, and Glucam® P20, respectively; and hydrophobically modified alkoxylated methyl glucosides, such as PEG-120 methyl glucose dioleate, PEG-120 methyl glucose trioleate, and PEG-20 methyl glucose sesquistearate, available from Lubrizol Advanced Materials, Inc., under the trade names, Glucamate® DOE-120, Glucamate™ LT, and Glucamate™ SSE-20, respectively, are also suitable. Other exemplary hydrophobically modified alkoxylated methyl glucosides are disclosed in United States Patent Nos. 6,573,375 and 6,727,357.

When utilized in combination with the anionic or the anionic and amphoteric detersive surfactant systems of the invention, the amount of active non-ionic surfactant ranges from 0.1 to 15 wt.% in one aspect, from 0.5 to 10 wt. % in another aspect, and from 1 to 5 wt.%, based on the total weight of the composition.

In one aspect of the invention, the cleansing composition of the invention comprises a crosslinked acrylic based stabilizer/thickener polymer in combination with at least one anionic surfactant and at least one silicone conditioning agent. In another aspect of the invention, the cleansing composition comprises a crosslinked acrylic based stabilizer/thickener polymer with at least one anionic surfactant and at least one amphoteric surfactant, and at least one silicone conditioning agent. In one aspect, the anionic surfactant is selected from alkyl sulfates, alkyl ether sulfates, alkyl sulphonates, alkaryl sulfonates, alkarylpolyether sulphates, and mixtures thereof wherein the alkyl group contains 10 to 18 carbon atoms, the aryl group is a phenyl, and the ether group contains 1 to 10 moles of ethylene oxide. Representative anionic surfactants include, but are not limited to, sodium and ammonium lauryl ether sulfate (ethoxylated with 1, 2, and 3 moles of ethylene oxide), sodium, ammonium, and triethanolamine lauryl sulfate.

Silicones

The cleansing composition of the present invention further includes a silicone conditioning agent in the form of silicone particles. The silicone conditioning agent is intermixed in the composition so as to be in the form of dispersed, insoluble particles or droplets. In one aspect of the invention the silicone oil can be in the form of pre-formed emulsified droplets or microemulsion.

The silicone conditioning agent may comprise volatile silicones, non-volatile silicones, and mixtures thereof. If volatile silicones are present, they are typically employed as a solvent or carrier for commercially available forms of non-volatile silicone fluid conditioning agents such as oils and gums. Volatile silicone fluids are often included in the conditioning package to improve silicone fluid deposition efficacy or to enhance the shine, sheen or glossiness of the hair. Volatile silicone materials are frequently included in formulations to enhance sensory attributes (e.g., feel) on the scalp and skin.

In one aspect, the silicone conditioning agent is non-volatile and insoluble in the aqueous personal care cleansing composition and includes silicone oils, gums, resins and mixtures thereof. By non-volatile is meant that the silicone has a very low vapor pressure at ambient temperature conditions (e.g., less than 267 Pa (2 mm Hg) at 20°C). The non-volatile silicone conditioning agent has a boiling point above 250°C in one aspect, above 260°C in another aspect, and above 275°C in a further aspect. Background information on silicones including sections discussing silicone oils, gums, and resins, as well as their manufacture, are found in Encyclopedia of Polymer Science and Engineering, vol. 15, 2d ed., pp 204-308, John Wiley & Sons, Inc. (1989).

The total concentration of silicone particles in the compositions of the present invention should be sufficient to provide the desired conditioning performance to the skin and hair, and generally ranges from 0.01 to 20 wt.% in one aspect, from 0.05 to 15 wt. % in another aspect, from 0.1 % to 10 wt.% in still another aspect, and from 1 to 5 wt.% in a further aspect, based on the weight of the total composition.

The silicones used in the present invention have an average particle size or droplet size ranging from 0.003 to 500 μm in a first aspect, from 0.05 to 200 μm in a second aspect, from 0.25 to 200 μm in a third aspect, from 0.5 to 150

μm in a fourth aspect, from 1 to 100 μm in a fifth aspect, from 5 to 80 μm in a sixth aspect, from 10 to 60 μm in an seventh aspect, and from 20 to 50 μm in an eighth aspect.

Silicone emulsions have an average silicone particle (droplet) size of less than 30 μm, less than 20 μm in another aspect, and less than 10 μm in a further aspect. In another aspect of the invention, the average silicone particle size of the silicone emulsion is less than 2 μm, and in another it ranges from 0.01 to 1 μm. Silicone emulsions having an average silicone particle (droplet) size of 0.15 μm or less are generally termed microemulsions and generally have an average particle size ranging from 0.003 to 0.15 μm.

The average particle size of the silicone conditioning agent particles can be measured by light scattering techniques well-known in the art for determining average particle size for emulsified liquids. One such method involves measuring particle size by means of a laser light scattering technique using a Horiba model LA 910 laser scattering particle size distribution analyzer (Horiba Instruments, Inc., Irvine, California.).

Silicone Oils

In one aspect, the silicone conditional agent is silicone oil. In one aspect the silicone oil is a polyorganosiloxane material. The non-volatile silicone conditioning agents have a viscosity ranging from about above 25 to 1,000,000 mPa·s at 25°C in one aspect, from 100 to 600,000 mPa·s in another aspect, and from 1000 to 100,000 mPa·s still another aspect, from 2,000 to 50,000 mPa·s in yet another aspect, and from 4,000 to 40,000 mPa·s in a further aspect. The viscosity is measured by means of a glass capillary viscometer as described by Dow Corning Corporate Test Method CTM004, dated July 20, 1970. In one aspect the silicone oils have an average molecular weight below 200,000 daltons. The average molecular weight can typically range from 400 to 199,000 daltons in one aspect, from 500 to 150,000 daltons in another aspect, from 1,000 to 100,000 daltons in still another aspect, from 5,000 to 65,000 daltons in a further aspect.

In one aspect, silicone oils suitable as conditioning agents are polyorganosiloxane materials selected from polyalkylsiloxanes, polyarylsiloxanes, polyalkylarylsiloxanes, hydroxyl terminated polyalkylsiloxanes, polyarylalkylsiloxanes, amino functional polyalkylsiloxanes, quaternary functional polyalkylsiloxanes, and mixtures thereof.

In one aspect, the silicone conditioning agent includes polyorganosiloxanes represented by Formula XII:

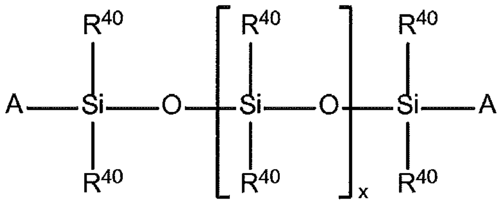

XII

wherein A independently represents hydroxy, methyl, methoxy, ethoxy, propoxy, and phenoxy; $R^{40}$ independently represents methyl, ethyl, propyl, phenyl, methylphenyl, phenylmethyl, a primary, secondary or tertiary amine, a quaternary group selected from a group selected from:

$-R^{41}-N(R^{42})CH_2CH_2N(R^{42})_2$;

$-R^{41}-N(R^{42})_2$;

$-R^{41}-N^+(R^{42})_3CA^-$;

and

$-R^{41}-N(R^{42})CH_2CH_2N(R^{42})H_2CA^-$

wherein $R^{41}$ is a linear or branched, hydroxyl substituted or unsubstituted alkylene or alkylene ether moiety containing 2 to 10 carbon atoms; $R^{42}$ is hydrogen, $C_1$-$C_{20}$ alkyl (e.g, methyl), phenyl or benzyl; q is an integer ranging from 2 to 8; $CA^-$ is a halide ion selected from chlorine, bromine, iodine and fluorine; and x is an integer ranging from 7 to 8000 in one aspect, from 50 to 5000 in another aspect, form 100 to 3000 in still another aspect, and from 200 to 1000 in a further aspect.

In one aspect, the amino functional silicone is represented by Formula XIIA:

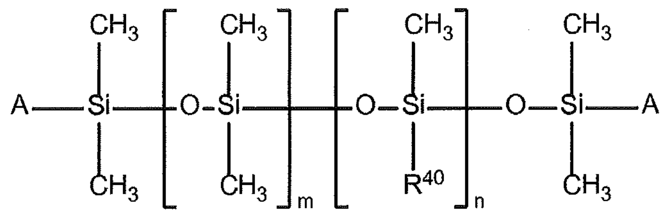 XIIA

wherein A independently represents hydroxy, methyl, methoxy, ethoxy, propoxy, and phenoxy; and $R^{40}$ is selected from:

$-R^{41}-N(R^{42})CH_2CH_2N(R^{42})_2$;

$-R^{41}-N(R^{42})_2$;

$-R^{41}-N^+(R^{42})_3CA^-$;

and

$-R^{41}-N(R^{42})CH_2CH_2N(R^{42})H_2CA^-$

wherein $R^{41}$ is a linear or branched, hydroxyl substituted or unsubstituted alkylene or alkylene ether moiety containing 2 to 10 carbon atoms; $R^{42}$ is hydrogen, $C_1$-$C_{20}$ alkyl (e.g, methyl), phenyl or benzyl; $CA^-$ is a halide ion selected from chlorine, bromine, iodine and fluorine; and the sum of m+n ranges from 7 to 1000 in one aspect, from 50 to 250 in another aspect, and from 100 to 200 in another aspect, subject to the proviso that m or n is not 0. In one aspect A is hydroxy and $R^{40}$ is-$(CH_2)_3NH(CH_2)_3NH_2$. In another aspect A is methyl and $R^{40}$ is-$(CH_2)_3NH(CH_2)_3NH_2$. In still another aspect A is methyl and $R^{40}$ is a quaternary ammonium moiety represented by $-(CH_2)_3OCH_2CH(OH)CH_2N^+(R^{42})_3$ $CA^-$; wherein $R^{42}$ and $CA^-$ are as previously defined.

Exemplary silicone oil conditioning agents include, but are not limited to, polydimethylsiloxanes (dimethicones), polydiethylsiloxanes, polydimethyl siloxanes having terminal hydroxyl groups (dimethiconols), polymethylphenylsiloxanes, phenylmethylsiloxanes, amino functional polydimethylsiloxanes (amodimethicones), and mixtures thereof.

Silicone Gums

Another silicone conditioning agent useful in the invention is a silicone gum. A silicone gum is a polyorganosiloxane material of the same general structure of the silicone oils set forth under Formula XII wherein A independently represents hydroxy, methyl, methoxy, ethoxy, propoxy, and phenoxy; $R^{40}$ independently represents methyl, ethyl, propyl, phenyl, methylphenyl, phenylmethyl, and vinyl. Silicone gums have a viscosity measured at 25°C of greater than 1,000,000 mPa·s. The viscosity can be measured by means of a glass capillary viscometer as described above for the silicone oils. In one aspect the silicone gums have an average molecular weight 200,000 daltons and above. The molecular weight can typically range from 200,000 to 1,000,000 daltons. It is recognized that the silicone gums described herein can also have some overlap with the silicone oils described previously. This overlap is not intended as a limitation on any of these materials.

Suitable silicone gums for use in the silicone component of compositions of the invention are polydimethylsiloxanes (dimethicones), optionally having terminal end groups such as hydroxyl (dimethiconols), polymethylvinylsiloxane, polydiphenylsiloxane, and mixtures thereof.

Silicone Resins

Silicone resins can be included as a silicone conditioning agent suitable for use in the compositions of the present invention. These resins are crosslinked polysiloxanes. The crosslinking is introduced through the incorporation of trifunctional and tetrafunctional silanes with monofunctional and/or difunctional silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetra-functional siloxane monomer units (and hence, a sufficient level of crosslinking) such that they form a rigid or hard film are considered to be silicone resins. The ratio of oxygen atoms to silicon atoms is indicative of the level of crosslinking in a particular silicone material. Silicone materials which have at least 1.1 oxygen atoms per silicon atom will generally be silicone resins herein. In one aspect, the ratio of oxygen:silicon atoms is at least 1.2:1.0. Silanes used in the manufacture of silicone resins include monomethyl-, dimethyl-, trimethyl-,

monophenyl-, diphenyl-, methylphenyl-, monovinyl-, and methylvinyl-chlorosilanes, and terachlorosilane, with the methyl substituted silanes being most commonly utilized.

Silicone materials and silicone resins can be identified according to a shorthand nomenclature system known to those of ordinary skill in the art as "MDTQ" nomenclature. Under this naming system, the silicone is described according to the presence of various siloxane monomer units which make up the silicone. The "MDTQ" nomenclature system is described in the publication entitled "Silicones: Preparation, Properties and Performance"; Dow Corning Corporation, 2005, and in U.S. Pat. No. 6,200,554.

Exemplary silicone resins for use in the compositions of the present invention include, but are not limited to MQ, MT, MTQ, MDT and MDTQ resins. In one aspect, methyl is the silicone resin substituent. In another aspect, the silicone resin is selected from a MQ resins, wherein the M:Q ratio is from 0.5:1.0 to 1.5:1.0 and the average molecular weight of the silicone resin is from 1000 to 10,000 daltons.

Volatile Silicones

The optional volatile silicones referred to above include linear and cyclic polydimethylsiloxanes (cyclomethicones), and mixtures thereof. The term "volatile" means that the silicone has a measurable vapor pressure, or a vapor pressure of at least 267 Pa (2 mm Hg) at 20°C. The volatile silicones have a viscosity of 25 mPa·s or less at 25°C in one aspect, from 0.65 to 10 mPa·s in another aspect, from 1 to 5 mPa·s in still another aspect, and from 1.5 to 3.5 mPa·s in a further aspect. A description of linear and cyclic volatile silicones is found in Todd and Byers, "Volatile Silicone Fluids for Cosmetics", Cosmetics and Toiletries, Vol. 91(1), pp. 27-32 (1976), and in Kasprzak, "Volatile Silicones", Soap/Cosmetics/Chemical Specialities, pp. 40-43 (December 1986).

The linear volatile silicones are silicone fluids, as described above in Formula XII but having viscosities of not more than 25 mPa·s. The cyclomethicones typically contain 3 to 7 dimethyl substituted silicon atoms in one aspect and from 3 to 5 in another aspect, alternating with oxygen atoms, in a cyclic ring structure.

Water

The cleansing compositions of the invention are aqueous based systems comprising water as the carrier. The exact level of water will vary with the levels of the remaining components formulated into the composition. Generally, the cleansing compositions of the invention comprise from 10 to 95 wt.% in one aspect, from 50 to 92 wt.% in another aspect, and from 60 to 90 wt.% water in a further aspect.

Optional Components

In addition to the components described above, the cleansing compositions may further comprise one or more optional components that are known or otherwise suitable for use on the hair, scalp or skin. Non-limiting examples of such optional components are disclosed in the International Cosmetic Ingredient Dictionary, Fifth Edition, 1993, and the Cosmetic, Toiletry, and Fragrance Association (CTFA) Cosmetic Ingredient Handbook, Second edition, 1992, each of which are incorporated by reference. Exemplary optional components are disclosed below.

Conditioning Oils

A further component that may be used in the compositions of the invention is a conditioning oil (other than a silicone) selected from a hydrocarbon oil or an ester oil. These auxiliary conditioning agent materials may enhance the conditioning benefits of the silicone materials used in the cleansing compositions of the invention.

Suitable hydrocarbon oils have at least 12 carbon atoms, and include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched chain isomers of these compounds, as well as of higher chain length hydrocarbons, can also be used. Also suitable are polymeric hydrocarbons of $C_2$-$C_6$ alkenyl monomers, such as polyisobutylene.

Suitable ester oils have at least 10 carbon atoms, and include esters with hydrocarbyl chains derived from fatty acids or alcohols. In one aspect the ester oils conform to the formula R'C(O)OR in which R' and R independently represent alkyl or alkenyl radicals and the sum of carbon atoms in R' and R is at least 10 in one aspect, and at least 20 in another aspect. Dialkyl and trialkyl and alkenyl esters of polycarboxylic acids can also be used.

In another aspect ester oils are fatty esters of mono-, di- and triglycerides, more specifically the mono-, di-, and tri-esters of glycerol derived from long chain carboxylic acids such as $C_1$-$C_{22}$ carboxylic acids. Examples of such materials include cocoa butter, palm stearin, sunflower oil, soybean oil and coconut oil.

Mixtures of any of the above described hydrocarbon and ester oils also can be used. The total combined amount

of hydrocarbon oil and/or ester oil in compositions of the invention may suitably range from 0.05 to 10 wt.% in one aspect, from 0.2 to 5 wt.%, and especially from 0.5 to 3 wt.% based on the weight of the total composition.

Cationic Polymers

Cationic polymers are components that can enhance the delivery of conditioning agents and/or provide auxiliary conditioning benefits to the hair, scalp or skin to improve and enhance the conditioning benefits delivered by the silicone conditioning agents of the invention. Cationic polymer refers to polymers containing at least one cationic moiety or at least one moiety that can be ionized to form a cationic moiety. Typically these cationic moieties are nitrogen containing groups such as quaternary ammonium or protonated amino groups. The cationic protonated amines can be primary, secondary, or tertiary amines. The cationic polymer typically has a cationic charge density ranging from 0.2 to 7 meq/g at the pH of the intended use of the composition. The average molecular weight of the cationic polymer ranges from 5,000 daltons to 10,000,000 daltons.

Non-limiting examples of such polymers are described in the CTFA International Cosmetic Ingredient Dictionary/Handbook via the CTFA website as well as the CTFA Cosmetic Ingredient Handbook, Ninth Ed., Cosmetic and Fragrance Assn., Inc., Washington D.C. (2002), can be used.

Non-limiting examples of suitable cationic polymers include copolymers of vinyl monomers having cationic protonated amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone or vinyl pyrrolidone.

Suitable cationic protonated amino and quaternary ammonium monomers, for inclusion in the cationic polymers of the composition herein, include vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts.

Other suitable cationic polymers for use in the compositions include copolymers of 1-vinyl-2-pyrrolidone and 1-vinyl-3-methylimidazolium salt (e.g., chloride salt) (CTFA, Polyquaternium-16); copolymers of 1-vinyl-2-pyrrolidone and dimethylaminoethyl methacrylate (CTFA, Polyquaternium-11); cationic diallyl quaternary ammonium-containing polymers, including, for example, dimethyldiallylammonium chloride homopolymer, copolymers of acrylamide and dimethyldiallylammonium chloride (CTFA, Polyquaternium-6 and Polyquaternium-7, respectively); amphoteric copolymers of acrylic acid including copolymers of acrylic acid and dimethyldiallylammonium chloride (CTFA, Polyquaternium-22); terpolymers of acrylic acid with dimethyldiallylammonium chloride and acrylamide (CTFA, Polyquaternium-39); terpolymers of acrylic acid with methacrylamidopropyl trimethylammonium chloride and methylacrylate (CTFA, Polyquaternium-47); terpolymers of acrylic acid, methacrylamidopropyl trimethylammonium chloride and acrylamide (CTFA, Polyquaternium-53). In one aspect suitable cationic substituted monomers are the cationic substituted dialkylaminoalkyl acrylamides, dialkylaminoalkyl methacrylamides, and combinations thereof.

Other suitable cationic polymers for use in the composition include polysaccharide polymers, such as cationic cellulose derivatives and cationic starch derivatives modified with a quaternary ammonium halide moiety. Exemplary cationic cellulose polymers are salts of hydroxyethyl cellulose reacted with trimethyl ammonium substituted epoxide (CTFA, Polyquaternium-10). Other suitable types of cationic cellulose include the polymeric quaternary ammonium salts of hydroxyethyl cellulose reacted with lauryl dimethyl ammonium substituted epoxide (CTFA, Polyquaternium-24).

Other suitable cationic polymers include cationic polygalactomannan derivatives such as guar gum derivatives and cassia gum derivatives, e.g., guar hydroxypropyltrimonium chloride, hydroxypropyl hydroxypropyltrimonium chloride guar and cassia hydroxypropyltrimonium chloride, respectively. Guar hydroxypropyltrimonium chloride is commercially available under the Jaguar™ trade name series from Rhodia Inc. and the N-Hance trade name series from Ashland Inc. Cassia hydroxypropyltrimonium chloride is commercially available under the Sensomer™ trade name series from Lubrizol Advanced Materials, Inc.

The amount of cationic polymer that may be utilized in the cleansing compositions of the invention range from 0.01 to 10 wt.% in one aspect, from 0.05 to 3 wt.% in another aspect, and from 0.1 to 1 wt.% in a further aspect, based on the weight of the total composition.

Particulates and Insoluble Components

Numerous particulate and substantially insoluble compounds and components requiring stabilization and/or suspension can be utilized in the cleansing compositions of the invention to deliver hair care, skin care and/or aesthetic benefits to the user. The particulate and insoluble components are insoluble in water. By insoluble in water is meant that the solubility in water at 25°C is 0.01 wt.% or less. Examples of such particulate and insoluble components include,

but are not limited to, pigments, exfoliants, anti-dandruff agents, pearlescent agents/opacifiers, and the like.

Pigments

Exemplary pigments are metal compounds or semi metallic compounds and may be used in ionic, nonionic or oxidized form. The pigments can be in this form either individually or in admixture or as individual mixed oxides or mixtures thereof, including mixtures of mixed oxides and pure oxides. Examples are the titanium oxides (e.g., $TO_2$), zinc oxides (e.g., ZnO), aluminum oxides (for example, $Al_2O_3$), iron oxides (for example, $Fe_2O_3$), manganese oxides (e.g., MnO), silicon oxides (e.g., $SiO_2$), silicates, cerium oxides, zirconium oxides (e.g., $ZrO_2$), barium sulfate ($BaSO_4$), nylon-12, and mixtures thereof.

Other examples of pigments include thermochromic dyes that change color with temperature, calcium carbonate, aluminum hydroxide, calcium sulfate, kaolin, ferric ammonium ferrocyanide, magnesium carbonate, carmine, barium sulfate, mica, bismuth oxychloride, zinc stearate, manganese violet, chromium oxide, titanium dioxide nanoparticles, barium oxide, ultramarine blue, bismuth citrate, hydroxyapatite, zirconium silicate, carbon black particles, and the like.

If utilized, the amount of pigment employed in the formulation should be sufficient to provide the desired product aesthetic effect and is well within the skill in the formulation art. In one aspect the amount of pigment typically utilized in the compositions of the invention range from 0.5 wt.% to 20 wt.% in one aspect, from 1 to 15 wt.% in another aspect, and from 5 to 10 wt. % in a further aspect, based on the total weight of the composition.

Exfoliating Agents

Cosmetically useful particulate exfoliating agents are known in the art, and the selection and amount is determined by the exfoliating effect desired from the use of the composition, as recognized by those skilled in the cosmetic arts. Useful exfoliating agents include, but are not limited to, natural abrasives, inorganic abrasives, synthetic polymers, and the like, and mixtures thereof. Representative exfoliants include, but are not limited to, ground or powdered pumice, stone, zeolites, nut shells (e.g., almond, pecan, walnut, coconut, and the like), nut meals (e.g., almond, and the like), fruit pits (e.g., apricot, avocado, olive, peach, and the like), hulls, seed and kernel (e.g., oat bran, corn meal, rice bran, grape seed, kiwi seed, wheat, jojoba seed, loofah seed, rose hip seed, and the like), plant matter (e.g., tea tree leaves, corn cob, fruit fibers, seaweed, loofah sponge, microcrystalline cellulose, and the like), bivalve shells (oyster shell, and the like), diatomaceous earth, calcium carbonate, dicalcium pyrophosphate, chalk, silica, kaolin clay, silicic acid, aluminum oxide, stannic oxide, sea salt (e.g., Dead Sea salt), talc, sugars (e.g., table, brown, and the like), polyethylene, polystyrene, microcrystalline polyamides (nylons), microcrystalline polyesters, polycarbonates, and stainless steel fibers. The foregoing exfoliants can be used in the form of granules, powders, flours, and fibers.

The exfoliating agents for use in the present invention include inorganic physical abrasive type exfoliating agents, a number of which are presented above. In this aspect of the present invention, the exfoliating agent comprises from 0.1 to 20 wt.% in one aspect, and from 0.5 to 10 wt.% in another aspect, based on the weight of the composition.

Anti-Dandruff Agents

Any suitable anti-dandruff agent can be employed in the compositions of the present invention. The anti-dandruff agents may be insoluble or water soluble. Exemplary anti-dandruff agents include, but are not limited to, sulfur, zinc pyrithione, zinc omadine, miconazole nitrate, selenium sulfide, piroctone olamine, N, N- bis(2- hydroxyethyl)undecenamide, cade oil, pine tar, Allium cepa extract Picea abies extract, and Undecyleneth-6, and the like, and mixtures thereof.

In one aspect of the invention, the anti-dandruff agents can be incorporated into the cleansing composition in an amount ranging from 0.001 to 10 wt.% in one aspect, from 0.1 to 5 wt.% in another aspect, and from 0.5 to 3 wt.% in a further aspect, based on the total weight of the stabilized composition.

Peariescent/Opacifying Agents

Some formulations are often opacified by deliberately incorporating pearlescent materials therein to achieve a cosmetically attractive pearl-like appearance, known as pearlescence. An opacifier often is included in a composition to mask an undesirable aesthetic property, such as to improve the color of a composition that is darkened due to the presence of a particular ingredient, or to mask the presence of a particulate material in the composition. Opacifiers also are included in aqueous compositions to improve the aesthetics and consumer acceptance of an otherwise esthetically unpleasing composition. For example, an opacifier can impart a pearlescent appearance to a clear composition, thereby communicating an appearance of creaminess, mildness and body to the consumer. Persons skilled in the art are aware of problems faced by formulators in consistently preparing a stable pearlescent formulation. A detailed discussion is found in the article "Opacifiers and pearling agents in shampoos" by Hunting, Cosmetic and Toiletries, Vol. 96, pages

65-78 (July 1981).

The opacifying or pearlescent material includes organic compounds and inorganic compounds. Typical examples of organic compounds are monoesters and/or diesters of ethylene glycol, propylene glycol, diethylene glycol, dipropylene glycol, triethylene glycol or tetraethylene glycol with fatty acids containing from 6 to 22 carbon atoms in one aspect, and from 12 to 18 carbon atoms in another aspect. Such fatty acids include caproic acid, caprylic acid, 2-ethyhexanoic acid, capric acid, lauric acid, isotridecanoic acid, myristic acid, palmitic acid, palmitoleic acid, stearic acid, isostearic acid, oleic acid, elaidic acid, petroselic acid, linoleic acid, linolenic acid, arachic acid, gadoleic acid, behenic acid, erucic acid, and mixtures thereof. In one aspect, ethylene glycol monostearate (EGMS) and/or ethylene glycol distearate (EGDS) and/or polyethylene glycol monostearate (PGMS) and/or polyethyleneglycol distearate (PGDS) are suitable pearlescent agents used in the composition.

Inorganic pearlescent agents include those selected from the group consisting of mica, metal oxide coated mica, silica coated mica, bismuth oxychloride coated mica, bismuth oxychloride, myristyl myristate, glass, metal oxide coated glass, various aluminum and magnesium salts, guanine, fish scales, glitter (polyester or metallic) and mixtures thereof.

Suitable micas include muscovite or potassium aluminum hydroxide fluoride. The platelets of mica are can be coated with a thin layer of metal oxide. Metal oxides are selected from the group consisting of rutile, titanium dioxide, ferric oxide, tin oxide, alumina and mixtures thereof.

A representative listing of opacifiers is found in the CTFA Cosmetic Ingredient Handbook, J. Nikitakis, ed., 1988, at page 75. Other pearlescent or opacifying materials are disclosed in U.S. Pat. No. 4,654,207; U.S. Pat. No. 5,019,376; and U.S. Pat. No. 5,384,114.

In one aspect, the amount of the pearlescent or opacifying material can be used in amounts ranging from 0.01 to 10 wt.% in one aspect, from 0.1 % to 5 wt.% in another aspect, and from 0.5 to 3 wt.% in a further aspect, based upon the total weight of the composition.

Other Insoluble Components

Other generally insoluble components suitable for use in the present compositions include clay, swellable clay, laponite, gas bubbles, liposomes, UV absorbers, antibacterial compositions, hair fixative, anti-wrinkling and anti-aging compositions, microsponges, cosmetic beads and flakes. Cosmetic beads, flakes and capsules can be included in a composition for aesthetic appearance or can function as micro- and macro-encapsulants for the delivery of benefit agents to the hair and skin. Exemplary bead components include, but are not limited to, agar beads, alginate beads, jojoba beads, gelatin beads, Styrofoam™ beads, polyacrylate, polymethylmethacrylate (PMMA), polyethylene beads, Unispheres™ and Unipearls™ cosmetic beads (Induchem USA, Inc., New York, NY), Lipocapsule™, Liposphere™, and Lipopearl™ microcapsules (Lipo Technologies Inc., Vandalia, OH), and Confetti II™ dermal delivery flakes (United-Guardian, Inc., Hauppauge, NY).

If utilized, the amount of these other insoluble components employed in the formulation should be sufficient to provide the desired product benefit and/or aesthetic effect and is well within the skill in the formulation art. In one aspect the amount of these components typically utilized in the compositions of the invention can range from 0.5 wt.% to 25 wt.% in one aspect, from 1 to 20 wt.% in another aspect, and from 5 to 10 wt. % in a further aspect, based on the total weight of the composition.

Optional Components

In another aspect, the compositions of the invention can include one or more optional components which are customarily used in personal care cleansing products and which do not interfere with the deposition properties of the composition. As optional components for inclusion in the compositions of the invention the following may be mentioned: fragrances, chelating agents, auxiliary suspending agents and viscosity modifiers, such as guar and xanthan gums, emulsifiers, preservatives, amino acids, peptides, proteins, provitamins such as panthenol, vitamins, herb and plant extracts, humectants such as glycerine, and mixtures thereof.

The amount of these additives range from 0 to 20 wt. % in one aspect, from 0.1 to 10 wt.%, and from 0.5 to 5 wt.%, based on the total weight of the composition. The amount of each additive is readily determined by one skilled in the formulation art, depending on the nature and intended function for the additive.

The personal care cleansing compositions of the invention may be used, for example, for washing and depositing silicone conditioning agents on keratin materials such as the hair, the skin, the eyelashes, the eyebrows, the nails, the lips, face, or the scalp. The compositions are applied topically to the desired area of the skin or hair in an amount sufficient to provide effective cleansing and effective delivery of silicone from the product. The compositions can be applied directly to the skin or hair or indirectly via the use of a cleansing puff, washcloth, sponge or other implement. The compositions may be in the form of a body wash, shower gel, bubble bath, two-in-one shampoo, conditioner, facial scrub, moisture

rinse, make-up removal product, and the like. The compositions are preferably diluted with water prior to, during, or after topical application, and then subsequently the skin or hair rinsed or dried off, preferably rinsed off of the applied surface using water.

The present invention may also be useful in rinse-off applications other than personal care compositions including pet care, auto care, home care and medical applications.

Shampoo Compositions

In one aspect, a personal care cleansing composition in which the polymer of this invention is useful is a conditioning shampoo. Typical components of the shampoo composition include at least one detersive surfactant; the stabilizer/thickener polymer described above, a silicone conditioning agent; water; and a pH adjusting agent (base and/or acid) sufficient to attain a pH of from 4 to 9.5 in one aspect, from 5.5 to 8.0 in another aspect, and from 6 to 7.5 in a further aspect. Optional ingredients may be included such as the adjuvants, additives and benefit agents discussed above, and mixtures thereof, including conditioning oils, cationic polymers, particulates and insoluble materials (e.g., pigments, anti-dandruff agents, pearlescent materials, opacifying materials, gas bubbles, propellants, cosmetic beads, flakes, and capsules), fragrances, chelating agents, auxiliary suspending agents and viscosity modifiers, emulsifiers, preservatives, amino acids, peptides, proteins, provitamins, vitamins, herb and plant extracts, humectants, and mixtures thereof.

In one aspect, the surfactant is an anionic surfactant. In another aspect, the surfactant is a mixture of an anionic surfactant and an amphoteric surfactant, in optional combination with a non-ionic surfactant. In one aspect, the anionic surfactant can be present in an amount ranging from 5% to 40% by weight, from 6% to 30% by weight in another aspect, and from 8% to 25% by weight in a further aspect, based on the total weight of the shampoo composition.

When mixtures of anionic and amphoteric surfactants are used, the ratio of anionic surfactant to amphoteric surfactant can range from 1:1 to 10:1 in one aspect, from 2.25:1 to 9:1 in another aspect, and from 4.5:1 to 7:1 in a further aspect.

The amount of stabilizer/thickener polymer can range from 0.5% to 5% by weight in one aspect, from 1 % to 3% by weight in another aspect, and from 1.5% to 2.5% by weight in a further aspect, based on the total weight of the shampoo composition.

Shampoo embodiments of the invention can be formulated as two-in-one shampoos, baby shampoos, conditioning shampoos, bodifying shampoos, moisturizing shampoos, temporary hair color shampoos, three-in-one shampoos, anti-dandruff shampoos, hair color maintenance shampoos, acid (neutralizing) shampoos, medicated shampoos, and salicylic acid shampoos, and the like.

Liquid Fatty Acid Soap Based Cleansers

In one aspect, a personal care cleansing composition in which the polymer of this invention is useful is a fatty acid soap based cleanser. Typical components of a fatty acid based soap cleanser, in addition to the stabilizer/thickener polymer of the invention are: at least one fatty acid salt; a silicone conditioning agent, water, an optional surfactant or mixture of surfactants; a sufficient amount of a pH adjusting agent (base and/or acid) to attain a pH of above 7 in one aspect, from 7.5 to 14 in another aspect, from 8 to 12 in still another aspect, and from 8.5 to 10 in a further aspect. Optional ingredients may be included such as the adjuvants, additives and benefit agents discussed above, and mixtures thereof, including conditioning oils, deposition aids, particulates and insoluble materials (e.g., pigments, anti-dandruff agents, pearlescent materials, opacifying materials, gas bubbles, cosmetic beads, flakes, and capsules), fragrances, chelating agents, auxiliary suspending agents and viscosity modifiers, emulsifiers, preservatives, amino acids, peptides, proteins, provitamins, vitamins, herb and plant extracts, humectants, and mixtures thereof.

In one aspect, the fatty acid soaps are selected from at least one the fatty acid salt (e.g., sodium, potassium, and ammonium) containing from 8 to 22 carbon atoms. In another aspect of the invention the liquid soap composition contains at least one fatty acid salt containing from 12 to 18 carbon atoms. The fatty acids utilized in the soaps can be saturated and unsaturated and can be derived from synthetic sources, as well as from the saponification of fats and natural oils by a suitable base (e.g., sodium, potassium and ammonium hydroxides). Exemplary saturated fatty acids include but are not limited to octanoic, decanoic, lauric, myristic, pentadecanoic, palmitic, margaric, steric, isostearic, nonadecanoic, arachidic, behenic, and the like, and mixtures thereof. Exemplary unsaturated fatty acids include but are not limited to the salts (e.g., sodium, potassium, ammonium) of myristoleic, palmitoleic, oleic, linoleic, linolenic, and the like, and mixtures thereof. The fatty acids can be derived from animal fat such as tallow or from vegetable oil such as coconut oil, red oil, palm kernel oil, palm oil, cottonseed oil, olive oil, soybean oil, peanut oil, corn oil, and mixtures thereof. The amount of fatty acid soap that can be employed in the liquid cleansing compositions of this embodiment ranges from 1% to 50% by weight in one aspect, from 10% to 35% by weight in another aspect, and from 12% to 25% by weight in a further aspect of the invention, based on the weight of the total composition.

The optional anionic surfactant can be present in the soap composition in an amount ranging from 1% to 25% by weight in one aspect, from 5% to 20% by weight in another aspect, and from 8% to 15% by weight in a further aspect, based on the weight of the total weight of the soap composition. Mixtures of anionic and amphoteric surfactants can be used. The ratio of anionic surfactant to amphoteric surfactant can range from 1:1 to 10:1 in one aspect, from 2.25:1 to 9:1 in another aspect, and from 4.5:1 to 7:1 in a further aspect.

In the foregoing soap embodiments of the invention, the amount of stabilizer/thickener polymer can range from 0.5% to 5% by weight in one aspect, from 1% to 3% by weight in another aspect, and from 1.5% to 2.5% by weight in a further aspect, based on the total weight of the soap composition.

The liquid fatty acid soap based cleanser embodiments of the invention can be formulated as body washes, bath gels, shower gels, liquid hand soaps, body scrubs; bubble baths, facial scrubs, and foot scrubs, two-in-one shampoos, baby shampoos, conditioning shampoos, bodifying shampoos, moisturizing shampoos, temporary hair color shampoos, three-in-one shampoos, anti-dandruff shampoos, hair color maintenance shampoos, acid (neutralizing) shampoos, medicated shampoos, and salicylic acid shampoos, and the like.

Multi-Phase Appearance

Visually distinct, multiple phase compositions where one phase is clear and another phase is opaque or pearlized are also envisioned. In one embodiment of the invention, a pattern comprising phases that are visually distinct from each other may be formed by mixing clear and opaque and/or pearlescent components. The visual distinction between each phase can be in color, texture, density, and the type of insoluble component contained therein. The specific pattern can be chosen from a wide variety of patterns, including, but not limited to the following examples: striped, marbled, rectilinear, interrupted striped, check, mottled, marbled, veined, clustered, speckled, geometric, spotted, ribbons, helical, swirl, arrayed, variegated, textured, grooved, ridged, waved, sinusoidal, spiral, twisted, curved, cycle, streaks, striated, contoured, anisotropic, laced, weave or woven, basket weave, spotted, and tessellated. The pattern results from the combination of the "multi-phase" composition by a method of manufacture described in U.S. Patent No. 6,213,166 (Thibiant et al.), U.S. Patent Publication No. US 2004/0219119 (Wei et al.), and U.S. Patent Publication No. US2011/0117225 (Wei et al.).

By the term "multi-phase" as used herein, is meant that each phase of the present compositions occupy separate but distinct physical spaces inside the package in which they are stored, but are in direct contact with one another (i.e., they are not separated by a barrier and they are not emulsified or mixed to any significant degree). In one embodiment of the present invention, the "multi-phase" compositions comprise at least two visually distinct phases, which are present within the container as a visually distinct pattern.

Each visually distinct phase can also include different insoluble materials and/or particulates such as pigments, cosmetic beads, cosmetic flakes, mica, air bubbles, exfoliants, pearlescent materials, opacifiers, silicones, botanicals, benefit agents, and the like as described herein and in the art.

Compositions of this invention demonstrate excellent stability with time in suspending insoluble components and/or benefit agents and stabilizing the visually distinct phases. Multiple-phase compositions are disclosed in U.S. Published Patent Application Nos. 2006/0079417, 2006/0079418, 2006/0079419, 2006/0079420, 2006/0079421, 2006/0079422, 2007/0009463, 2007/0072781, 2007/0280976, and 2008/0317698.

Desirably, the stable multi-phase embodiments of the invention comprise at least two visually distinct phases that are packaged in a transparent or translucent container or package such that the consumer can view the pattern through the container or package.

This invention is illustrated by the following examples. Unless specifically indicated otherwise, parts and percentages are given by weight.

Test Methods

Sensory Panel Testing of Conditioning Attributes

Two-in-one shampoo formulations are compared by a trained panel (at least 3 panelists) for conditioning attributes using a forced choice test design between two treated hair tresses. Hair tresses treated with a 2-in-1 shampoo formulation that includes a rheology modifier polymer of the invention (Formulation 1) are compared to hair tresses treated with an identical base shampoo formulation containing a commercially available rheology modifier (Formulation 2). Each panelist is asked to indicate which tress performs better for each of 4 sensory attributes evaluated in comparing the two test formulations on the treated hair tresses. The sensory attributes evaluated by the panel include (1) ease of wet combing, (2) wet feel (slippery feel or wet conditioned feel), (3) ease of dry combing, and (4) dry feel (soft feel or dry conditioned feel). The test protocol utilizes a matrix design of 6 treated tresses (3 replicates for each of test Formulations 1 and 2). The test matrix allows for the direct blind comparison of the 3 replicate treated tresses of Formulation

1 versus the 3 replicate treated tresses of Formulation 2. By permutation of the 3 replicate treatments for each of Formulations 1 and 2, nine comparisons of paired tresses (Formulation 1 versus Formulation 2) are possible. The matrix is designed such that duplicate evaluations are included from the panel members. A total of 36 comparisons are carried out with the matrix design. A statistical analysis (Z-value calculation of preference of Formulation 1 versus Formulation 2) is used to determine the level of confidence that Formulation 1 is statistically different (better or worse for the selected sensory attribute) from Formulation 2.

Hair Tress Preparation Procedure for Sensory Panel Testing or Silicone Deposition Testing

Hair tresses (Caucasian virgin brown hair or Caucasian bleached hair) weighing 2.5 g (dry wt.) are prewashed with a stripping shampoo (surfactant iso-propanol mixture containing 10 wt.% sodium lauryl sulfate and 10 wt. % iso-propanol) and thoroughly rinsed under warm tap water to remove the shampoo. Excess water is removed by pinching each tress between the index finger and the middle finger and gently pulling the tress through the gap of the fingers. The damp tress is placed on top of the weighing dish and 0.5 g of the test shampoo formulation is applied evenly down the length of the hair tress. The shampoo is massaged into the tress from the root to the tip of the hair tress. The tress is then rinsed under warm tap water for approximately 60 seconds. While rinsing, the tress is combed through its length at least 20 to 25 times to ensure that all residual shampoo is removed. The treatment step is repeated a second time for a total of two washes/rinses.

Wool Swatch Preparation Procedure for Silicone Deposition Testing

Wool swatches (Wool muslin, STC EMPA 217), cut into swatches 2 in. x 6 in. with pinked edges; Testfabrics, Inc., West Pittston, PA, (www.testfabrics.com) are prewashed with a stripping shampoo (surfactant iso-propanol mixture containing 10 wt.% sodium lauryl sulfate and 10 wt.% iso-propanol) and thoroughly rinsed under warm tap water to remove the shampoo. Excess water is removed by squeezing the swatch with the fingers. The damp swatch is placed on top of a weighing dish and 0.25 g of the test shampoo formulation is applied evenly down the length of the swatch. The shampoo is massaged into the swatch and the swatch is then rinsed under warm tap water for approximately 60 seconds. The treatment step is repeated a second time for a total of two wash/rinse cycles.

Viscosity

Brookfield rotating spindle method (all viscosity measurements reported herein are conducted by the Brookfield method whether mentioned or not): The viscosity measurements are calculated in mPa·s, employing a Brookfield rotating spindle viscometer, Model RVT (Brookfield Engineering Laboratories, Inc.), at about 20 revolutions per minute (rpm), at ambient room temperature of about 20 to 25°C (hereafter referred to as viscosity). Spindle sizes are selected in accordance with the standard operating recommendations from the manufacturer. Generally, spindle sizes are selected as follows:

| Spindle Size No. | Viscosity Range (mPa·s) |
|---|---|
| 1 | 1 - 50 |
| 2 | 500 - 1,000 |
| 3 | 1,000 - 5,000 |
| 4 | 5,000 - 10,000 |
| 5 | 10,000 - 20,000 |
| 6 | 20,000 - 50,000 |
| 7 | >50,000 |

The spindle size recommendations are for illustrative purposes only. The artisan of ordinary skill in the art will select a spindle size appropriate for the system to be measured.

Yield Value

Yield Value, also referred to as Yield Stress, is defined as the initial resistance to flow under stress. It is measured by the Brookfield Yield Value (BYV) Extrapolation Method using a Brookfield viscometer (Model RVT) at ambient room

temperature of about 20 to 25°C. The Brookfield viscometer is used to measure the torque necessary to rotate a spindle through a liquid sample at speeds of 0.5 to 100 rpm. Multiplying the torque reading by the appropriate constant for the spindle and speed gives the apparent viscosity. Yield Value is an extrapolation of measured values to a shear rate of zero. The BYV is calculated by the following equation:

$$\text{BYV, dyn/cm}^2 = (\eta_{\alpha 1} - \eta_{\alpha 2})/100$$

where $\eta_{\alpha 1}$ and $\eta_{\alpha 2}$ = apparent viscosities obtained at two different spindle speeds (0.5 rpm and 1.0 rpm, respectively). These techniques and the usefulness of the Yield Value measurement are explained in Technical Data Sheet Number 244 (Revision: 5/98) from Lubrizol Advanced Materials, Inc., herein incorporated by reference.

Suspension Stability Test Suspension

The ability of a polymer system to suspend active and/or aesthetically pleasing insoluble oily and particulate materials is important from the standpoint of product efficacy and appeal. A six dram vial (approximately 70 mm high x 25 mm in diameter) is filled to the 50 mm point with a shampoo test formulation. Each sample vial is centrifuged to remove any trapped air bubbles contained in the formulation. About 10 Unispheres™ UAE-509, Ultramarine Blue beads, commercially available from InduChem AG, are stirred gently with a wooden stick until they are uniformly dispersed throughout the sample. The position of approximately 4 of the beads within each sample vial is noted by drawing a circle around the bead with black marker pen on the outer glass surface of the vial and photographed to establish the initial position of the beads within the formulation. The vials are placed in a 45°C oven to age for a 12 week period. The bead suspension properties of each sample are visually evaluated at the conclusion of the 12 week test period. If the initial position of all 4 of the circled beads is unchanged following the conclusion of the test period the sample passes. If the initial position of one or more of the 4 circled beads changes following the conclusion of the test period the sample fails.

For pearlescence stability testing the test shampoo is placed into a 6 dram vial in the same amount and conditions as described for the bead stability test except that 0.1 wt.% of mica pearlizing agent (based on the total weight of the test sample) is homogeneously dispersed throughout the shampoo. The vials are placed in a 45°C oven to age for a 12 week period. At the conclusion of the test period, the test samples are visually inspected for phase separation or mica settling to the bottom of the vial. Test samples that retain the pearlescence effect after the conclusion of the 12 week test period pass. Samples that exhibit the slightest hint of phase separation and/or particle settling fail.

Turbidity Testing

The turbidity of a composition containing a polymer of the invention is determined in Nephelometric Turbidity Units (NTU) employing a Nephelometric turbidity meter with distilled water (NTU = 0) as the standard.

Silicone Deposition Measurement

The amount of silicone (silicon atoms) deposited on hair tress or wool muslin swatch samples treated with a 2-in-1 shampoo composition is measured by X-Ray fluorescence (XRF) spectroscopy. A wavelength dispersive XRF spectrometer (PANalytical Axios Advanced Sequential 4 kW spectrometer - Model Number PW4400) interfaced with SuperQ 4 software application and fitted with a rhodium tube with an InSb crystal is utilized to facilitate high sensitivity for silicon atom detection corresponding to Si K alpha band. The samples are analyzed using a qualitative program to measure intensities across a two-theta scan range from 139.75° to 147.99° with a peak maximum at 144.53°. The samples are scanned in a vacuum environment using a tube voltage of 25 kV and a current of 160 mA. Scanning speed is 0.05 °2-Theta/sec. with 0.02 °2-Theta step size.

X-rays from the instrument excite silicon atoms deposited on the surface of the hair tress or wool swatch causing them to emit energy and fluoresce. The silicone fluorescence is detected and recorded as counts per second. Higher count rates are indicative of higher silicon atom deposition. The amount of silicon atoms detected is directly proportional to the amount of silicone conditioner deposited on the hair or wool. Samples for XRF analysis are prepared by cutting each treated hair tress into ½ in. lengths and placing the cut lengths into a sample cup having a 6 $\mu$ thick polyethylene support substrate formed into the bottom. A polyethylene spacer is placed on each cut tress to hold it onto the substrate. In the case of muslin wool swatches, the swatch is folded and set into the sample cup.

An average reading of three tresses or three wool swatches per formulation is reported. Wool swatches are an economical model substrate for human hair. The absolute silicone deposition values will be higher for wool swatches than human hair tresses, but the relative silicone deposition values for shampoo formulations containing small particle

size silicone are similar for wool swatches and hair.

Abbreviations

The following abbreviations and trade names are utilized in the examples.

ABBREVIATIONS and Trade Names

| Abbreviation | Chemical Name |
|---|---|
| CEA | β-Carboxyethyl acrylate (Aldrich, Bimax, Rhodia, LZA) |
| EA | Ethyl acrylate |
| VA | Vinyl acetate |
| MA | Methyl acrylate |
| n-BA | n-Butyl acrylate |
| 2-EHA | 2-ethylhexyl acrylate |
| SMA | Stearyl methacrylate |
| HPMA | 2-Hydroxypropyl methacrylate, 3-Hydroxypropyl methacrylate, |
| HEMA | 2-Hydroxyethyl methacrylate |
| MAA | Methacrylic acid |
| AMPS® Monomer | 2-acrylamido-2-methylpropane sulfonic acid (Lubrizol Advanced Materials, Inc.) |
| CSEM-25 | Cetylstearyl ethoxylated -25 methacrylate |
| BEM | Behenyl ethoxylated-25 methacrylate |
| t-BAm | Tertiary butylacrylamide |
| t-OAm | Tertiary octylacrylamide |
| n-VP | n-vinyl pyrrolidone |
| CTLMAA | Silicone macromer |
| STY | Styrene |
| ACE™ Monomer | Reaction product of acrylic acid with the glycidyl ester of neodecanoic acid (Momentive Specialty Chemicals Inc.) |
| MPEG | Methoxylated polyethyleneglycol methacrylate |
| VEOVA-10 | Vinyl neodecanoate |
| RAL 100 | Allyl polyalkylene glycol ether |
| APE | Allyl pentaerythritol |
| AS | Allyl sucrose |
| TEGDMA | Triethyleneglycol dimethacrylate |
| TMPDAE | Trimethylolpropane diallyl ether |
| TMPTA | Trimethylolpropane triacrylate |
| Calfax® DB-45 surfactant | Sodium dodecyl diphenyl oxide disulfonate (Pilot Chemical Company) |
| Sulfochem™ ES-2 anionic surfactant | Sodium Laureth-2 sulfate (Lubrizol Advanced Materials, Inc.) |
| Chembetaine™ amphoteric surfactant | Cocamidopropylbetaine (Lubrizol Advanced Materials, Inc.) |

(continued)

| Abbreviation | Chemical Name |
|---|---|
| Dow Corning® DC 2-1352 silicone emulsion | INCI Name[1]: Dimethicone (and) Laureth-23 (and) C12-15 Pareth-3 (Dow Corning Corporation) (0.5 μm) |
| Dow Corning® DC 2-1491 silicone emulsion | INCI Name[1]: Dimethicone (and) Laureth-23 (and) C12-15 Pareth-3 (Dow Corning Corporation) (20 μm) |
| Dow Corning® DC 2-1664 silicone emulsion | INCI Name[1]: Dimethicone (and) Laureth-4 (and) Laureth-23 (Dow Corning Corporation) (0.16μm) |
| Dow Corning® DC 2-1784 silicone | INCI Name[1]: Dimethiconol (and) TEA- dodecylbenzenesulfonate (Dow Corning Corporation) (1 μm) |
| emulsion | |
| Jaguar® C13-S cationic guar | Guar Hydroxypropyltrimonium Chloride (Rhodia Group) |
| Glydant® Plus preservative | DMDM Hydantoin (and) lodopropynyl Butylcarbamate (Lonza Group Ltd.) |
| Timiron® Starlight Gold Pearlescence Agent | Mica Cosmetic Pigment (Merck Performance Materials) |
| Carbopol® Aqua SF-1 polymer | INCI Name[1] Acrylates Copolymer. A crosslinked copolymer of two or more monomers consisting of acrylic acid, methacrylic acid or one of their simple esters; Supplied as a polymer emulsion 30 wt.% active solids (Lubrizol Advanced Materials, Inc.) |
| Carbopol® Aqua SF-2 polymer | INCI Name: Acrylates Crosspolymer-4. A copolymer of acrylic acid, methacrylic acid or one of its simple esters, crosslinked with trimethylolpropane triacrylate; Supplied as a polymer emulsion 30 wt.% active solids (Lubrizol Advanced Materials, Inc.) |
| Polygel™ W400 polymer | INCI Name: Acrylates Copolymer. A crosslinked copolymer of two or more monomers consisting of acrylic acid, methacrylic acid or one of their simple esters; Supplied as a polymer emulsion 30 wt.% active solids (3V-Sigma SpA) |
| Rheocare® TTA polymer | INCI Name: Acrylates Copolymer. A crosslinked copolymer of two or more monomers consisting of acrylic acid, methacrylic acid or one of their simple esters; Supplied as a polymer emulsion 30 wt.% active solids (BASF SE /Cognis Corporation). |
| Aculyn™ 38 polymer | INCI Name: Acrylates/Vinyl Neodecanoate Crosspolymer. A crosslinked copolymer of two or more monomers consisting of acrylic acid, methacrylic acid or one of their simple esters; Supplied as a polymer emulsion 28 wt.% active solids (The Dow Chemical Company) |
| Carbopol® 980 polymer | INCI Name: Carbomer. A homopolymer of acrylic acid crosslinked with an allyl ether of pentaerythritol, an allyl ether of sucrose, or an allyl ether of propylene. |
| [1]INCI name is the International Nomenclature Cosmetic Ingredient name assigned to a cosmetic ingredient by the International Nomenclature Committee of the Cosmetic, Toiletry, and Fragrance Association (CTFA), Washington, DC, USA, now known as the Personal Care Products Council (PCPC). INCI Names and their definitions are published in the International Cosmetic Ingredient Dictionary and Handbook. | |

Example 1

The following example illustrates a polymerization process for CEA copolymer. Into an agitator equipped first reactor containing 28 grams of deionized water (D.I.), 3.33 grams of sodium lauryl sulfate (30 percent active in water wt./wt.), 64.7 grams of ethyl acrylate, 35 grams of CEA, and 0.3 gram of TMPTA are added under nitrogen atmosphere and mixed at 500 rpm to form a monomer emulsion. To an agitator equipped second reactor is added 230 grams of deionized water and 0.32 grams of sodium lauryl sulfate (30 percent active in water wt./wt.). The contents of the second

reactor are heated to about 90°C with mixing agitation (200 rpm) under a nitrogen atmosphere. When the contents of the second reactor reaches a temperature of approximately 90°C, 2.5 grams of an ammonium persulfate solution (2.0 percent aqueous solution wt./wt.) is injected into the heated surfactant solution. The monomer emulsion from the feed reactor is gradually metered at a feed rate of 0.92 grams/minute into the second reactor over a period of 150 minutes at a reaction temperature maintained at approximately 90°C. After 30 minutes, 0.24 percent ammonium persulfate solution (aqueous solution wt./wt.) is also metered at 0.10 mL/minute into the reaction mixture in the second reactor along with the emulsion monomer feed. The temperature of the reaction is maintained at about 90°C for an additional two and half hours to complete the polymerization. The resulting polymer emulsion product is cooled to room temperature, discharged from the reactor and recovered.

Examples 2-9

The polymers of Examples 2 through 9 are synthesized as set forth in Example 1. The monomer components for these polymers are set forth in Table 1 below.

TABLE 1

| Ex. No. | EA (wt.%) | MA (wt.%) | n-BA (wt.%) | CEA (wt.%) | TMPTA (wt.%) | MAA (wt.%) | HPMA (wt.%) | HEMA (wt.%) | CSEM (wt.%) | Total |
|---|---|---|---|---|---|---|---|---|---|---|
| 2 | 64.7 | -- | -- | 30 | 0.3 | 5 | -- | -- | -- | 100 |
| 3 | 64.7 | -- | -- | 25 | 0.3 | 10 | -- | -- | -- | 100 |
| 4 | 64.7 | -- | -- | 20 | 0.3 | 15 | -- | -- | -- | 100 |
| 5 | 64.7 | -- | -- | 10 | 0.3 | 25 | -- | -- | -- | 100 |
| 6 | 54.7 | -- | -- | 15 | 0.3 | 20 | -- | -- | 10 | 100 |
| 7 | 54.6 | -- | -- | 25 | 0.4 | 15 | 5 | -- | -- | 100 |
| 8 | 44.6 | -- | 5 | 25 | 0.4 | 15 | -- | 10 | -- | 100 |
| 9 | 44.6 | 5 | -- | 25 | 0.4 | 15 | -- | 10 | -- | 100 |

Example 10 (Two Stage Polymer Synthesis)

Into an agitator equipped first (feed) reactor containing 14.0 grams of deionized (D.I.) water and 1.33 grams of sodium lauryl sulfate (30% active in water wt./wt.), 0.89 grams of Calfax® DB-45 surfactant (45% active in water wt./wt.), 58.4 grams of ethyl acrylate, 5.9 grams of hydroxypropyl methacrylate, 23.6 grams of methacrylic acid, 11.8 grams of CEA, and 0.4 grams of trimethylolpropane triacrylate are added under nitrogen atmosphere and mixed at 500 rpm to form a monomer emulsion. To an agitator equipped second reactor are added 220 grams of deionized water and 0.37 grams of sodium lauryl sulfate (30% active in water wt./wt.). The contents of the second reactor are heated with mixing agitation (200 rpm) under a nitrogen atmosphere. When the contents of the second reactor reaches a temperature of approximately 84°C, 2.550 grams of ammonium persulfate solution (2.0% aqueous solution wt./wt.) is injected into the heated surfactant solution. The monomer emulsion from the feed reactor is gradually metered (0.83 g/min.) into the second reactor over a period of about 75 minutes at a reaction temperature maintained at approximately 88°C and allowed to react in a first stage polymerization reaction to form first stage polymer particles of crosslinked ethyl acrylate/carboxyethyl acrylate/ hydroxypropyl methacrylate/ methacrylic acid/ trimethylolpropane triacrylate copolymer. Following the initial addition of the monomer emulsion into the second reactor, the second stage monomer emulsion is prepared in the feed reactor by adding 14 grams of deionized water (D.I), 1.33 grams of sodium lauryl sulfate (30% active in water wt./wt.), 0.89 grams of Calfax® DB-45 surfactant (45% active in water wt./wt.), 43.1 grams of ethyl acrylate, 26.1 grams of CEA, 13 grams of hydroxypropyl methacrylate, 17.4 grams of methacrylic acid, and 0.4 grams of trimethylolpropane triacrylate. The monomer emulsion containing excess amount of CEA is then metered into the second reactor over a period of 75 minutes at a controlled rate (1.03 g/min.) at a temperature maintained at approximately 88°C and polymerized in the presence of the first stage polymer particles in a second stage reaction to form a crosslinked CEA-rich polymer second stage (over the first stage polymer particles) comprising polymerized ethyl acrylate/ carboxyethyl acrylate/ hydroxypropyl methacrylate/ methacrylic acid/ trimethylolpropane triacrylate copolymer. With the emulsion monomer feed, 18.2 grams of ammonium persulfate (0.37% aqueous solution wt./wt.) is simultaneously metered into the reaction mixture in the second reactor and the temperature of the reaction is maintained at about 88°C for an additional two and half hours to complete polymerization. The resulting polymer emulsion product is cooled to room

temperature, discharged from the reactor and recovered.

Examples 11-42

Staged polymers are prepared as set forth in Example 10 except that the monomer components and amounts differ as set forth in Tables 2 and 2A, respectively.

TABLE 2
(First Stage Monomer Components)

| Ex. No. | EA | VA | n-VP | styrene | t-BAm | t-OAm | SMA | ACE | CEA | TMPTA | MAA | HPMA | MPEG | AMPS® | CTLMA A | Total |
|---|---|---|---|---|---|---|---|---|---|---|---|---|---|---|---|---|
| 11 | 58.4 | | | | | | | | 17.7 | 0.4 | 17.7 | 5.9 | | | | 100 |
| 12 | 62.1 | | | | | | | | 12.5 | 0.4 | 18.8 | 6.3 | | | | 100 |
| 13 | 58.4 | | | | | | | | 11.8 | 0.4 | 23.6 | 5.9 | | | | 100 |
| 14 | 58.4 | | | | | | | | 11.8 | 0.4 | 23.6 | 5.9 | | | | 100 |
| 15 | 56.9 | | | | | | | | 17.5 | 0.5 | 17.5 | 5.8 | | 1.8 | | 100 |
| 16 | 58.1 | | | | | | | | 16.1 | 0.4 | 18.8 | 5.4 | | 1.1 | | 100 |
| 17 | 63.5 | | | | | | | | 16.1 | 0.4 | 18.8 | | | 1.1 | | 100 |
| 18 | 58.4 | | | | | | | | 17.7 | 0.4 | 17.7 | 5.9 | | | | 100 |
| 19 | 41.6 | | | 26.8 | | | | | 13.4 | 0.3 | 13.4 | 4.5 | | | | 100 |
| 20 | 62.0 | | 6.3 | | | | | | 31.3 | 0.4 | | | | | | 100 |
| 21 | 62.0 | | | | | 6.3 | | | 31.3 | 0.4 | | | | | | 100 |
| 22 | 62.0 | | | | | | | | 31.3 | 0.4 | | | 6.3 | | | 100 |
| 23 | 65.8 | | | | | | | | 31.0 | 0.4 | | | | | 2.8 | 100 |
| 24 | 58.4 | | | | 5.9 | | | | 17.7 | 0.4 | 17.7 | | | | | 100 |
| 25 | 58.4 | | | | | | | | 17.7 | 0.4 | 17.7 | 5.9 | | | | 100 |
| 26 | 58.7 | | | | | | | | 17.7 | 0.1 | 17.7 | 5.9 | | | | 100 |
| 27 | 58.4 | | | | | | | | 17.7 | 0.4 | 17.7 | 5.9 | | | | 100 |
| 28 | 58.4 | | | | | | | | 17.7 | 0.4 | 17.7 | 5.9 | | | | 100 |
| 29 | 58.4 | | | | | | | | 17.7 | 0.4 | 17.7 | 5.9 | | | | 100 |
| 30 | 54.9 | | | | | | 3.5 | | 17.7 | 0.4 | 17.7 | 5.9 | | | | 100 |
| 31 | 54.9 | | | | | | | 3.5 | 17.7 | 0.4 | 17.7 | 5.9 | | | | 100 |
| 32 | 58.4 | | | | | | | | 17.7 | 0.4 | 17.7 | 5.9 | | | | 100 |
| 33 | 63.7 | | | | | | | | 18.0 | 0.4 | 18.0 | | | | | 100 |
| 34 | 63.0 | | | | | | | | 18.3 | 0.4 | 18.3 | | | | | 100 |
| 35 | 63.8 | | | | | | | | 17.9 | 0.4 | 17.9 | | | | | 100 |
| 36 | 63.4 | | | | | | | | 18.1 | 0.4 | 18.1 | | | | | 100 |
| 37 | 64.1 | | | | | | | | 17.8 | 0.4 | 17.8 | | | | | 100 |
| 38 | 63.8 | | | | | | | | 17.9 | 0.4 | 17.9 | | | | | 100 |
| 39 | 62.9 | | | | | | | | 18.4 | 0.4 | 18.4 | | | | | 100 |
| 40 | 62.9 | | | | | | | | 18.4 | 0.4 | 18.4 | | | | | 100 |
| 41 | 34.9 | 23.6 | | | | | | | 11.8 | 0.4 | 23.6 | 5.9 | | | | 100 |
| 42 | 34.9 | 23.6 | | | | | | | 11.8 | 0.4 | 23.6 | 5.9 | | | | 100 |

TABLE 2A
(Second Stage Monomer Components)

| Ex. No. | EA | VA | CEA | TMPTA | MAA | HPMA | t-OAm | 2EHA | n-VP | t-BuAm | SMA | ACE | MPEG | CTLMAA | S CSEM | BEM | TMPDAE | APE | AS | TEGDMA | VEOVA 10 | RAL 100 | Total |
|---|---|---|---|---|---|---|---|---|---|---|---|---|---|---|---|---|---|---|---|---|---|---|---|
| 11 | 43.1 | | 30.4 | 0.4 | 13.0 | 13.0 | | | | | | | | | | | | | | | | | 100 |
| 12 | 41.3 | | 25.0 | 0.4 | 12.5 | 12.5 | | | | | | | | | 8.3 | | | | | | | | 100 |
| 13 | 43.1 | | 26.1 | 0.4 | 17.4 | 4.3 | | | | | | | | | | 8.7 | | | | | | | 100 |
| 14 | 43.1 | | 8.7 | 0.4 | 34.8 | 4.3 | | | | | | | | | | 8.7 | | | | | | | 100 |
| 15 | 42.6 | | 30.6 | 0.5 | 13.1 | 13.1 | | | | | | | | | | | | | | | | | 100 |
| 16 | 50.4 | | 28.0 | 0.6 | 16.3 | 4.7 | | | | | | | | | | | | | | | | | 100 |
| 17 | 55.1 | | 28.0 | 0.6 | 16.3 | 0.0 | | | | | | | | | | | | | | | | | 100 |
| 18 | 43.1 | | 30.4 | 0.4 | 13.0 | 13.0 | | | | | | | | | | | | | | | | | 100 |
| 19 | 47.2 | | 26.5 | 0.4 | 15.2 | 10.7 | | | | | | | | | | | | | | | | | 100 |
| 20 | 45.0 | | 22.7 | 0.5 | 20.4 | 6.8 | | | 4.5 | | | | | | | | | | | | | | 100 |
| 21 | 45.0 | | 22.7 | 0.5 | 20.4 | 6.8 | 4.5 | | | | | | | | | | | | | | | | 100 |
| 22 | 45.0 | | 22.7 | 0.5 | 20.4 | 6.8 | | | | | | | 4.5 | | | | | | | | | | 100 |
| 23 | 48.4 | | 22.8 | 0.5 | 20.4 | 6.8 | | | | | | | | 1.0 | | | | | | | | | 100 |
| 24 | 43.1 | | 30.4 | 0.4 | 13.0 | 8.7 | | | | 4.3 | | | | | | | | | | | | | 100 |
| 25 | 43.1 | | 30.4 | 0.3 | 13.0 | 13.0 | | | | | | | | | | | 0.2 | | | | | | 100 |

| Ex. No. | EA | VA | CEA | TMPTA | MAA | HPMA | t-OAm | 2EHA | n-VP | t-BuAm | SMA | ACE | MPEG | CTLMAA | S CSEM | BEM | TMPDAE | APE | AS | TEGDMA | VEOVA 10 | RAL 100 | Total |
|---|---|---|---|---|---|---|---|---|---|---|---|---|---|---|---|---|---|---|---|---|---|---|---|
| 26 | 43.3 | | 30.4 | 0.0 | 13.0 | 13.0 | | | | | | | | | | | | 0.2 | | | | | 100 |
| 27 | 43.1 | | 30.4 | 0.3 | 13.0 | 13.0 | | | | | | | | | | | | | 0.2 | | | | 100 |
| 28 | 43.2 | | 30.4 | 0.3 | 13.0 | 13.0 | | | | | | | | | | | | 0.1 | | | | | 100 |
| 29 | 43.2 | | 30.4 | 0.3 | 13.0 | 13.0 | | | | | | | | | | | 0.1 | | | | | | 100 |
| 30 | 40.5 | | 30.4 | 0.4 | 13.0 | 13.0 | | | | | 2.6 | | | | | | | | | | | | 100 |
| 31 | 40.5 | | 30.4 | 0.4 | 13.0 | 13.0 | | | | | | 2.6 | | | | | | | | | | | 100 |
| 32 | 43.0 | | 30.4 | 0.3 | 13.0 | 13.0 | | | | | | | | | | | | | | 0.2 | | | 100 |
| 33 | 45.5 | | 30.0 | 0.4 | 12.9 | 8.6 | | | | | | | | 2.6 | | | | | | | | | 100 |
| 34 | 43.7 | | 29.6 | 0.4 | 12.7 | 8.5 | 5.1 | | | | | | | | | | | | | | | | 100 |
| 35 | 46.0 | | 30.1 | 0.6 | 12.9 | 8.6 | 1.7 | | | | | | | | | | | | | | | | 100 |
| 36 | 44.9 | | 29.9 | 0.4 | 12.8 | 8.5 | 3.4 | | | | | | | | | | | | | | | | 100 |
| 37 | 46.7 | | 30.3 | 0.6 | 13.0 | 8.6 | 0.9 | | | | | | | | | | | | | | | | 100 |
| 38 | 46.0 | | 30.1 | 0.6 | 12.9 | 8.6 | | 1.7 | | | | | | | | | | | | | | | 100 |
| 39 | 43.4 | | 29.6 | 0.6 | 12.7 | 8.5 | | | | | | | | | | | | | | 0.2 | 5.1 | | 100 |
| 40 | 43.4 | | 29.6 | 0.6 | 12.7 | 8.5 | | | | | | | | | | | | | | 0.2 | | 5.1 | 100 |
| 41 | 25.7 | 17.4 | 26.1 | 0.4 | 17.4 | 4.3 | | | | | | | | | | 8.7 | | | | 0.0 | | 0.0 | 100 |
| 42 | 25.7 | 17.4 | 26.1 | 0.4 | 17.4 | 13.0 | | | | | | | | | | | | | | 0.0 | | 0.0 | 100 |

Example 43

This polymer synthesized as set forth in Example 1 using vinyl acetate in place of ethyl acrylate. The polymerization reaction is conducted at a lower temperature, 70°C. The monomer components utilized in the synthesis are shown in below in Table 3.

TABLE 3

| Ex. No. | VA | CEA | TMPTA | MAA | Total |
|---|---|---|---|---|---|
| 43 | 59.6 | 25 | 0.4 | 15 | 100 |

Example 44

This example illustrates the rheological and physical properties of gels made from the polymers of Examples 1-43. The gels are prepared as mucilages of 1.0 and 2.5 wt.% total polymer solids in water and subsequently neutralized with an aqueous solution of 18% NaOH (wt./wt.). Rheological and physical properties, e.g., Brookfield viscosity (BV), yield value (YV), and turbidity (NTU), are measured and listed in Tables 4 and 4A.

TABLE 4

| Polymer No. | 1.0% pH | Viscosity (mPa·s) | Yield Value (Dyn/cm$^2$) | NTU | 2.5% pH | Viscosity (mPa·s) | Yield Value (Dyn/cm$^2$) | NTU |
|---|---|---|---|---|---|---|---|---|
| 1 | 7.4 | 270 | 7 | 143 | 7.93 | 4,770 | 370 | 277 |
| 2 | 6.96 | 1,200 | 53 | 171 | 7.17 | 22,900 | 2,300 | 175 |
| 3 | 7.21 | 3,350 | 262 | 136 | 7.24 | 31,800 | 3,530 | 108 |
| 4 | 6.98 | 5,050 | 454 | 153 | 7.00 | 17,700 | 1,900 | 73.8 |
| 5 | 7.04 | 7,150 | 676 | 50.5 | 6.88 | 14,100 | 1,360 | 11.2 |
| 6 | 7.07 | 2,900 | 237 | 114 | 6.85 | 102,000 | 7,120 | 37.4 |
| 7 | 6.96 | 3,240 | 223 | 23.6 | 6.56 | 8,850 | 716 | 18.8 |
| 8 | 6.9 | 218 | 5 | 281 | 7 | 28,700 | 2,900 | 229 |
| 9 | 7.13 | 1,390 | 81 | 154 | 7 | 26,500 | 2,850 | 101 |
| 10 | 6.83 | 4,160 | 311 | 7.67 | 6.63 | 8,350 | 684 | 5.97 |
| 11 | 6.61 | 3,870 | 290 | 11.2 | 6.48 | 8,750 | 742 | 21.4 |
| 12 | 6.92 | 8,200 | 780 | 12.2 | 6.82 | 27,100 | 2,230 | 11.8 |
| 13 | 6.79 | 13,100 | 1830 | 15.2 | 6.78 | 40,100 | 3,290 | 13.2 |
| 14 | 6.93 | 15,600 | 185 | 19.2 | 6.86 | 67,500 | 430 | 13.5 |
| 15 | 6.99 | 3,170 | 249 | 39.2 | 6.93 | 9,700 | 870 | 14 |

| Polymer No. | 1.0% pH | Viscosity (mPa·s) | Yield Value (Dyn/cm$^2$) | NTU | 2.5% pH | Viscosity (mPa·s) | Yield Value (Dyn/cm$^2$) | NTU |
|---|---|---|---|---|---|---|---|---|
| 16 | 7.04 | 3,920 | 320 | 21.1 | 7.02 | 8,750 | 782 | 6.88 |
| 17 | 7.11 | 5,350 | 478 | 33.4 | 6.99 | 12,400 | 1,170 | 9.11 |
| 18 | 6.61 | 4,040 | 266 | 6.76 | 6.47 | 9,600 | 748 | 20.9 |
| 19 | 6.94 | 2,410 | 171 | 42.5 | 6.81 | 5,050 | 386 | 132 |
| 20 | 7.06 | 36 | 0 | 214 | 6.74 | 11,800 | 1060 | 496 |
| 21 | 7.18 | 2,510 | 185 | 67.2 | 7.02 | 6,400 | 524 | 91.5 |
| 22 | 6.9 | 655 | 32 | 313 | 6.72 | 30,100 | 3130 | 326 |
| 23 | 6.78 | 2,900 | 227 | 68.9 | 6.7 | 7,450 | 648 | 84 |
| 24 | 6.67 | 1,920 | 124 | 4.43 | 6.56 | 3,540 | 246 | 5.3 |
| 25 | 6.74 | 760 | 40 | 240 | 6.72 | 24,000 | 2630 | 94.7 |
| 26 | 6.72 | 3,650 | 301 | 71.6 | 6.65 | 17,900 | 1900 | 41 |
| 27 | 6.81 | 2,770 | 214 | 79.2 | 6.62 | 14,200 | 1430 | 28.4 |
| 28 | 6.64 | 3,320 | 251 | 9.84 | 6.59 | 7,300 | 630 | 11.4 |
| 29 | 6.55 | 2,580 | 189 | 4.86 | 6.48 | 5,600 | 458 | 11.7 |
| 30 | 6.76 | 2,040 | 134 | 14.2 | 6.45 | 4,320 | 292 | 25.7 |
| 31 | 6.85 | 2,100 | 146 | 15 | 6.68 | 4,250 | 328 | 17.9 |
| 32 | 6.76 | 2,470 | 160 | 10.3 | 6.84 | 5,350 | 392 | 18.7 |
| 33 | 6.68 | 1,470 | 82 | 2.76 | 6.6 | 2,970 | 191 | 8.14 |
| 34 | 6.65 | 1,440 | 91 | 4.8 | 6.65 | 2,700 | 181 | 3.4 |
| 35 | 6.8 | 2,680 | 159 | 7.3 | 6.62 | 6,350 | 462 | 14.4 |
| 36 | 6.78 | 1,850 | 124 | 7.1 | 6.74 | 3,390 | 240 | 5.2 |
| 37 | 6.83 | 3,290 | 200 | 6.7 | 6.76 | 7,850 | 576 | 7.6 |
| 38 | 6.95 | 2,020 | 140 | 7.6 | 6.67 | 4,010 | 304 | 4.6 |
| 39 | 6.87 | 3,010 | 235 | 29.8 | 6.56 | 6,800 | 574 | 16 |
| 40 | 6.8 | 2,640 | 189 | 12.2 | 6.59 | 5,500 | 442 | 9.3 |
| 41 | 7.06 | 26,200 | 2150 | 28 | 6.62 | 150,000 | 3100 | 13.2 |
| 42 | 6.73 | 4,300 | 292 | 23.4 | 6.52 | 6,850 | 518 | 18.6 |
| 43 | 7.17 | 1,840 | 52 | opaque | 6.84 | 8,650 | 402 | opaque |

Example 45

Two-in-one conditioning shampoos containing the stabilizer/thickener polymers prepared from a monomer composition comprising CEA in accordance with Examples 1-43 are formulated with the components set forth in Table 5. Identical two-in-one comparative shampoo compositions are similarly prepared except that they are formulated with non-CEA containing commercially available stabilizer/thickener polymers. The commercially available stabilizer/thickener polymers are commonly employed in shampoo compositions for their ability to stably suspend silicones and other particulate materials such as pearlescence agents (e.g., mica and/or beads). The shampoo formulations are prepared with the components set forth in Table 5.

TABLE 5

| Component | Amount Active (wt.%) |
|---|---|
| PART A | |

(continued)

| Component | Amount Active (wt.%) |
|---|---|
| D.I. Water | q.s. to 100 |
| Stabilizer/Thickener Polymer (polymer solids) | See Table 6 |
| Sulfochem™ ES-2 anionic surfactant | 14 |
| Chembetaine™ amphoteric surfactant | 3 |
| PART B | |
| Jaguar® C13-S cationic guar deposition aid | 0.25 |
| PART C | |
| Dow Corning® silicone emulsion (DC 2-1352) | 2 |
| Glydant® Plus Preservative | 0.22 |
| Timiron® Starlight Gold Pearlescence Agent | 0.1 |
| Unispheres™ UAE-509, Ultramarine Blue Beads | 10 beads |
| PART D | 0.25 |
| NaOH (18% aqueous wt./wt.) neutralizing agent | q.s. to pH 6 |

The shampoo compositions are prepared in accordance with the following procedure:

Part A:

1) Disperse the stabilizer/thickener polymer in D.I. water;
2) Add anionic and amphoteric surfactants and mix for 15 min.

Part B:

3) Prepare a 2 wt.% (wt./wt.) dispersion of cationic guar in D.I. water and mix with Part A components.

Part C:

4) Add Part C components to Part AB component mixture and mix until homogeneous;

4) Adjust the pH of the ABC component mixture with NaOH to a pH of about 6.

The shampoo compositions are visually evaluated for their ability to produce a stable pearlescence effect (mica suspension stability) and/or the ability to stably suspend beads. Silicone deposition on wool test swatches is also assessed. The results are set forth in Table 6.

TABLE 6

| Polymer Ex. No. | Active Polymer Solids (wt.%) | Si Peak Intensity (kcps) | Mica Suspension (Pearlescence) 12 wks. @ 45°C | Bead Suspension 12 wks. @ 45°C |
|---|---|---|---|---|
| C-1[1] | 1.5 | 14.4 | Pass | Pass |
| C-1[1] | 2.5 | 6.8 | Pass | Pass |
| C-2[2] | 1.5 | 16.0 | Pass | Pass |
| C-3[3] | 1.5 | 15.14 | Pass | Pass |
| C-4[4] | 1.5 | 19.7 | Pass | Pass |
| C-5[5] | 1.5 | 5.1 | Pass | Pass |

(continued)

| Polymer Ex. No. | Active Polymer Solids (wt.%) | Si Peak Intensity (kcps) | Mica Suspension (Pearlescence) 12 wks. @ 45°C | Bead Suspension 12 wks. @ 45°C |
|---|---|---|---|---|
| B-6[6] | 0 | 66.3 | Fail | Fail |
| B-7[6] | 0 | 63.1 | Fail | Fail |
| 1 | 2.5 | 63.6 | -- | Pass |
| 2 | 1.5 | 39 | -- | Pass |
| 3 | 1.5 | 53.5 | -- | Pass |
| 4 | 1.5 | 29.9 | -- | Pass |
| 5 | 1.5 | 31.7 | -- | Pass |
| 6 | 1.5 | 57.70 | -- | Pass |
| 7 | 1.5 | 61.8 | -- | Pass |
| 8 | 1.5 | 47.8 | -- | Pass |
| 9 | 1.5 | 51.5 | -- | Pass |
| 10 | 2.5 | 47.8 | -- | Pass |
| 11 | 2.5 | 62.6 | Pass | Pass |
| 12 | 2.5 | 49.1 | -- | Pass |
| 13 | 2.5 | 55.1 | Pass | Pass |
| 14 | 2.0 | 37.7 | -- | Pass |
| 15 | 2.5 | 40.2 | -- | Pass |
| 16 | 2.5 | 33.4 | -- | Pass |
| 17 | 2.5 | 26.2 | -- | Pass |
| 18 | 2.5 | 59.2 | -- | Pass |
| 19 | 2.5 | 36.2 | -- | Pass |
| 20 | 2.5 | 50.6 | -- | Pass |
| 21 | 2.5 | 22.6 | -- | Pass |
| 22 | 2.5 | 45.0 | -- | Pass |
| 23 | 2.5 | 24.9 | -- | Pass |
| 24 | 2.5 | 33.6 | -- | Pass |
| 25 | 2.5 | 54.9 | -- | Pass |
| 26 | 2.5 | 51.5 | -- | Pass |
| 27 | 2.5 | 53.2 | -- | Pass |
| 28 | 2.5 | 41.8 | -- | Pass |
| 29 | 2.5 | 35.2 | -- | Pass |
| 30 | 2.5 | 32.6 | -- | Pass |
| 31 | 2.5 | 25.7 | -- | Pass |
| 32 | 2.5 | 50.5 | -- | Pass |
| 33 | 2.5 | 28.8 | -- | Pass |
| 34 | 2.5 | 23.2 | -- | Pass |

(continued)

| Polymer Ex. No. | Active Polymer Solids (wt.%) | Si Peak Intensity (kcps) | Mica Suspension (Pearlescence) 12 wks. @ 45°C | Bead Suspension 12 wks. @ 45°C |
|---|---|---|---|---|
| 35 | 2.5 | 56.9 | -- | Pass |
| 36 | 2.5 | 35.9 | -- | Pass |
| 37 | 2.5 | 65.5 | -- | Pass |
| 38 | 2.5 | 40.8 | -- | Pass |
| 39 | 2.5 | 34.4 | -- | Pass |
| 40 | 2.5 | 37.7 | -- | Pass |
| 41 | 2.5 | 37.2 | -- | Pass |
| 42 | 2.5 | 51.7 | -- | Pass |
| 43 | 1.5 | 53.2 | -- | Fail |

[1]Carbopol® Aqua SF-1 polymer emulsion
[2]Carbopol® Aqua SF-2 polymer emulsion
[3]Aculyn™ 38 polymer emulsion
[4]Rheocare™ TTA polymer emulsion
[5]Polygel™ W400 polymer emulsion
[6]Blank formulation (contains no stabilizer/thickener polymer)

Higher levels of non-CEA containing stabilizer/thickener polymer decreases silicone deposition with increasing concentration. This is seen in the results for polymer C1 as the concentration of polymer in the two-in-one shampoo formulation increases from 1.5 wt.% to 2.5 wt.%. It is also evident that non-CEA containing polymers adversely influence silicone deposition, as two-in-one shampoos formulated without any stabilizer/thickener polymer (blank formulations) deposit significantly more silicone than non-CEA containing polymers. From the data it is apparent that stabilizer/thickener polymers prepared from a CEA monomer mitigate the loss of silicone deposition when included in two-in-one conditioning shampoos.

Example 46

A sensory panel test is conducted comparing the conditioning performance of two-in-one shampoos prepared in Example 44 to a shampoo containing a commercial stabilizer/thickener polymer. European brown hair tresses treated with the shampoo compositions formulated with the polymer of Examples 11 and 13 (2.5 wt.%) and the shampoo formulated with polymer C-1 (1.5 wt.%) are evaluated for sensory attributes in accordance with the methodology set forth in the Sensory Panel Testing protocol described above. The invention formulations containing 2.5 wt.% the polymers of Examples 11 and 13 display statistically significant better wet and dry conditioning properties in contrast to the formulation containing 1.5 wt.% of the commercially available C-1 stabilizer/thickener polymer on European brown hair. The results of the panel test indicate that hair tresses treated with the invention formulations have better wet combing attributes (99% confidence level), better wet feel attributes (99% confidence level), better dry combing attributes (99% confidence level) and better dry feel attributes (99% confidence level) than the comparative formulation.

Example 47

Two-in-one pearlescent conditioning shampoo compositions including the polymer of Example 11 and the commercially available polymers C-1 and C-6 stabilizer/thickeners are formulated with the components set forth in Table 7.

TABLE 7

| Component | Invention Formulation 1 Active (wt %) | Comparative Formulation C-1 Active (wt %) | Comparative Formulation C-6 Active (wt %) |
|---|---|---|---|
| Sulfochem™ ES-2 anionic surfactant | 14 | 14 | 14 |
| Chembetaine™ amphoteric surfactant | 3 | 3 | 3 |
| Silicone Emulsion (DC 2-1352) | 2 | 2 | 2 |
| Polymer of Example 11 | 2.5 | -- | -- |
| Polymer C-1[1] | -- | 1.5 | -- |
| Polymer C-6[2] | -- | -- | 0.4 |
| Jaguar™ C13-S cationic guar deposition aid | 0.25 | 0.25 | 0.25 |
| Timiron® Starlight Gold Cosmetic Mica Pigment | 0.1 | 0.1 | 0.1 |
| D.I. Water | q.s. to 100 | q.s. to 100 | q.s. to 100 |
| NaOH (18 % aqueous wt./wt.) | q.s. to pH 6.0 | q.s. to pH 6.0 | q.s. to pH 6.0 |

[1]Carbopol® Aqua SF-1 polymer emulsion
[2]Carbopol® 980 powdered Carbomer

The formulations are visually evaluated for the ability to disperse mica pigment and create a pearlescence effect. One of the known benefits of the commercial polymer C-1 stabilizer/thickener polymer is its ability to stably disperse mica and enhance pearlescent shine. The shampoo formulation of the invention containing the polymer of Example 11 compares identically with the commercial polymer C-1. Conversely, the mica particles agglomerated in the shampoo formulated with commercial polymer C-6 (powdered Carbomer) and no pearlescence effect was observed. Rheological data for each of the formulations is presented in Table 8.

TABLE 8

| Rheological and Silicone Deposition Data | Invention Formulation 1 | Comparative Formulation C-1 | Comparative Formulation C-6 |
|---|---|---|---|
| Brookfield viscosity at 20 rpm (mPa·s) | 4,840 | 3,500 | 290 |
| Beads suspension for 12 weeks at 45°C | passed | passed | failed |
| Mica pearlescence | passed | passed | failed |
| Net Si Peak Intensity (kcps) | 15.0 | 6.8 | 13.0 |

Shampoo formulations comprising the polymer of the invention and the commercial C-1 stabilizer/thickener polymer yield shampoo compositions with good stability, high particulate suspension ability and excellent pearlescence. The shampoo formulation containing the polymer of the invention exhibited significantly higher silicone deposition on European brown hair tresses compared to the C-1 formulation and similar silicone deposition to the C-6 formulation.

Example 48

The stabilizer/thickener polymers of Examples 11, 13 and C-1 are formulated into two-in-one shampoo compositions utilizing the components set forth in Table 9. The components are formulated by the method disclosed in Example 44. Each of the formulations is evaluated for Brookfield viscosity, pearlescence and silicone deposition. The

results are set forth in Table 10.

TABLE 9

| Component | Invention Formulation 2 Active (wt %) | Invention Formulation 3 Active (wt %) | Comparative Formulation C-1 Active (wt %) |
|---|---|---|---|
| Sulfochem™ ES-2 anionic surfactant | 14 | 14 | 14 |
| Chembetaine™ amphoteric surfactant | 3 | 3 | 3 |
| Silicone Emulsion (DC-1664) | 2 | 2 | 2 |
| Polymer C-1 | | | 1.5 |
| Polymer of Example 11 | 2.5 | -- | -- |
| Polymer of Example 13 | | 2.5 | -- |
| Jaguar™ C13-S cationic guar deposition aid | 0.25 | 0.25 | 0.25 |
| Timiron® Starlight Gold Cosmetic Mica Pigment (Rona) | 0.1 | 0.1 | 0.1 |
| D.I. Water | q.s. to 100 | q.s. to 100 | q.s. to 100 |
| NaOH (18 % aqueous wt./wt.) | q.s. to pH 6.0 | q.s. to pH 6.0 | q.s. to pH 6.0 |
| [1]Carbopol® Aqua SF-1 polymer emulsion | | | |

TABLE 10

| Rheological and Silicone Deposition Data | Invention Formulation 2 | Invention Formulation 3 | Comparative Formulation C-1 |
|---|---|---|---|
| Brookfield viscosity at 20 rpm (mPa·s) | 3,620 | 19,850 | 3,270 |
| Mica pearlescence | Pass | Pass | Pass |
| Net Si Peak Intensity (kcps) | 18.1 | 13.7 | 9.8 |

The formulations comprising the polymers of the invention prepared from CEA exhibit significantly higher amounts of silicone deposition on European brown hair tresses compared to the commercially available polymer C-1 which is not prepared from CEA.

Example 49

The stabilizer/thickener polymers of Examples 11 and 13 are formulated into two-in-one shampoo compositions utilizing the components set forth in Table 11. A commercially available stabilizer/thickener polymer is similarly formulated. The components are formulated by the method disclosed in Example 44. Each of the shampoo formulations is evaluated for Brookfield viscosity, pearlescence and silicone deposition. The results are set forth in Table 12.

TABLE 11

| Component | Invention Formulation 4 Active (wt %) | Invention Formulation 5 Active (wt %) | Comparison Formulation C-1 Active (wt %) |
|---|---|---|---|
| Sulfochem™ ES-2 anionic surfactant | 14 | 14 | 14 |
| Chembetaineamphoteric surfactant | 3 | 3 | 3 |

(continued)

| Component | Invention Formulation 4 Active (wt %) | Invention Formulation 5 Active (wt %) | Comparison Formulation C-1 Active (wt %) |
|---|---|---|---|
| Silicone Emulsion (DC 2-1784) | 2 | 2 | 2 |
| Polymer of Example 11 | 2.5 | -- | |
| Polymer of Example 13 | -- | 2.5 | -- |
| Polymer C1 | | | 1.5 |
| Jaguar™ C13-S cationic guar deposition aid | 0.25 | 0.25 | 0.25 |
| Timiron® Starlight Gold Cosmetic Mica Pigment (Rona) | 0.1 | 0.1 | 0.1 |
| D.I. Water | q.s. to 100 | q.s. to 100 | q.s. to 100 |
| NaOH (18 % aqueous wt./wt.) | q.s. to pH 6.0 | q.s. to pH 6.0 | q.s. to pH 6.0 |

TABLE 12

| Rheological and Silicone Deposition Data | Invention Formulation 4 | Invention Formulation 5 | Comparison Formulation C-1 |
|---|---|---|---|
| Brookfield viscosity at 20 rpm (mPa·s) | 3,040 | 16,150 | 3,130 |
| Mica pearlescence | Pass | Pass | Pass |
| Net Si Peak Intensity (kcps) | 10.2 | 10.1 | 9.7 |

The results demonstrate that shampoos formulated with significantly higher concentrations of the stabilizer/thickener polymers of the invention prepared from CEA are able to deposit as much or more silicone than shampoos formulated with a commercial stabilizer/thickener polymer that is not prepared from CEA on European brown hair tresses. This is surprising since the deposition of silicone from thickened shampoos containing acrylic stabilizer/thickener polymers of the C-1 type is known to be inversely proportional to the amount of polymer present. Accordingly, it can be concluded that acrylic polymers prepared from CEA mitigate the loss of silicone compared to acrylic polymers that are not prepared from CEA.

Example 50

The stabilizer/thickener polymers of Examples 11 and 13 are formulated into two-in-one shampoo compositions utilizing the components set forth in Table 13. A commercially available stabilizer/thickener polymer is similarly formulated. The components are formulated by the method disclosed in Example 44. Each of the formulations is evaluated for Brookfield viscosity, pearlescence and silicone deposition. The results are set forth in Table 14.

TABLE 13

| Component | Invention Formulation 6 Active (wt %) | Invention Formulation 7 Active (wt %) | Comparative Formulation 3 Active (wt %) |
|---|---|---|---|
| Sulfochem™ ES-2 anionic surfactant | 14 | 14 | 14 |
| Chembetaine™ amphoteric surfactant | 3 | 3 | 3 |
| Silicone Emulsion (DC 2-1491) | 2 | 2 | 2 |

(continued)

| Component | Invention Formulation 6 Active (wt %) | Invention Formulation 7 Active (wt %) | Comparative Formulation 3 Active (wt %) |
|---|---|---|---|
| Polymer of Example 11 | 2.5 | -- | |
| Polymer of Example 13 | -- | 2.5 | -- |
| Polymer C1 | | | 1.5 |
| Jaguar™ C13-S cationic guar deposition aid | 0.25 | 0.25 | 0.25 |
| Timiron® Starlight Gold Cosmetic Mica Pigment (Rona) | 0.1 | 0.1 | 0.1 |
| D.I. Water | q.s. to 100 | q.s. to 100 | q.s. to 100 |
| NaOH (18 % aqueous wt./wt.) | q.s. to pH 6.0 | q.s. to pH 6.0 | q.s. to pH 6.0 |

TABLE 14

| Rheological and Silicone Deposition Data | Invention Formulation 6 | Invention Formulation 7 | Comparative Formulation 3 |
|---|---|---|---|
| Brookfield viscosity at 20 rpm (mPa·s) | 3,330 | 18,400 | 3,240 |
| Mica pearlescence | Pass | Pass | Pass |
| Net Si Peak Intensity (kcps) | 4.5 | 4.4 | 4.8 |

As in the example above, the results demonstrate that shampoos formulated with significantly higher concentrations of the stabilizer/thickener polymers of the invention prepared from CEA are able to deposit as much silicone than shampoos formulated with a commercial stabilizer/thickener polymer that is not prepared from CEA on European brown hair tresses. This is surprising since the deposition of silicone from thickened shampoos containing acrylic stabilizer/thickener polymers of the C-1 type is known to be inversely proportional to the amount of polymer present. Accordingly, it can be concluded that acrylic polymers prepared from CEA mitigate the loss of silicone compared to acrylic polymers that are not prepared from CEA.

## Claims

**1.** A personal care composition comprising:

A) at least one detersive surfactant selected from anionic, amphoteric, cationic, or nonionic surfactant;
B) at least one silicone conditioning agent;
C) water; and
D) a stabilizer/thickener polymer comprising a crosslinked acrylic copolymer prepared from a monomer composition comprising:

a) from 10 to 35 wt. %, from 15 to 35 wt. %, from 20 to 35 wt. %, or from 25 to 30 wt. % of a carboxyethyl acrylate monomer represented by the formula:

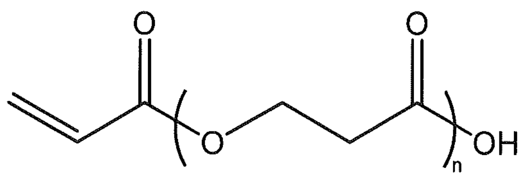

I

wherein n is an integer ranging from 1 to 10, from 2 to 8, or from 3 to 5;

b) from 30 to 70 wt.%, from 40 to 70 wt.%, from 50 to 70 wt.%, or from 55 to 65 wt. % of a monomer selected from ethyl acrylate, vinyl acetate, or mixtures thereof;

c) from 0 to 40 wt.%, from 5 to 40 wt.%, from 10 to 40 wt.%, from 15 to 35 wt. %, or from 20 or 25 to 30 wt. % of (meth)acrylic acid; and

d) from 0.001 to 5 wt.%, from 0.05 to 3.5 wt.%, from 0.5 to 3 wt.%, or from 1 to 2.5 wt. % of a polyunsaturated crosslinking monomer containing at least two polymerizable ethylenically unsaturated moieties.

**2.** A personal care composition of claim 1, wherein said monomer composition further comprises from 0.01 to 15 wt.% of at least one copolymerizable monomer selected from:

e) at least one $C_1$-$C_{22}$ alkyl (meth)acrylate other than ethyl acrylate;

f) at least one $C_1$-$C_8$ hydroxyalkyl (meth)acrylate;

g) at least one (meth)acrylamide selected from (meth)acrylamide, N-($C_1$-$C_{10}$)alkyl (meth)acrylamide, N,N-di($C_1$-$C_{10}$)alkyl (meth)acrylamide, N-($C_1$-$C_{10}$)alkylamino($C_1$-$C_{10}$)alkyl(meth)acrylamide or N,N-di($C_1$-$C_{10}$)alkylamino($C_1$-$C_{10}$)alkyl(meth)acrylamide;

h) at least one N-vinyl amide and/or N-vinyl lactam;

i) at least one an associative monomer comprising (i) a polymerizable ethylenically unsaturated end group portion, (ii) a polyoxyalkylene mid-section portion, and (iii) a hydrophobic end group portion containing 7 to 30 carbon atoms;

j) at least one semi-hydrophobic monomer selected from at least one monomer represented by formulas VI and VII:

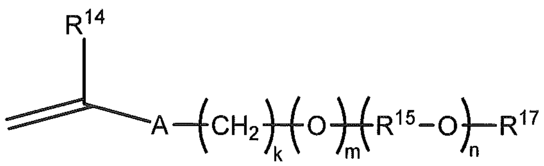 VI

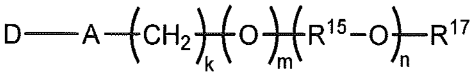 VII

wherein $R^{14}$ is hydrogen or methyl; A is $-CH_2C(O)O-$, $-C(O)O-$, $-O-$, $-CH_2O-$,$-NHC(O)NH-$, $-C(O)NH-$, $-Ar-(CE_2)_z-NHC(O)O-$, $-Ar-(CE_2)$,$-NHC(O)NH-$, or $-CH_2CH_2NHC(O)-$; Ar is a divalent arylene; E is H or methyl; z is 0 or 1; k is an integer ranging from 0 to 30, and m is 0 or 1, with the proviso that when k is 0, m is 0, and when k is in the range of 1 to 30, m is 1;

$(R^{15}-O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150, from 5 to 120, or from 10 to 60; $R^{17}$ is selected from hydrogen and a linear or branched $C_1$-$C_4$ alkyl group; and D represents a vinyl or an allyl moiety;

k) at least one silicone macromer;

l) at least one vinyl ester of an aliphatic carboxylic acid containing an acyl moiety having 3 to 21 carbon atoms;

m) at least one alpha-olefinic monomer;

n) at least one monomer represented by the formula:

$$CH_2=C(R)C(O)OAOR^2$$

wherein A is a divalent radical selected from $-CH_2CH(OH)CH_2-$ and $-CH_2CH(CH_2OH)-$, R is selected from hydrogen or methyl, and $R^2$ is an acyl residue of a linear or branched, saturated or unsaturated $C_{10}$ to $C_{22}$ fatty acid;

o) at least one copolymerizable sulfonic acid containing monomer selected from styrenesulfonic acid, 2-(meth)acrylamido-2-methylpropane sulfonic acid, vinylsulfonic acid, allylsulfonic acid, methallylsulfonic acid; and salts thereof; and

combinations of monomers e) through o).

3. A personal care composition of claim 2, wherein said $C_1$-$C_{22}$ alkyl (meth)acrylate monomer other than ethyl acrylate is represented by the formula:

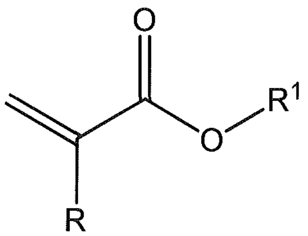

II

wherein R is hydrogen or methyl, and $R^1$ is methyl or a linear or branched $C_3$-$C_{22}$ alkyl group.

4. A personal care composition of any of the preceding claims 2 or 3, wherein said $C_1$-$C_8$ hydroxyalkyl (meth)acrylate monomer is represented by the formula:

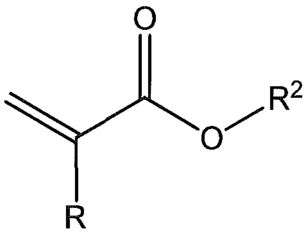

III

wherein R is hydrogen or methyl; and $R^2$ is a linear or branched $C_1$-$C_8$ hydroxyalkyl group.

5. A personal care composition of any of the preceding claims 2 to 4, wherein said (meth)acrylamide monomer is represented by the formulas:

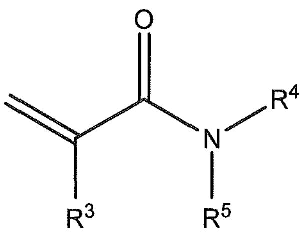

IV

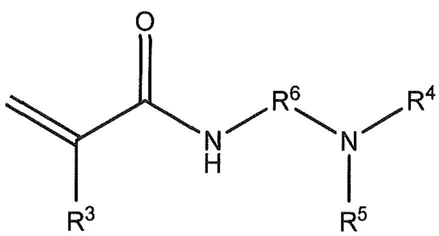

IVA

wherein $R^3$ represents hydrogen or methyl, $R^4$ and $R^5$ independently represent hydrogen, $C_1$ to $C_{10}$ alkyl and $C_1$ to $C_{10}$ hydroxyalkyl, and $R^6$ represents $C_1$ to $C_5$ alkylene.

6. A personal care composition of any of the preceding claims 2 to 5, wherein said N-vinyl amide is selected from N-vinylformamide, N-methyl-N-vinylformamide, N-(hydroxymethyl)-N-vinylformamide, N-vinylacetamide, N-vinylmethylacetamide, N-(hydroxymethyl)-N-vinylacetamide, and mixtures thereof; and said N-vinyl lactam is selected from N-vinyl-2-pyrrolidinone, N-(1-methyl vinyl) pyrrolidinone, N-vinyl-2-piperidone, N-vinyl-2-caprolactam, N-vinyl-5-methyl pyrrolidinone, N-vinyl-3,3-dimethyl pyrrolidinone, N-vinyl-5-ethyl pyrrolidinone and N-vinyl-6-methyl piperidone, and mixtures thereof.

**7.** A personal care composition of any of the preceding claims 2 to 6, wherein said associative monomer is represented by formulas V and/or VA:

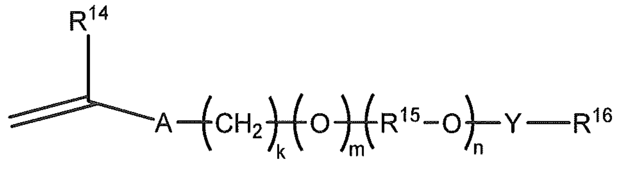

V

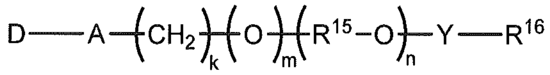

VA

wherein $R^{14}$ is hydrogen or methyl; A is $-CH_2C(O)O-$, $-C(O)O-$, $-O-$, $-CH_2O-$ ,$-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; a divalent alkylene radical containing 1 to 5 carbon atoms; Ar is a divalent arylene; E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety;
$(R^{15}-O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150, from 10 to 120, or from 15 to 60; Y is $-R^{15}O-$, $-R^{15}NH-$, $-C(O)-$, $-C(O)NH-$, $-R^{15}NHC(O)NH-$, or $-C(O)NHC(O)-$; $R^{16}$ is a substituted or unsubstituted alkyl selected from a $C_8$-$C_{30}$ linear alkyl, a $C_8$-$C_{30}$ branched alkyl, a $C_7$-$C_{30}$ carbocyclic alkyl, a $C_2$-$C_{30}$ alkyl-substituted phenyl, an araalkyl substituted phenyl, and an aryl-substituted $C_2$-$C_{30}$ alkyl; wherein the $R^{16}$ alkyl group, carbocyclic alkyl group, aryl group, phenyl group optionally comprises one or more substituents selected from the group selected from a methyl group, hydroxyl group, an alkoxyl group, benzyl group styryl group, and a halogen group, or

wherein said associative monomer is represented by formula VB:

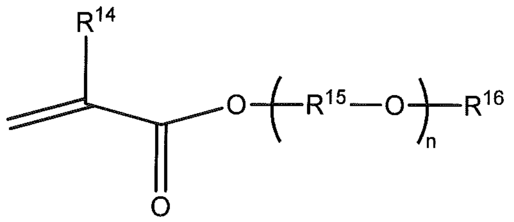

VB

wherein $R^{14}$ is hydrogen or methyl; $R^{15}$ is a divalent alkylene moiety independently selected from $C_2H_4$, $C_3H_6$, and $C_4H_8$, and n represents an integer ranging from 10 to 60, $(R^{15}-O)$ can be arranged in a random or a block configuration; $R^{16}$ is a substituted or unsubstituted alkyl selected from a $C_8$-$C_{30}$ linear alkyl, a $C_8$-$C_{30}$ branched alkyl, a $C_7$-$C_{30}$ carbocyclic alkyl, a $C_2$-$C_{30}$ alkyl-substituted phenyl, an araalkyl substituted phenyl, and an aryl-substituted $C_2$-$C_{30}$ alkyl, wherein the $R^{16}$ alkyl group, aryl group, phenyl group optionally comprises one or more substituents selected from the group consisting of a hydroxyl group, an alkoxyl group, benzyl group styryl group, and a halogen group.

**8.** A personal care composition of any of the preceding claims 2 to 7, wherein said semi-hydrophobic monomer comprises (i) a polymerizable ethylenically unsaturated end group portion, (ii) a polyoxyalkylene mid-section portion, and (iii) an end group portion selected from hydrogen or an alkyl group containing 1 to 4 carbon atoms.

**9.** A personal care composition of any of the preceding claims 2 to 8, wherein said silicon macromer comprises (i) a polymerizable citraconate end group portion, (ii) a polyoxyalkylene mid-section portion, and (iii) a dimethicone end group portion.

**10.** A personal care composition of any of the preceding claims 2 to 9, wherein said vinyl ester of a carboxylic acid is represented by the formula:

$$CH_2{=}CHOC(O)R^{18} \qquad IX$$

wherein $R^{18}$ is selected from a linear or branched $C_2$-$C_{21}$ alkyl group.

**11.** A personal care composition of any of the preceding claims 2 to 10, wherein said alpha-olefinic monomer is represented by the formula:

$$CH_2{=}CH\text{-}R^{19} \qquad X$$

wherein $R^{19}$ represents a moiety selected from hydrogen, a linear or branched $C_1$-$C_8$ alkyl, or aryl.

**12.** A personal care composition of any of the preceding claims 2 to 11, wherein said polyunsaturated crosslinking monomer is selected from the esterification product of (meth)acrylic acid and a linear or branched polyol having 2 to 12 carbon atoms; the etherification product of allyl alcohol and a linear or branched polyol having 2 to 12 carbon atoms; divinyl glycol, divinyl benzene; and methylenebisacrylamide.

**13.** A personal care composition of any of the preceding claims 2 to 12, wherein said copolymerizable sulfonic acid containing monomer is selected from styrenesulfonic acid, acrylamidomethylpropanesulfonic acid, vinylsulfonic acid, vinylphosphonic acid, allylsulfonic acid, methallylsulfonic acid; and salts thereof.

**14.** A personal care composition of any of the preceding claims, wherein said crosslinked acrylic copolymer comprises repeating units polymerized from:

a) from 10 to 35 wt.% of carboxyethyl acrylate
b) from 40 to 70 wt.% of a monomer selected from ethyl acrylate, vinyl acetate, or mixtures thereof;
c) 0 to 40 wt.% of a methacrylic acid; and
d) 0.1 to 1 wt.% of a polyunsaturated crosslinking monomer containing at least two polymerizable ethylenically unsaturated moieties.

**15.** A personal care composition of any of the preceding claims, wherein said silicone conditioning agent is non-volatile silicone selected from a silicone oil, silicone gum, silicone resin and mixtures thereof.

**16.** A method of reducing the loss of silicone deposition from a detersive conditioning composition for keratinous substrates, said composition comprised of from 3 to 40 wt.% of an anionic and amphoteric surfactant combination and from 0.01 to 20 wt.% of a silicone conditioning agent, wherein the weight ratio of active anionic surfactant to active amphoteric surfactant ranges from 1:1 to 10:1, said method comprised of adding thereto from 0.01 to 25 wt.% of a crosslinked acrylic copolymer prepared from a monomer composition comprising:

a) from 10 to 35 wt.% of carboxyethyl acrylate;
b) from 30 to 70 wt.% of a monomer selected from ethyl acrylate, vinyl acetate, or mixtures thereof;
c) from 0 to 40 wt.% of (meth)acrylic acid; and
d) from 0.001 to 5 wt.% of a polyunsaturated crosslinking monomer containing at least two polymerizable ethylenically unsaturated moieties.

**17.** Use of the personal care composition as defined in any of claims 1 to 14 as a detersive conditionaing composition for keratinous substrates.

**Patentansprüche**

**1.** Körperpflegezusammensetzung, umfassend:

A) wenigstens ein reinigendes Tensid, das aus einem anionischen, amphoteren, kationischen oder nichtionischen Tensid ausgewählt ist;
B) wenigstens ein Silikon-Konditionierungsmittel;

C) Wasser; und

D) ein Stabilisator-/Verdickungsmittel-Polymer, das ein vernetztes Acrylcopolymer umfasst, welches aus einer Monomerzusammensetzung hergestellt ist, die Folgendes umfasst:

a) 10 bis 35 Gew.-%, 15 bis 35 Gew.-%, 20 bis 35 Gew.-% oder 25 bis 30 Gew.-% eines Carboxyethylacrylat-Monomers, das durch die Formel

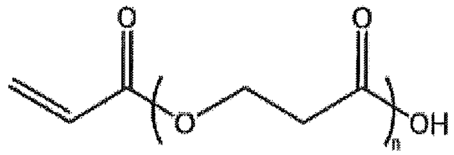

I

dargestellt wird, wobei n eine ganze Zahl im Bereich von 1 bis 10, 2 bis 8 oder 3 bis 5 ist;

b) 30 bis 70 Gew.-%, 40 bis 70 Gew.-%, 50 bis 70 Gew.-% oder 55 bis 65 Gew.-% eines Monomers, das aus Ethylacrylat, Vinylacetat oder Gemischen davon ausgewählt ist;

c) 0 bis 40 Gew.-%, 5 bis 40 Gew.-%, 10 bis 40 Gew.-%, 15 bis 35 Gew.-% oder 20 oder 25 bis 30 Gew.-% (Meth)acrylsäure; und

d) 0,001 bis 5 Gew.-%, 0,05 bis 3,5 Gew.-%, 0,5 bis 3 Gew.-% oder 1 bis 2,5 Gew.-% eines mehrfach ungesättigten vernetzenden Monomers, das wenigstens zwei polymerisierbare ethylenisch ungesättigte Struktureinheiten enthält.

**2.** Körperpflegezusammensetzung gemäß Anspruch 1, wobei die Monomerzusammensetzung weiterhin 0,01 bis 15 Gew.-% wenigstens eines copolymerisierbaren Monomers umfasst, das ausgewählt ist aus:

e) wenigstens einem $C_1$-$C_{22}$-Alkyl(meth)acrylat, das kein Ethylacrylat ist;

f) wenigstens einem $C_1$-$C_8$-Hydroxyalkyl(meth)acrylat;

g) wenigstens einem (Meth)acrylamid, das aus (Meth)acrylamid, N-($C_1$-$C_{10}$)-Alkyl(meth)acrylamid, N,N-Di($C_1$-$C_{10}$)alkyl(meth)acrylamid, N-($C_1$-$C_{10}$)-Alkylamino($C_1$-$C_{10}$)alkyl(meth)acrylamid oder N,N-Di-($C_1$-$C_{10}$)alkylamino($C_1$-$C_{10}$)alkyl(meth)acrylamid ausgewählt ist;

h) wenigstens einem N-Vinylamid und/oder N-Vinyllactam;

i) wenigstens einem assoziativen Monomer, das (i) einen polymerisierbaren, ethylenisch ungesättigten Endgruppenteil, (ii) einen Polyoxyalkylen-Mittelabschnittteil und (iii) einen hydrophoben Endgruppenteil, der 7 bis 30 Kohlenstoffatome enthält, umfasst;

j) wenigstens einem semihydrophoben Monomer, das aus wenigstens einem Monomer ausgewählt ist, welches durch die Formeln VI und VII dargestellt wird:

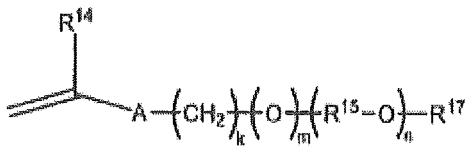

VI

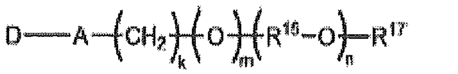

VII

wobei $R^{14}$ Wasserstoff oder Methyl ist, A = $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$ oder $-CH_2CH_2NHC(O)-$ ist, Ar ein zweiwertiges Arylen ist, E = H oder Methyl ist, z = 0 oder 1 ist, k eine ganze Zahl im Bereich von 0 bis 30 ist und m = 0 oder 1 ist, mit der Maßgabe, dass m = 0 ist, wenn k = 0 ist, und m = 1 ist, wenn k im Bereich von 1 bis 30 liegt, $(R^{15}-O)_n$ eine Polyoxyalkylen-Struktureinheit ist, bei der es sich um ein Homopolymer, ein statistisches Copolymer oder ein Blockcopolymer von $C_2$-$C_4$-Oxyalkyleneinheiten handeln kann, $R^{15}$ eine zweiwertige Alkylen-Struktureinheit ist, die aus $C_2H_4$, $C_3H_6$ oder $C_4H_8$ und Kombinationen davon ausgewählt ist, und n eine ganze Zahl im Bereich von 2 bis 150, 5 bis 120 oder 10 bis 60 ist, $R^{17}$ aus Wasserstoff und einer linearen oder verzweigten $C_1$-$C_4$-Alkylgruppe ausgewählt ist und D für eine Vinyl- oder Allyl-Struktureinheit steht;

k) wenigstens einem Silikonmakromer;

l) wenigstens einem Vinylester einer aliphatischen Carbonsäure, die eine Acyl-Struktureinheit mit 3 bis 21

Kohlenstoffatomen enthält;
m) wenigstens einem $\alpha$-olefinischen Monomer;
n) wenigstens einem Monomer, das durch die Formel

$$CH_2=C(R)C(O)OAOR^2$$

dargestellt wird, wobei A ein zweiwertiger Rest ist, der aus $-CH_2CH(OH)CH_2-$ und $-CH_2CH(CH_2OH)-$ ausgewählt ist, R aus Wasserstoff oder Methyl ausgewählt ist und $R^2$ ein Acylrest einer linearen oder verzweigten, gesättigten oder ungesättigten $C_{10}$- bis $C_{22}$-Fettsäure ist;
o) wenigstens einem copolymerisierbaren sulfonsäurehaltigen Monomer, das aus Styrolsulfonsäure, 2-(Meth)acrylamido-2-methylpropansulfonsäure, Vinylsulfonsäure, Allylsulfonsäure, Methallylsulfonsäure und Salzen davon ausgewählt ist; und

Kombinationen der Monomere e) bis o).

**3.** Körperpflegezusammensetzung gemäß Anspruch 2, wobei das $C_1$-$C_{22}$-Alkyl(meth)acrylat-Monomer, das kein Ethylacrylat ist, durch die Formel

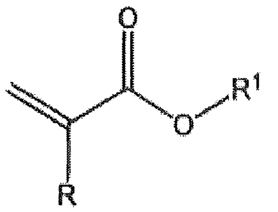

II

dargestellt wird, wobei R Wasserstoff oder Methyl ist und $R^1$ Methyl oder eine lineare oder verzweigte $C_3$-$C_{22}$-Alkylgruppe ist.

**4.** Körperpflegezusammensetzung gemäß einem der vorstehenden Ansprüche 2 oder 3, wobei das $C_1$-$C_8$-Hydroxyalkyl(meth)acrylat-Monomer durch die Formel

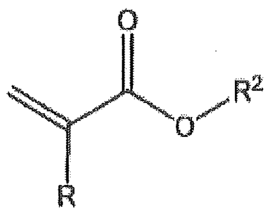

III

dargestellt wird, wobei R Wasserstoff oder Methyl ist und $R^2$ eine lineare oder verzweigte $C_1$-$C_8$-Hydroxyalkylgruppe ist.

**5.** Körperpflegezusammensetzung gemäß einem der vorstehenden Ansprüche 2 bis 4, wobei das (Meth)acrylamid-Monomer durch die Formeln

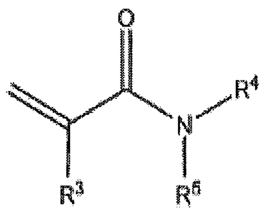

IV

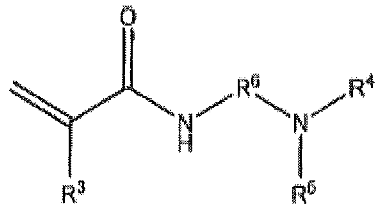

IVA

dargestellt wird, wobei $R^3$ für Wasserstoff oder Methyl steht, $R^4$ und $R^5$ unabhängig für Wasserstoff, $C_1$- bis $C_{10}$-Alkyl und $C_1$- bis $C_{10}$-Hydroxyalkyl stehen und $R^6$ für $C_1$- bis $C_5$-Alkylen steht.

**6.** Körperpflegezusammensetzung gemäß einem der vorstehenden Ansprüche 2 bis 5, wobei das N-Vinylamid aus N-Vinylformamid, N-Methyl-N-vinylformamid, N-(Hydroxymethyl)-N-vinylformamid, N-Vinylacetamid, N-Vinylmethylacetamid, N-(Hydroxymethyl)-N-vinylacetamid und Gemischen davon ausgewählt ist und das N-Vinyllactam aus N-Vinyl-2-pyrrolidinon, N-(1-Methylvinyl)pyrrolidinon, N-Vinyl-2-piperidon, N-Vinyl-2-caprolactam, N-Vinyl-5-methylpyrrolidinon, N-Vinyl-3,3-dimethylpyrrolidinon, N-Vinyl-5-ethylpyrrolidinon und N-Vinyl-6-methylpiperidon und Gemischen davon ausgewählt ist.

**7.** Körperpflegezusammensetzung gemäß einem der vorstehenden Ansprüche 2 bis 6, wobei das assoziative Monomer durch die Formeln V und/oder VA dargestellt wird:

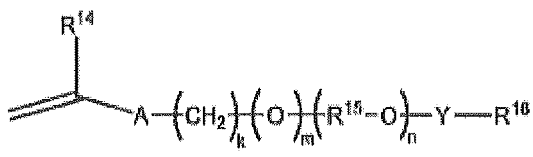

V

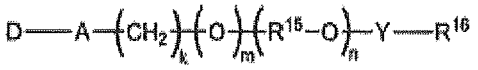

VA ,

wobei $R^{14}$ Wasserstoff oder Methyl ist, A = $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$ oder $-CH_2CH_2NHC(O)-$ ist, ein zweiwertiger Alkylenrest, das 1 bis 5 Kohlenstoffatome enthält, Ar ein zweiwertiges Arylen ist, E = H oder Methyl ist, z = 0 oder 1 ist, k eine ganze Zahl im Bereich von 0 bis 30 ist und m = 0 oder 1 ist, mit der Maßgabe, dass m = 0 ist, wenn k = 0 ist, und m = 1 ist, wenn k im Bereich von 1 bis 30 liegt, D für eine Vinyl- oder Allyl-Struktureinheit steht, $(R^{15}-O)_n$ eine Polyoxyalkylen-Struktureinheit ist, bei der es sich um ein Homopolymer, ein statistisches Copolymer oder ein Blockcopolymer von $C_2$-$C_4$-Oxyalkyleneinheiten handeln kann, $R^{15}$ eine zweiwertige Alkylen-Struktureinheit ist, die aus $C_2H_4$, $C_3H_6$ oder $C_4H_8$ und Kombinationen davon ausgewählt ist, und n eine ganze Zahl im Bereich von 2 bis 150, 10 bis 120 oder 15 bis 60 ist, Y = $-R^{15}O-$, $-R^{15}NH-$, $-C(O)-$, $-C(O)NH-$, $-R^{15}NHC(O)NH-$ oder $-C(O)NHC(O)-$ ist, $R^{16}$ ein substituiertes oder unsubstituiertes Alkyl ist, das aus einem linearen $C_8$-$C_{30}$-Alkyl, einem verzweigten $C_8$-$C_{30}$-Alkyl, einem carbocyclischen $C_7$-$C_{30}$-Alkyl, einem mit $C_2$-$C_{30}$-Alkyl substituierten Phenyl, einem Aralkylsubstituierten Phenyl und einem arylsubstituierten $C_2$-$C_{30}$-Alkyl ausgewählt ist, wobei die $R^{16}$-Alkylgruppe, die carbocyclische Alkylgruppe, die Arylgruppe und die Phenylgruppe gegebenenfalls einen oder mehrere Substituenten umfassen, die aus der Gruppe ausgewählt sind, die aus einer Methylgruppe, Hydroxygruppe, Alkoxygruppe, Benzylgruppe, Styrylgruppe und Halogengruppe besteht, oder
wobei das assoziative Monomer durch Formel VB dargestellt wird:

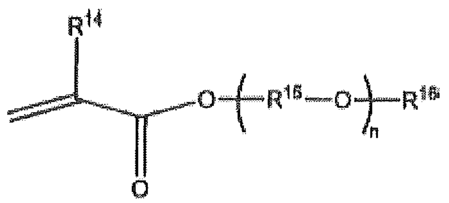

VB

wobei $R^{14}$ Wasserstoff oder Methyl ist, $R^{15}$ eine zweiwertige Alkylen-Struktureinheit ist, die unabhängig aus $C_2H_4$,

$C_3H_6$ und $C_4H_8$ ausgewählt ist, und n für eine ganze Zahl im Bereich von 10 bis 60 steht, ($R^{15}$-O) in einer statistischen oder einer Blockkonfiguration angeordnet sein kann, $R^{16}$ ein substituiertes oder unsubstituiertes Alkyl ist, das aus einem linearen $C_8$-$C_{30}$-Alkyl, einem verzweigten $C_8$-$C_{30}$-Alkyl, einem carbocyclischen $C_7$-$C_{30}$-Alkyl, einem mit $C_2$-$C_{30}$-Alkyl substituierten Phenyl, einem Aralkylsubstituierten Phenyl und einem arylsubstituierten $C_2$-$C_{30}$-Alkyl ausgewählt ist, wobei die $R^{16}$-Alkylgruppe, die die Arylgruppe und die Phenylgruppe gegebenenfalls einen oder mehrere Substituenten umfassen, die aus der Gruppe ausgewählt sind, die aus einer Hydroxygruppe, Alkoxygruppe, Benzylgruppe, Styrylgruppe und Halogengruppe besteht.

**8.** Körperpflegezusammensetzung gemäß einem der vorstehenden Ansprüche 2 bis 7, wobei das semihydrophobe Monomer (i) einen polymerisierbaren, ethylenisch ungesättigten Endgruppenteil, (ii) einen Polyoxyalkylen-Mittelabschnittteil und (iii) einen Endgruppenteil, der aus Wasserstoff oder einer Alkylgruppe, die 1 bis 4 Kohlenstoffatome enthält, ausgewählt ist, umfasst.

**9.** Körperpflegezusammensetzung gemäß einem der vorstehenden Ansprüche 2 bis 8, wobei das Silikonmakromer (i) einen polymerisierbaren Citraconat-Endgruppenteil, (ii) einen Polyoxyalkylen-Mittelabschnittteil und (iii) einen Dimethicon-Endgruppenteil umfasst.

**10.** Körperpflegezusammensetzung gemäß einem der vorstehenden Ansprüche 2 bis 9, wobei der Vinylester einer Carbonsäure durch die Formel

$$CH_2{=}CHOC(O)_R{}^{18} \qquad \text{IX}$$

dargestellt wird, wobei $R^{18}$ aus einer linearen oder verzweigten $C_2$-$C_{21}$-Alkylgruppe ausgewählt ist.

**11.** Körperpflegezusammensetzung gemäß einem der vorstehenden Ansprüche 2 bis 10, wobei das $\alpha$-olefinische Monomer durch die Formel

$$CH_2{=}CH\text{-}R^{19} \qquad \text{X}$$

dargestellt wird, wobei $R^{19}$ für eine Struktureinheit steht, die aus Wasserstoff, einem linearen oder verzweigten $C_1$-$C_8$-Alkyl oder Aryl ausgewählt ist.

**12.** Körperpflegezusammensetzung gemäß einem der vorstehenden Ansprüche 2 bis 11, wobei das mehrfach ungesättigte vernetzende Monomer aus dem Veresterungsprodukt von (Meth)acrylsäure und einem linearen oder verzweigten Polyol mit 2 bis 12 Kohlenstoffatomen, dem Veresterungsprodukt von Allylalkohol und einem linearen oder verzweigten Polyol mit 2 bis 12 Kohlenstoffatomen, Divinylglycol, Divinylbenzol und Methylenbisacrylamid ausgewählt ist.

**13.** Körperpflegezusammensetzung gemäß einem der vorstehenden Ansprüche 2 bis 12, wobei das copolymerisierbare sulfonsäurehaltige Monomer aus Styrolsulfonsäure, Acrylamidomethylpropansulfonsäure, Vinylsulfonsäure, Vinylphosphonsäure, Allylsulfonsäure, Methallylsulfonsäure und Salzen davon ausgewählt ist.

**14.** Körperpflegezusammensetzung gemäß einem der vorstehenden Ansprüche, wobei das vernetzte Acrylcopolymer Repetiereinheiten umfasst, die aus

a) 10 bis 35 Gew.-% Carboxyethylacrylat;
b) 40 bis 70 Gew.-% eines Monomers, das aus Ethylacrylat, Vinylacetat oder Gemischen davon ausgewählt ist;
c) 0 bis 40 Gew.-% einer Methacrylsäure; und
d) 0,1 bis 1 Gew.-% eines mehrfach ungesättigten vernetzenden Monomers, das wenigstens zwei polymerisierbare ethylenisch ungesättigte Struktureinheiten enthält,

polymerisiert sind.

**15.** Körperpflegezusammensetzung gemäß einem der vorstehenden Ansprüche, wobei es sich bei dem Silikon-Konditionierungsmittel um ein nichtflüchtiges Silikon handelt, das aus einem Silikonöl, Silikonkautschuk, Silikonharz und Gemischen davon ausgewählt ist.

**16.** Verfahren zum Reduzieren des Verlusts an Silikonablagerungen aus einer reinigenden Konditionierungszusammensetzung für keratinöse Substrate, wobei die Zusammensetzung aus 3 bis 40 Gew.-% einer Kombination aus einem anionischen und einem amphoteren Tensid und 0,01 bis 20 Gew.-% eines Silikon-Konditionierungsmittels besteht, wobei das Gewichtsverhältnis des aktiven anionischen Tensids zu dem aktiven amphoteren Tensid im Bereich von 1:1 bis 10:1 liegt, wobei das Verfahren das Hinzufügen von 0,01 bis 25 Gew.-% eines vernetzten Acrylcopolymers umfasst, das aus einer Monomerzusammensetzung hergestellt ist, die Folgendes umfasst:

a) 10 bis 35 Gew.-% Carboxyethylacrylat;
b) 30 bis 70 Gew.-% eines Monomers, das aus Ethylacrylat, Vinylacetat oder Gemischen davon ausgewählt ist;
c) 0 bis 40 Gew.-% (Meth)acrylsäure; und
d) 0,001 bis 5 Gew.-% eines mehrfach ungesättigten vernetzenden Monomers, das wenigstens zwei polymerisierbare ethylenisch ungesättigte Struktureinheiten enthält.

**17.** Verwendung der Körperpflegezusammensetzung gemäß einem der Ansprüche 1 bis 14 als reinigende Konditionierungszusammensetzung für keratinöse Substrate.

## Revendications

**1.** Composition pour les soins personnels comprenant:

A) au moins un tensioactif détersif choisi parmi un tensioactif anionique, amphotère, cationique ou non ionique;
B) au moins un agent de conditionnement silicone;
C) de l'eau; et
D) un polymère stabilisant/épaississant comprenant un copolymère acrylique réticulé préparé à partir d'une composition de monomères comprenant:

a) de 10 à 35 % en poids, de 15 à 35 % en poids, de 20 à 35 % en poids, ou de 25 à 30 % en poids d'un monomère d'acrylate de carboxyéthyle représenté par la formule:

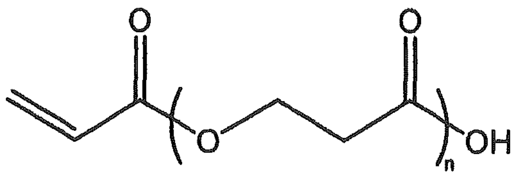

I

où n est un entier allant de 1 à 10, de 2 à 8, ou de 3 à 5;
b) de 30 à 70 % en poids, de 40 à 70 % en poids, de 50 à 70 % en poids, ou de 55 à 65 % en poids d'un monomère choisi parmi l'acrylate d'éthyle, l'acétate de vinyle, ou les mélanges de ceux-ci;
c) de 0 à 40 % en poids, de 5 à 40 % en poids, de 10 à 40 % en poids, de 15 à 35 % en poids, ou de 20 ou 25 à 30 % en poids d'acide (méth)acrylique; et
d) de 0,001 à 5 % en poids, de 0,05 à 3,5 % en poids, de 0,5 à 3 % en poids, ou de 1 à 2,5 % en poids d'un monomère réticulant polyinsaturé contenant au moins deux groupements éthyléniquement insaturés polymérisables.

**2.** Composition pour les soins personnels selon la revendication 1, où ladite composition de monomères comprend en outre de 0,01 à 15 % en poids d'au moins un monomère copolymérisable choisi parmi:

e) au moins un (méth)acrylate de $C_1$-$C_{22}$ alkyle autre que l'acrylate d'éthyle;
f) au moins un (méth)acrylate de $C_1$-$C_8$ hydroxyalkyle;
g) au moins un (méth)acrylamide choisi parmi le (méth)acrylamide, un N-($C_1$-$C_{10}$)alkyl-(méth)acrylamide, un N,N-di($C_1$-$C_{10}$)alkyl-(méth)acrylamide, un N-($C_1$-$C_{10}$)alkylamino($C_1$-$C_{10}$)alkyl(méth)acrylamide ou un N,N-di($C_1$-$C_{10}$)alkylamino($C_1$-$C_{10}$)alkyl(méth)acrylamide;
h) au moins un N-vinylamide et/ou N-vinyl-lactame;
i) au moins un monomère associatif comprenant (i) une partie formant groupe terminal éthyléniquement insaturée polymérisable, (ii) une partie formant section moyenne polyoxyalkylène, et (iii) une partie formant groupe terminal hydrophobe contenant 7 à 30 atomes de carbone;
j) au moins un monomère semi-hydrophobe choisi parmi au moins un monomère représenté par les formules

VI et VII:

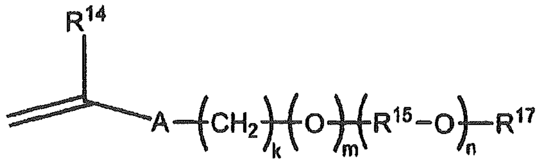

VI

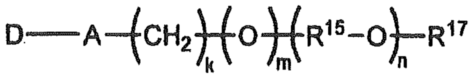

VII

où $R^{14}$ est l'hydrogène ou méthyle; A est $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, ou $-CH_2CH_2NHC(O)-$; Ar est un arylène divalent; E est H ou méthyle; z est 0 ou 1; k est un entier allant de 0 à 30, et m est 0 ou 1, avec la condition que quand k est 0, m est 0, et quand k est dans la plage de 1 à 30, m est 1; $(R^{15}-O)_n$ est un groupement polyoxyalkylène, qui peut être un homopolymère, un copolymère statistique, ou un copolymère séquencé d'unités de $C_2$-$C_4$ oxyalkylène, $R^{15}$ est un groupement alkylène divalent choisi parmi $C_2H_4$, $C_3H_6$, ou $C_4H_8$, et les combinaisons de ceux-ci; et n est un entier dans la plage de 2 à 150, de 5 à 120, ou de 10 à 60; $R^{17}$ est choisi parmi l'hydrogène et un groupe $C_1$-$C_4$ alkyle linéaire ou ramifié; et D représente un groupement vinyle ou un groupement allyle;

k) au moins un macromère de silicone;

1) au moins un vinylester d'un acide carboxylique aliphatique contenant un groupement acyle ayant 3 à 21 atomes de carbone;

m) au moins un monomère alpha-oléfinique;

n) au moins un monomère représenté par la formule:

$$CH_2=C(R)C(O)OAOR^2$$

où A est un radical divalent choisi parmi $-CH_2CH(OH)CH_2-$ et $-CH_2CH(CH_2OH)-$, R est choisi parmi l'hydrogène ou méthyle, et $R^2$ est un résidu acyle d'un acide gras $C_{10}$ à $C_{22}$ saturé ou insaturé, linéaire ou ramifié;

o) au moins un monomère contenant un acide sulfonique copolymérisable choisi parmi l'acide styrènesulfonique, l'acide 2-(méth)-acrylamido-2-méthylpropanesulfonique, l'acide vinylsulfonique, l'acide allylsulfonique, l'acide méthallylsulfonique; et les sels de ceux-ci; et

les combinaisons de monomères e) à o).

**3.** Composition pour les soins personnels selon la revendication 2, où ledit monomère de (méth)acrylate de $C_1$-$C_{22}$ alkyle autre que l'acrylate d'éthyle est représenté par la formule:

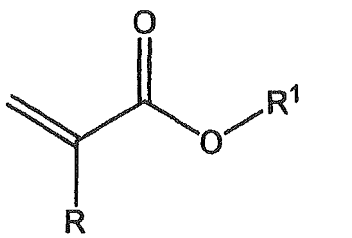

II

où R est l'hydrogène ou méthyle, et $R^1$ est méthyle ou un groupe $C_3$-$C_{22}$ alkyle linéaire ou ramifié.

**4.** Composition pour les soins personnels selon l'une quelconque des revendications 2 ou 3 précédentes, où ledit monomère de (méth)acrylate de $C_1$-$C_8$ hydroxyalkyle est représenté par la formule:

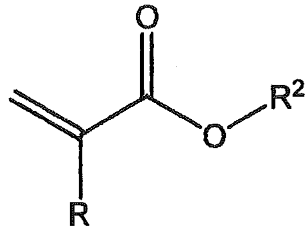

III

où R est l'hydrogène ou méthyle; et $R^2$ est un groupe $C_1$-$C_8$ hydroxyalkyle linéaire ou ramifié.

**5.** Composition pour les soins personnels selon l'une quelconque des revendications 2 à 4 précédentes, où ledit monomère de (méth)acrylamide est représenté par les formules:

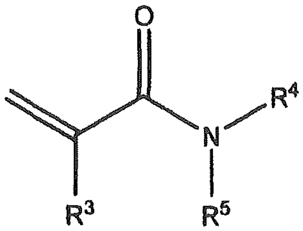

IV

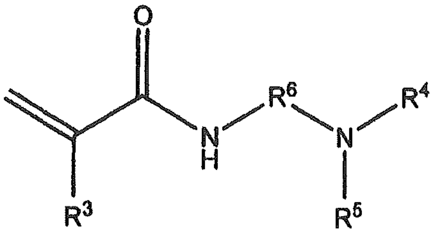

IVA

où $R^3$ représente l'hydrogène ou méthyle, $R^4$ et $R^5$ représentent indépendamment l'hydrogène, $C_1$ à $C_{10}$ alkyle et $C_1$ à $C_{10}$ hydroxyalkyle, et $R^6$ représente $C_1$ à $C_5$ alkylène.

**6.** Composition pour les soins personnels selon l'une quelconque des revendications 2 à 5 précédentes, où ledit N-vinylamide est choisi parmi le N-vinylformamide, le N-méthyl-N-vinylformamide, le N-(hydroxyméthyl)-N-vinylformamide, le N-vinylacétamide, le N-vinylméthylacétamide, le N-(hydroxyméthyl)-N-vinylacétamide, et les mélanges de ceux-ci; et ledit N-vinyl-lactame est choisi parmi la N-vinyl-2-pyrrolidinone, la N-(l-méthylvinyl)-pyrrolidinone, la N-vinyl-2-pipéridone, le N-vinyl-2-caprolactame, la N-vinyl-5-méthylpyrrolidinone, la N-vinyl-3,3-diméthylpyrrolidinone, la N-vinyl-5-éthylpyrrolidinone et la N-vinyl-6-méthylpipéridone, et les mélanges de ceux-ci.

**7.** Composition pour les soins personnels selon l'une quelconque des revendications 2 à 6 précédentes, où ledit monomère associatif est représenté par les formules V et/ou VA:

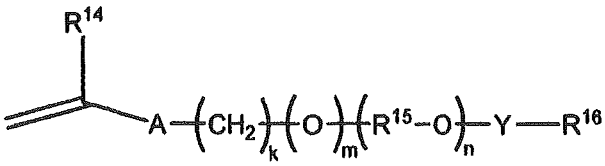

V

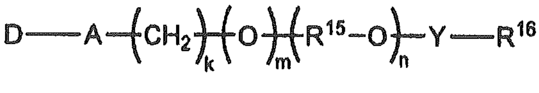

VA

où $R^{14}$ est l'hydrogène ou méthyle; A est $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, ou $-CH_2CH_2NHC(O)-$; un radical alkylène divalent contenant 1 à 5

atomes de carbone; Ar est un arylène divalent; E est H ou méthyle; z est 0 ou 1; k est un entier allant de 0 à 30, et m est 0 ou 1, avec la condition que quand k est 0, m est 0, et quand k est dans la plage de 1 à 30, m est 1; D représente un groupement vinyle ou un groupement allyle; $(R^{15}\text{-}O)_n$ est un groupement polyoxyalkylène, qui peut être un homopolymère, un copolymère statistique, ou un copolymère séquencé d'unités de $C_2$-$C_4$ oxyalkylène, $R^{15}$ est un groupement alkylène divalent choisi parmi $C_2H_4$, $C_3H_6$, ou $C_4H_8$, et les combinaisons de ceux-ci; et n est un entier dans la plage de 2 à 150, de 10 à 120, ou de 15 à 60; Y est $-R^{15}O-$, $R^{15}NH-$, -C(O)-, -C(O)NH-, $-R^{15}NHC(O)NH-$, ou -C(O)NHC(O)-; $R^{16}$ est un alkyle substitué ou non substitué choisi parmi un $C_8$-$C_{30}$ alkyle linéaire, un $C_8$-$C_{30}$ alkyle ramifié, un $C_7$-$C_{30}$ alkyle carbocyclique, un phényle $C_2$-$C_{30}$ alkyl-substitué, un phényle substitué par araalkyle, et un $C_2$-$C_{30}$ alkyle aryl-substitué; où le groupe alkyle, le groupe alkyle carbocyclique, le groupe aryle, le groupe phényle $R^{16}$ comprend éventuellement un ou plusieurs substituants choisis dans le groupe choisi parmi un groupe méthyle, un groupe hydroxyle, un groupe alcoxyle, un groupe benzyle, un groupe styryle, et un groupe halogène, ou bien où ledit monomère associatif est représenté par la formule VB:

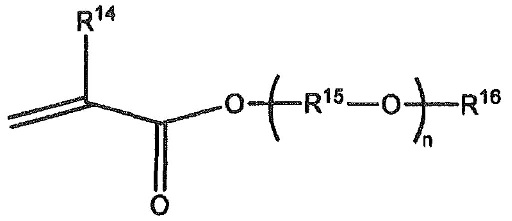

VB

où $R^{14}$ est l'hydrogène ou méthyle; $R^{15}$ est un groupement alkylène divalent choisi indépendamment parmi $C_2H_4$, $C_3H_6$, et $C_4H_8$, et n représente un entier allant de 10 à 60, $(R^{15}\text{-}O)$ peut être disposé dans une configuration statistique ou séquencée; $R^{16}$ est un alkyle substitué ou non substitué choisi parmi un $C_8$-$C_{30}$ alkyle linéaire, un $C_8$-$C_{30}$ alkyle ramifié, un $C_7$-$C_{30}$ alkyle carbocyclique, un phényle $C_2$-$C_{30}$ alkyl-substitué, un phényle substitué par araalkyle, et un $C_2$-$C_{30}$ alkyle aryl-substitué, où le groupe alkyle, le groupe aryle, le groupe phényle $R^{16}$ comprend éventuellement un ou plusieurs substituants choisis dans le groupe consistant en un groupe hydroxyle, un groupe alcoxyle, un groupe benzyle, un groupe styryle et un groupe halogène.

**8.** Composition pour les soins personnels selon l'une quelconque des revendications 2 à 7 précédentes, où ledit monomère semi-hydrophobe comprend (i) une partie formant groupe terminal éthyléniquement insaturé polymérisable, (ii) une partie formant section moyenne polyoxyalkylène, et (iii) une partie formant groupe terminal choisie parmi l'hydrogène ou un groupe alkyle contenant 1 à 4 atomes de carbone.

**9.** Composition pour les soins personnels selon l'une quelconque des revendications 2 à 8 précédentes, où ledit macromère de silicone comprend (i) une partie formant groupe terminal citraconate polymérisable, (ii) une partie formant section moyenne polyoxyalkylène, et (iii) une partie formant groupe terminal diméthicone.

**10.** Composition pour les soins personnels selon l'une quelconque des revendications 2 à 9 précédentes, où ledit vinylester d'un acide carboxylique est représenté par la formule:

$$CH_2{=}CHOC(O)R^{18} \qquad \text{IX}$$

où $R^{18}$ est choisi parmi un groupe $C_2$-$C_{21}$ alkyle linéaire ou ramifié.

**11.** Composition pour les soins personnels selon l'une quelconque des revendications 2 à 10 précédentes, où ledit monomère alpha-oléfinique est représenté par la formule:

$$CH_2{=}CH\text{-}R^{19} \qquad \text{X}$$

où $R^{19}$ représente un groupement choisi parmi l'hydrogène, un $C_1$-$C_8$ alkyle linéaire ou ramifié, ou aryle.

**12.** Composition pour les soins personnels selon l'une quelconque des revendications 2 à 11 précédentes, où ledit monomère réticulant polyinsaturé est choisi parmi le produit d'estérification d'acide (méth)acrylique et d'un polyol linéaire ou ramifié ayant 2 à 12 atomes de carbone; le produit d'éthérification de l'alcool allylique et d'un polyol linéaire ou ramifié ayant 2 à 12 atomes de carbone; le divinylglycol, le divinylbenzène; et le méthylènebisacrylamide.

**13.** Composition pour les soins personnels selon l'une quelconque des revendications 2 à 12 précédentes, où ledit monomère contenant un acide sulfonique copolymérisable est choisi parmi l'acide styrènesulfonique, l'acide acrylamidométhylpropanesulfonique, l'acide vinylsulfonique, l'acide vinyl-phosphonique, l'acide allylsulfonique, l'acide méthallylsulfonique; et les sels de ceux-ci.

**14.** Composition pour les soins personnels selon l'une quelconque des revendications précédentes, où ledit copolymère acrylique réticulé comprend des unités répétées polymérisées à partir de:

a) de 10 à 35 % en poids d'acrylate de carboxyéthyle ;
b) de 40 à 70 % en poids d'un monomère choisi parmi l'acrylate d'éthyle, l'acétate de vinyle, ou les mélanges de ceux-ci;
c) 0 à 40 % en poids d'un acide méthacrylique; et
d) 0,1 à 1 % en poids d'un monomère réticulant polyinsaturé contenant au moins deux groupements éthyléniquement insaturés polymérisables.

**15.** Composition pour les soins personnels selon l'une quelconque des revendications précédentes, où ledit agent de conditionnement silicone est un silicone non-volatile choisi parmi une huile de silicone, une gomme de silicone, une résine de silicone et les mélanges de celles-ci.

**16.** Procédé de réduction de la perte de dépôt de silicone à partir d'une composition de conditionnement détersive pour substrats kératineux, ladite composition comprenant de 3 à 40 % en poids d'une combinaison de tensioactifs anionique et amphotère et de 0,01 à 20 % en poids d'un agent de conditionnement silicone, où le rapport en poids du tensioactif anionique actif au tensioactif amphotère actif va de 1:1 à 10:1, ledit procédé comprenant l'addition à celle-ci de 0,01 à 25 % en poids d'un copolymère acrylique réticulé préparé à partir d'une composition de monomères comprenant:

a) de 10 à 35 % en poids d'acrylate de carboxyéthyle;
b) de 30 à 70 % en poids d'un monomère choisi parmi l'acrylate d'éthyle, l'acétate de vinyle, ou les mélanges de ceux-ci;
c) de 0 à 40 % en poids d'acide (méth)acrylique; et
d) de 0,001 à 5 % en poids d'un monomère réticulant polyinsaturé contenant au moins deux groupements éthyléniquement insaturés polymérisables.

**17.** Utilisation de la composition pour les soins personnels telle qu'elle est définie dans l'une quelconque des revendications 1 à 14 comme composition de conditionnement détersive pour substrats kératineux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5302658 A
- US 6635702 B
- US 20030108503 A
- US 7504094 B
- US 2004001796 A
- US 2011064688 A
- US 2008311066 A
- EP 0036294 A
- US 6140435 A, Zanotti-Russo
- US 33156 A
- US 5294692 A
- US 5011978 A
- US 4421902 A, Chang
- US 4384096 A, Sonnabend
- US 4514552 A, Shay
- US 4600761 A, Ruffner
- US 4616074 A, Ruffner
- US 5292843 A, Jenkins
- US 5770760 A, Robinson
- US 5412142 A, Wilkerson, III
- US 7772421 A, Yang
- US 3332880 A
- US 5104646 A
- US 5106609 A
- US 3929678 A
- US 2658072 A
- US 2438091 A
- US 2528378 A
- US 6573375 B
- US 6727357 B
- US 6200554 B
- US 4654207 A
- US 5019376 A
- US 5384114 A
- US 6213166 B, Thibiant
- US 20040219119 A, Wei
- US 20110117225 A, Wei
- US 20060079417 A
- US 20060079418 A
- US 20060079419 A
- US 20060079420 A
- US 20060079421 A
- US 20060079422 A
- US 20070009463 A
- US 20070072781 A
- US 20070280976 A
- US 20080317698 A


### Non-patent literature cited in the description


- Cosmetic Science and Technology Series. Conditioning Agents for Hair and Skin. Marcel Dekker, Inc, 1999, vol. 2
- McCutcheon's, Emulsifiers and Detergents. M. C. Publishing Co, 2003
- Encyclopedia of Polymer Science and Engineering. John Wiley & Sons, Inc, 1989, vol. 15, 204-308
- Silicones: Preparation, Properties and Performance. Dow Corning Corporation, 2005
- **TODD ; BYERS.** Volatile Silicone Fluids for Cosmetics. *Cosmetics and Toiletries,* 1976, vol. 91 (1), 27-32
- **KASPRZAK.** Volatile Silicones. *Soap/Cosmetics/Chemical Specialities,* December 1986, 40-43
- International Cosmetic Ingredient Dictionary. 1993
- Cosmetic, Toiletry, and Fragrance Association (CTFA) Cosmetic Ingredient Handbook. 1992
- CTFA International Cosmetic Ingredient Dictionary/Handbook via the CTFA website as well as the CTFA Cosmetic Ingredient Handbook. Cosmetic and Fragrance Assn., Inc, 2002
- **HUNTING.** Opacifiers and pearling agents in shampoos. *Cosmetic and Toiletries,* July 1981, vol. 96, 65-78
- CTFA Cosmetic Ingredient Handbook. 1988, 75